# EUROPEAN PATENT SPECIFICATION

(11) **EP 3 068 878 B1**
(45) Date of publication and mention of the grant of the patent: **04.09.2019**
(21) Application number: 14799422.2
(22) Date of filing: 14.11.2014
(51) Int. Cl.: C12N 9/36, C12N 15/62, A61K 38/00

(54) **MODIFIED EL188 ENDOLYSIN SEQUENCE**
MODIFIZIERTE EL188-ENDOLYSINSEQUENZ
SÉQUENCE D'ENDOLYSINE EL188 MODIFIÉE

(30) Priority: 14.11.2013 WO PCT/EP2013/073872
(43) Date of publication of application: 21.09.2016
(73) Proprietor: Lysando AG, 9497 Triesenberg (LI)
(72) Inventor: ANOSOVA, Irina, 14482 Potsdam (DE)
(74) Representative: Rückerl, Florian
(86) International application number: PCT/EP2014/074678
(87) International publication number: WO 2015/071437

(56) References cited:
- WO-A1-2011/134998
- WO-A2-2010/149792
- WO-A2-2012/085259
- YVES BRIERS ET AL: "Muralytic activity and modular structure of the endolysins of Pseudomonas aeruginosa bacteriophages ?KZ and EL", MOLECULAR MICROBIOLOGY, vol. 65, no. 5, 30 July 2007 (2007-07-30), pages 1334-1344, XP055119325, ISSN: 0950-382X, DOI: 10.1111/j.1365-2958.2007.05870.x

### SEQUENCE LISTING

<110> Lysando AG
<120> Modified EL188 endolysin sequence
<130> LYS-022 PCT 1
<150> PCT/EP2013/073872
   <151> 2013-11-14
<160> 251
<170> PatentIn version 3.5
<210> 1
   <211> 292
   <212> PRT
   <213> Artificial Sequence
<220>
   <223> Consensus Sequence
<220>
   <221> MISC_FEATURE
   <222> (1)..(1)
   <223> Xaa can be any naturally occurring amino acid or absent; in particular it can be methionine
<220>
   <221> MISC_FEATURE
   <222> (120)..(120)
   <223> Xaa can be any naturally occurring amino acid; in particular serine or glycine
<220>
   <221> MISC_FEATURE
   <222> (126)..(126)
   <223> Xaa can be any naturally occurring amino acid; in particular alanine or glutamic acid
<220>
   <221> MISC_FEATURE
   <222> (128)..(128)
   <223> Xaa can be any naturally occurring amino acid; in particular threonine or lysine
<220>
   <221> MISC_FEATURE
   <222> (134)..(134)
   <223> Xaa can be any naturally occurring amino acid; in particular tryptophan or isoleucin
<220>
   <221> MISC_FEATURE
   <222> (135)..(135)
   <223> Xaa can be any naturally occurring amino acid; in particular serine
<220>
   <221> MISC_FEATURE
   <222> (138)..(138)
   <223> Xaa can be any naturally occurring amino acid; in particular histidine or leucine
<220>
   <221> MISC_FEATURE
   <222> (171)..(171)
   <223> Xaa can be any naturally occurring amino acid; in particular glutamic acid or glycine
<220>
   <221> MISC_FEATURE
   <222> (173)..(173)
   <223> Xaa can be any naturally occurring amino acid; in particular tyrosine or threonine
<220>
   <221> MISC_FEATURE
   <222> (182)..(182)
   <223> Xaa can be any naturally occurring amino acid; in particular alanine or threonine
<220>
   <221> MISC_FEATURE
   <222> (187)..(187)
   <223> Xaa can be any naturally occurring amino acid; in particular aspargine or glycine
<220>
   <221> MISC_FEATURE
   <222> (197)..(197)
   <223> Xaa can be any naturally occurring amino acid; in particular aspartic acid or serine
<220>
   <221> MISC_FEATURE
   <222> (198)..(198)
   <223> Xaa can be any naturally occurring amino acid; in particular glutamine acid or alanine
<220>
   <221> MISC_FEATURE
   <222> (204)..(204)
   <223> Xaa can be any naturally occurring amino acid; in particular leucine or tyrosine
<220>
   <221> MISC_FEATURE
   <222> (230)..(230)
   <223> Xaa can be any naturally occurring amino acid; in particular phenylalanine or leucine
<220>
   <221> MISC_FEATURE
   <222> (231)..(231)
   <223> Xaa can be any naturally occurring amino acid; in particular histidine or tyrosine
<220>
   <221> MISC_FEATURE
   <222> (234)..(234)
   <223> Xaa can be any naturally occurring amino acid; in particular serine or alanine
<400> 1
   Xaa Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Xaa Gly Lys Val Ser Pro Xaa Phe Xaa
           115                 120                 125
   Ala Lys Val Lys Asp Xaa Xaa Gly Val Xaa Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Xaa Ala Xaa Gly Leu Ile
                   165                 170                 175
   Gln Phe Met Ser Pro Xaa Ala Asn Asp Leu Xaa Val Pro Leu Ser Val
               180                 185                 190
   Ile Arg Ser Met Xaa Xaa Leu Thr Gln Leu Asp Xaa Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Xaa Xaa Pro Ala Xaa Val Gly Lys Lys Ala Asp
   225                 230                 235                 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr
   290
<210> 2
   <211> 291
   <212> PRT
   <213> Artificial sequence
<220>
   <223> EL188 endolysin without N-terminal methionine
<400> 2
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 3
   <211> 292
   <212> PRT
   <213> Unknown
<220>
   <223> ELgp188
<400> 3
   Met Asn Phe Arg Thr Lys Asn Gly Tyr Arg Asp Leu Gln Ala Leu Val
   1 5 10 15
   Lys Glu Leu Gly Leu Tyr Thr Gly Gln Ile Asp Gly Val Trp Gly Lys
   20 25 30
   Gly Thr Ser Ser Ser Thr Glu Thr Leu Leu Arg Gly Tyr Ala Glu Val
   35 40 45
   Val Gly Lys Asn Thr Gly Gly Ile Gly Leu Pro Thr Thr Ser Asp Ala
   50 55 60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
       290
<210> 4
   <211> 292
   <212> PRT
   <213> Artificial Sequence
<220>
   <223> Mutated EL188 with S120G
<400> 4
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Gly Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr
   290
<210> 5
   <211> 292
   <212> PRT
   <213> Artificial Sequence
<220>
   <223> Mutated EL188 with A126E and T128K
<400> 5
   Met Asn Phe Arg Thr Lys Asn Gly Tyr Arg Asp Leu Gln Ala Leu Val
   1 5 10 15
   Lys Glu Leu Gly Leu Tyr Thr Gly Gln Ile Asp Gly Val Trp Gly Lys
   20 25 30
   Gly Thr Ser Ser Ser Thr Glu Thr Leu Leu Arg Gly Tyr Ala Glu Val
   35 40 45
   Val Gly Lys Asn Thr Gly Gly Ile Gly Leu Pro Thr Thr Ser Asp Ala
   50 55 60
   Ser Gly Tyr Asn Val Ile Thr Ala Leu Gln Arg Asn Leu Ala Phe Leu
   65 70 75 80
   Gly Leu Tyr Ser Leu Thr Val Asp Gly Ile Trp Gly Asn Gly Thr Leu
   85 90 95
   Ser Gly Leu Asp Lys Ala Phe Glu Val Tyr Lys Glu Arg Tyr Arg Thr
   100 105 110
   Pro Thr Tyr Asp Ile Ala Trp Ser Gly Lys Val Ser Pro Glu Phe Lys
   115 120 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
       290
<210> 6
   <211> 292
   <212> PRT
   <213> Artificial Sequence
<220>
   <223> Mutated EL188 with W134I
<400> 6
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Ile Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
   290
<210> 7
   <211> 292
   <212> PRT
   <213> Artificial Sequence
<220>
   <223> Mutated EL188 with H138L
<400> 7
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val Leu Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr
   290
<210> 8
   <211> 292
   <212> PRT
   <213> Artificial Sequence
<220>
   <223> Mutated EL188 with E171G
<400> 8
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
   115 120 125
   Ala Lys Val Lys Asp Trp Cys Gly Val His Val Pro Asn His Arg Ala
   130 135 140
   Pro His Trp Leu Met Ala Cys Met Ala Phe Glu Thr Gly Gln Thr Phe
   145 150 155 160
   Ser Pro Ser Ile Lys Asn Ala Ala Gly Ser Gly Ala Tyr Gly Leu Ile
   165 170 175
   Gln Phe Met Ser Pro Ala Ala Asn Asp Leu Asn Val Pro Leu Ser Val
   180 185 190
   Ile Arg Ser Met Asp Gln Leu Thr Gln Leu Asp Leu Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr
   290
<210> 9
   <211> 292
   <212> PRT
   <213> Artificial Sequence
<220>
   <223> Mutated EL188 with Y173T
<400> 9
   Met Asn Phe Arg Thr Lys Asn Gly Tyr Arg Asp Leu Gln Ala Leu Val
   1 5 10 15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Thr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
   275 280 285
   Val Ile Ser Tyr
   290
<210> 10
   <211> 292
   <212> PRT
   <213> Artificial Sequence
<220>
   <223> Mutated EL188 with A182T
<400> 10
   Met Asn Phe Arg Thr Lys Asn Gly Tyr Arg Asp Leu Gln Ala Leu Val
   1 5 10 15
   Lys Glu Leu Gly Leu Tyr Thr Gly Gln Ile Asp Gly Val Trp Gly Lys
   20 25 30
   Gly Thr Ser Ser Ser Thr Glu Thr Leu Leu Arg Gly Tyr Ala Glu Val
   35 40 45
   Val Gly Lys Asn Thr Gly Gly Ile Gly Leu Pro Thr Thr Ser Asp Ala
   50 55 60
   Ser Gly Tyr Asn Val Ile Thr Ala Leu Gln Arg Asn Leu Ala Phe Leu
   65 70 75 80
   Gly Leu Tyr Ser Leu Thr Val Asp Gly Ile Trp Gly Asn Gly Thr Leu
   85 90 95
   Ser Gly Leu Asp Lys Ala Phe Glu Val Tyr Lys Glu Arg Tyr Arg Thr
   100 105 110
   Pro Thr Tyr Asp Ile Ala Trp Ser Gly Lys Val Ser Pro Ala Phe Thr
   115 120 125
   Ala Lys Val Lys Asp Trp Cys Gly Val His Val Pro Asn His Arg Ala
   130 135 140
   Pro His Trp Leu Met Ala Cys Met Ala Phe Glu Thr Gly Gln Thr Phe
   145 150 155 160
   Ser Pro Ser Ile Lys Asn Ala Ala Gly Ser Glu Ala Tyr Gly Leu Ile
   165 170 175
   Gln Phe Met Ser Pro Thr Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
       290
<210> 11
   <211> 292
   <212> PRT
   <213> Artificial Sequence
<220>
   <223> Mutated EL188 with N187G
<400> 11
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
   85 90 95
   Ser Gly Leu Asp Lys Ala Phe Glu Val Tyr Lys Glu Arg Tyr Arg Thr
   100 105 110
   Pro Thr Tyr Asp Ile Ala Trp Ser Gly Lys Val Ser Pro Ala Phe Thr
   115 120 125
   Ala Lys Val Lys Asp Trp Cys Gly Val His Val Pro Asn His Arg Ala
   130 135 140
   Pro His Trp Leu Met Ala Cys Met Ala Phe Glu Thr Gly Gln Thr Phe
   145 150 155 160
   Ser Pro Ser Ile Lys Asn Ala Ala Gly Ser Glu Ala Tyr Gly Leu Ile
   165 170 175
   Gln Phe Met Ser Pro Ala Ala Asn Asp Leu Gly Val Pro Leu Ser Val
   180 185 190
   Ile Arg Ser Met Asp Gln Leu Thr Gln Leu Asp Leu Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr
   290
<210> 12
   <211> 292
   <212> PRT
   <213> Artificial Sequence
<220>
   <223> Mutated EL188 with D197S and Q198A
<400> 12
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Ser Ala Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
       290
<210> 13
   <211> 292
   <212> PRT
   <213> Artificial Sequence
<220>
   <223> Mutated EL188 with L204Y
<400> 13
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145 150 155 160
   Ser Pro Ser Ile Lys Asn Ala Ala Gly Ser Glu Ala Tyr Gly Leu Ile
   165 170 175
   Gln Phe Met Ser Pro Ala Ala Asn Asp Leu Asn Val Pro Leu Ser Val
   180 185 190
   Ile Arg Ser Met Asp Gln Leu Thr Gln Leu Asp Tyr Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr
   290
<210> 14
   <211> 292
   <212> PRT
   <213> Artificial Sequence
<220>
   <223> Mutated EL188 with F230L
<400> 14
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Leu His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
       290
<210> 15
   <211> 292
   <212> PRT
   <213> Artificial Sequence
<220>
   <223> Mutated EL188 with H231Y
<400> 15
   Met Asn Phe Arg Thr Lys Asn Gly Tyr Arg Asp Leu Gln Ala Leu Val
   1 5 10 15
   Lys Glu Leu Gly Leu Tyr Thr Gly Gln Ile Asp Gly Val Trp Gly Lys
   20 25 30
   Gly Thr Ser Ser Ser Thr Glu Thr Leu Leu Arg Gly Tyr Ala Glu Val
   35 40 45
   Val Gly Lys Asn Thr Gly Gly Ile Gly Leu Pro Thr Thr Ser Asp Ala
   50 55 60
   Ser Gly Tyr Asn Val Ile Thr Ala Leu Gln Arg Asn Leu Ala Phe Leu
   65 70 75 80
   Gly Leu Tyr Ser Leu Thr Val Asp Gly Ile Trp Gly Asn Gly Thr Leu
   85 90 95
   Ser Gly Leu Asp Lys Ala Phe Glu Val Tyr Lys Glu Arg Tyr Arg Thr
   100 105 110
   Pro Thr Tyr Asp Ile Ala Trp Ser Gly Lys Val Ser Pro Ala Phe Thr
   115 120 125
   Ala Lys Val Lys Asp Trp Cys Gly Val His Val Pro Asn His Arg Ala
   130 135 140
   Pro His Trp Leu Met Ala Cys Met Ala Phe Glu Thr Gly Gln Thr Phe
   145 150 155 160
   Ser Pro Ser Ile Lys Asn Ala Ala Gly Ser Glu Ala Tyr Gly Leu Ile
   165 170 175
   Gln Phe Met Ser Pro Ala Ala Asn Asp Leu Asn Val Pro Leu Ser Val
   180 185 190
   Ile Arg Ser Met Asp Gln Leu Thr Gln Leu Asp Leu Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Phe Tyr Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
       290
<210> 16
   <211> 292
   <212> PRT
   <213> Artificial Sequence
<220>
   <223> Mutated EL188 with S234A
<400> 16
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ala Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
       290
<210> 17
   <211> 292
   <212> PRT
   <213> Artificial Sequence
<220>
   <223> Mutated EL188 with C135S
<400> 17
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
   35 40 45
   Val Gly Lys Asn Thr Gly Gly Ile Gly Leu Pro Thr Thr Ser Asp Ala
   50 55 60
   Ser Gly Tyr Asn Val Ile Thr Ala Leu Gln Arg Asn Leu Ala Phe Leu
   65 70 75 80
   Gly Leu Tyr Ser Leu Thr Val Asp Gly Ile Trp Gly Asn Gly Thr Leu
   85 90 95
   Ser Gly Leu Asp Lys Ala Phe Glu Val Tyr Lys Glu Arg Tyr Arg Thr
   100 105 110
   Pro Thr Tyr Asp Ile Ala Trp Ser Gly Lys Val Ser Pro Ala Phe Thr
   115 120 125
   Ala Lys Val Lys Asp Trp Ser Gly Val His Val Pro Asn His Arg Ala
   130 135 140
   Pro His Trp Leu Met Ala Cys Met Ala Phe Glu Thr Gly Gln Thr Phe
   145 150 155 160
   Ser Pro Ser Ile Lys Asn Ala Ala Gly Ser Glu Ala Tyr Gly Leu Ile
   165 170 175
   Gln Phe Met Ser Pro Ala Ala Asn Asp Leu Asn Val Pro Leu Ser Val
   180 185 190
   Ile Arg Ser Met Asp Gln Leu Thr Gln Leu Asp Leu Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr
   290
<210> 18
   <211> 292
   <212> PRT
   <213> Artificial Sequence
<220>
   <223> Mutated EL188 with N187G and F230L
<400> 18
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Gly Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Leu His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
       290
<210> 19
   <211> 292
   <212> PRT
   <213> Artificial Sequence
<220>
   <223> Mutated EL188 with A182T, N187G and F230L
<400> 19
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
                   100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Thr Ala Asn Asp Leu Gly Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Leu His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr
       290
<210> 20
   <211> 291
   <212> PRT
   <213> Artificial Sequence
<220>
   <223> Mutated EL188 with S120G and without N-terminal methionine
<400> 20
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Gly Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 21
   <211> 291
   <212> PRT
   <213> Artificial Sequence
<220>
   <223> Mutated EL188 with A126E and T128K and without N-terminal methionine
<400> 21
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Glu Phe Lys Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 22
   <211> 291
   <212> PRT
   <213> Artificial Sequence
<220>
   <223> Mutated EL188 with W134I and without N-terminal methionine
<400> 22
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Ile Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr
       290
<210> 23
   <211> 291
   <212> PRT
   <213> Artificial Sequence
<220>
   <223> Mutated EL188 with H138L and without N-terminal methionine
<400> 23
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val Leu Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 24
   <211> 291
   <212> PRT
   <213> Artificial Sequence
<220>
   <223> Mutated EL188 with E171G and without N-terminal methionine
<400> 24
   Asn Phe Arg Thr Lys Asn Gly Tyr Arg Asp Leu Gln Ala Leu Val Lys
   1 5 10 15
   Glu Leu Gly Leu Tyr Thr Gly Gln Ile Asp Gly Val Trp Gly Lys Gly
   20 25 30
   Thr Ser Ser Ser Thr Glu Thr Leu Leu Arg Gly Tyr Ala Glu Val Val
   35 40 45
   Gly Lys Asn Thr Gly Gly Ile Gly Leu Pro Thr Thr Ser Asp Ala Ser
   50 55 60
   Gly Tyr Asn Val Ile Thr Ala Leu Gln Arg Asn Leu Ala Phe Leu Gly
   65 70 75 80
   Leu Tyr Ser Leu Thr Val Asp Gly Ile Trp Gly Asn Gly Thr Leu Ser
   85 90 95
   Gly Leu Asp Lys Ala Phe Glu Val Tyr Lys Glu Arg Tyr Arg Thr Pro
   100 105 110
   Thr Tyr Asp Ile Ala Trp Ser Gly Lys Val Ser Pro Ala Phe Thr Ala
   115 120 125
   Lys Val Lys Asp Trp Cys Gly Val His Val Pro Asn His Arg Ala Pro
   130 135 140
   His Trp Leu Met Ala Cys Met Ala Phe Glu Thr Gly Gln Thr Phe Ser
   145 150 155 160
   Pro Ser Ile Lys Asn Ala Ala Gly Ser Gly Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 25
   <211> 291
   <212> PRT
   <213> Artificial Sequence
<220>
   <223> Mutated EL188 with Y173T and without N-terminal methionine
<400> 25
   Asn Phe Arg Thr Lys Asn Gly Tyr Arg Asp Leu Gln Ala Leu Val Lys
   1 5 10 15
   Glu Leu Gly Leu Tyr Thr Gly Gln Ile Asp Gly Val Trp Gly Lys Gly
   20 25 30
   Thr Ser Ser Ser Thr Glu Thr Leu Leu Arg Gly Tyr Ala Glu Val Val
   35 40 45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Thr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr
       290
<210> 26
   <211> 291
   <212> PRT
   <213> Artificial Sequence
<220>
   <223> Mutated EL188 with A182T and without N-terminal methionine
<400> 26
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Thr Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
   210 215 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 27
   <211> 291
   <212> PRT
   <213> Artificial Sequence
<220>
   <223> Mutated EL188 with N187G and without N-terminal methionine
<400> 27
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
               115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Gly Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr
       290
<210> 28
   <211> 291
   <212> PRT
   <213> Artificial Sequence
<220>
   <223> Mutated EL188 with D197S and Q198A and without N-terminal methionine
<400> 28
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Ser Ala Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
   275 280 285
   Ile Ser Tyr
   290
<210> 29
   <211> 291
   <212> PRT
   <213> Artificial Sequence
<220>
   <223> Mutated EL188 with L204Y and without N-terminal methionine
<400> 29
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
   180 185 190
   Arg Ser Met Asp Gln Leu Thr Gln Leu Asp Tyr Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 30
   <211> 291
   <212> PRT
   <213> Artificial Sequence
<220>
   <223> Mutated EL188 with F230L and without N-terminal methionine
<400> 30
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
   100 105 110
   Thr Tyr Asp Ile Ala Trp Ser Gly Lys Val Ser Pro Ala Phe Thr Ala
   115 120 125
   Lys Val Lys Asp Trp Cys Gly Val His Val Pro Asn His Arg Ala Pro
   130 135 140
   His Trp Leu Met Ala Cys Met Ala Phe Glu Thr Gly Gln Thr Phe Ser
   145 150 155 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Leu His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 31
   <211> 291
   <212> PRT
   <213> Artificial Sequence
<220>
   <223> Mutated EL188 with H231Y and without N-terminal methionine
<400> 31
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe Tyr Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr
       290
<210> 32
   <211> 291
   <212> PRT
   <213> Artificial Sequence
<220>
   <223> Mutated EL188 with S234A and without N-terminal methionine
<400> 32
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe His Pro Ala Ala Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 33
   <211> 291
   <212> PRT
   <213> Artificial Sequence
<220>
   <223> Mutated EL188 with C135S and without N-terminal methionine
<400> 33
   Asn Phe Arg Thr Lys Asn Gly Tyr Arg Asp Leu Gln Ala Leu Val Lys
   1 5 10 15
   Glu Leu Gly Leu Tyr Thr Gly Gln Ile Asp Gly Val Trp Gly Lys Gly
   20 25 30
   Thr Ser Ser Ser Thr Glu Thr Leu Leu Arg Gly Tyr Ala Glu Val Val
   35 40 45
   Gly Lys Asn Thr Gly Gly Ile Gly Leu Pro Thr Thr Ser Asp Ala Ser
   50 55 60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Ser Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr
       290
<210> 34
   <211> 291
   <212> PRT
   <213> Artificial Sequence
<220>
   <223> Mutated EL188 with N187G and F230L and without N-terminal methionine
<400> 34
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Gly Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Leu His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr
   290
<210> 35
   <211> 291
   <212> PRT
   <213> Artificial Sequence
<220>
   <223> Mutated EL188 with A182T, N187G and F230L and without N-terminal methionine
<400> 35
   Asn Phe Arg Thr Lys Asn Gly Tyr Arg Asp Leu Gln Ala Leu Val Lys
   1 5 10 15
   Glu Leu Gly Leu Tyr Thr Gly Gln Ile Asp Gly Val Trp Gly Lys Gly
   20 25 30
   Thr Ser Ser Ser Thr Glu Thr Leu Leu Arg Gly Tyr Ala Glu Val Val
   35 40 45
   Gly Lys Asn Thr Gly Gly Ile Gly Leu Pro Thr Thr Ser Asp Ala Ser
   50 55 60
   Gly Tyr Asn Val Ile Thr Ala Leu Gln Arg Asn Leu Ala Phe Leu Gly
   65 70 75 80
   Leu Tyr Ser Leu Thr Val Asp Gly Ile Trp Gly Asn Gly Thr Leu Ser
   85 90 95
   Gly Leu Asp Lys Ala Phe Glu Val Tyr Lys Glu Arg Tyr Arg Thr Pro
   100 105 110
   Thr Tyr Asp Ile Ala Trp Ser Gly Lys Val Ser Pro Ala Phe Thr Ala
   115 120 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Thr Ala Asn Asp Leu Gly Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Leu His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr
       290
<210> 36
   <211> 6
   <212> PRT
   <213> artificial
<220>
   <223> synthetic sequence
<400> 36
   Lys Arg Lys Lys Arg Lys
   1               5
<210> 37
   <211> 5
   <212> PRT
   <213> artificial
<220>
   <223> synethtic sequence
<220>
   <221> misc_feature
   <222> (3)..(3)
   <223> Xaa can be any naturally occurring amino acid
<400> 37
   Lys Arg Xaa Lys Arg
   1               5
<210> 38
   <211> 5
   <212> PRT
   <213> artificial
<220>
   <223> synthetic sequence
<400> 38
   Lys Arg Ser Lys Arg
   1               5
<210> 39
   <211> 5
   <212> PRT
   <213> artificial
<220>
   <223> synthetic sequence
<400> 39
   Lys Arg Gly Ser Gly
   1               5
<210> 40
   <211> 9
   <212> PRT
   <213> artificial
<220>
   <223> synthetic sequence
<400> 40
   Lys Arg Lys Lys Arg Lys Lys Arg Lys
   1               5
<210> 41
   <211> 9
   <212> PRT
   <213> artificial
<220>
   <223> synthetic sequence
<400> 41
   Arg Arg Arg Arg Arg Arg Arg Arg Arg
   1               5
<210> 42
   <211> 8
   <212> PRT
   <213> artificial
<220>
   <223> synthetic sequence
<400> 42
   Lys Lys Lys Lys Lys Lys Lys Lys
   1               5
<210> 43
   <211> 10
   <212> PRT
   <213> artificial
<220>
   <223> synthetic sequence
<400> 43
   Lys Arg Lys Lys Arg Lys Lys Arg Lys Lys
   1               5                   10
<210> 44
   <211> 12
   <212> PRT
   <213> artificial
<220>
   <223> synthetic sequence
<400> 44
   Lys Arg Lys Lys Arg Lys Lys Arg Lys Lys Arg Lys
   1               5                   10
<210> 45
   <211> 14
   <212> PRT
   <213> artificial
<220>
   <223> synthetic sequence
<400> 45
   Lys Arg Lys Lys Arg Lys Lys Arg Lys Lys Arg Lys Lys Arg
   1 5 10
<210> 46
   <211> 16
   <212> PRT
   <213> artificial
<220>
   <223> synthetic sequence
<400> 46
   Lys Lys Lys Lys Lys Lys Lys Lys Lys Lys Lys Lys Lys Lys Lys Lys
   1 5 10 15
<210> 47
   <211> 18
   <212> PRT
   <213> artificial
<220>
   <223> synthetic sequence
<400> 47
   Lys Arg Lys Lys Arg Lys Lys Arg Lys Lys Arg Lys Lys Arg Lys Lys
   1 5 10 15
   Arg Lys
<210> 48
   <211> 19
   <212> PRT
   <213> artificial
<220>
   <223> synthetic sequence
<400> 48
   Lys Arg Lys Lys Arg Lys Lys Arg Lys Lys Arg Lys Lys Arg Lys Lys
   1 5 10 15
   Arg Lys Lys
<210> 49
   <211> 19
   <212> PRT
   <213> artificial
<220>
   <223> synthetic sequence
<400> 49
   Arg Arg Arg Arg Arg Arg Arg Arg Arg Arg Arg Arg Arg Arg Arg Arg
   1 5 10 15
   Arg Arg Arg
<210> 50
   <211> 19
   <212> PRT
   <213> artificial
<220>
   <223> synthetic sequence
<400> 50
   Lys Lys Lys Lys Lys Lys Lys Lys Lys Lys Lys Lys Lys Lys Lys Lys
   1 5 10 15
   Lys Lys Lys
<210> 51
   <211> 20
   <212> PRT
   <213> artificial
<220>
   <223> synthetic sequence
<400> 51
   Lys Arg Lys Lys Arg Lys Lys Arg Lys Arg Ser Lys Arg Lys Lys Arg
   1 5 10 15
   Lys Lys Arg Lys
   20
<210> 52
   <211> 21
   <212> PRT
   <213> artificial
<220>
   <223> synthetic sequence
<400> 52
   Lys Arg Lys Lys Arg Lys Lys Arg Lys Arg Ser Lys Arg Lys Lys Arg
   1 5 10 15
   Lys Lys Arg Lys Lys
   20
<210> 53
   <211> 21
   <212> PRT
   <213> artificial
<220>
   <223> synthetic sequence
<400> 53
   Lys Arg Lys Lys Arg Lys Lys Arg Lys Lys Arg Lys Lys Arg Lys Lys
   1               5                   10                  15
   Arg Lys Lys Arg Lys
               20
<210> 54
   <211> 22
   <212> PRT
   <213> artificial
<220>
   <223> synthetic sequence
<400> 54
   Lys Arg Lys Lys Arg Lys Lys Arg Lys Arg Gly Ser Gly Lys Arg Lys
   1               5                   10                  15
   Lys Arg Lys Lys Arg Lys
               20
<210> 55
   <211> 24
   <212> PRT
   <213> artificial
<220>
   <223> synthetic sequence
<400> 55
   Lys Arg Lys Lys Arg Lys Lys Arg Lys Arg Gly Ser Gly Ser Gly Lys
   1               5                   10                  15
   Arg Lys Lys Arg Lys Lys Arg Lys
               20
<210> 56
   <211> 25
   <212> PRT
   <213> artificial
<220>
   <223> synthetic sequence
<400> 56
   Lys Arg Lys Lys Arg Lys Lys Arg Lys Lys Arg Lys Lys Arg Lys Lys
   1               5                   10                  15
   Arg Lys Lys Arg Lys Lys Arg Lys Lys
               20                  25
<210> 57
   <211> 31
   <212> PRT
   <213> artificial
<220>
   <223> synthetic sequence
<400> 57
   Lys Arg Lys Lys Arg Lys Lys Arg Lys Arg Ser Lys Arg Lys Lys Arg
   1               5                   10                  15
   Lys Lys Arg Lys Arg Ser Lys Arg Lys Lys Arg Lys Lys Arg Lys
               20                  25                  30
<210> 58
   <211> 38
   <212> PRT
   <213> artificial
<220>
   <223> synthetic sequence
<400> 58
   Lys Arg Lys Lys Arg Lys Lys Arg Lys Arg Gly Ser Gly Ser Gly Lys
   1               5                   10                  15
   Arg Lys Lys Arg Lys Lys Arg Lys Gly Ser Gly Ser Gly Lys Arg Lys
               20                  25                  30
   Lys Arg Lys Lys Arg Lys
           35
<210> 59
   <211> 39
   <212> PRT
   <213> artificial
<220>
   <223> synthetic sequence
<400> 59
   Lys Arg Lys Lys Arg Lys Lys Arg Lys Lys Arg Lys Lys Arg Lys Lys
   1               5                   10                  15
   Arg Lys Lys Arg Lys Lys Arg Lys Lys Arg Lys Lys Arg Lys Lys Arg
               20                  25                  30
   Lys Lys Arg Lys Lys Arg Lys
           35
<210> 60
   <211> 42
   <212> PRT
   <213> artificial
<220>
   <223> synthetic sequence
<400> 60
   Lys Arg Lys Lys Arg Lys Lys Arg Lys Arg Ser Lys Arg Lys Lys Arg
   1               5                   10                  15
   Lys Lys Arg Lys Arg Ser Lys Arg Lys Lys Arg Lys Lys Arg Lys Arg
               20                  25                  30
   Ser Lys Arg Lys Lys Arg Lys Lys Arg Lys
           35                  40
<210> 61
   <211> 37
   <212> PRT
   <213> Homo sapiens
<400> 61
   Leu Leu Gly Asp Phe Phe Arg Lys Ser Lys Glu Lys Ile Gly Lys Glu
   1               5                   10                  15
   Phe Lys Arg Ile Val Gln Arg Ile Lys Asp Phe Leu Arg Asn Leu Val
               20                  25                  30
   Pro Arg Thr Glu Ser
           35
<210> 62
   <211> 29
   <212> PRT
   <213> unknown
<220>
   <223> SMAP-29 sheep
<400> 62
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly
               20                  25
<210> 63
   <211> 13
   <212> PRT
   <213> unknown
<220>
   <223> Indolicidine bovine
<400> 63
   Ile Leu Pro Trp Lys Trp Pro Trp Trp Pro Trp Arg Arg
   1               5                   10
<210> 64
   <211> 18
   <212> PRT
   <213> unknown
<220>
   <223> Protegrin Porcine
<400> 64
   Arg Gly Gly Arg Leu Cys Tyr Cys Arg Arg Arg Phe Cys Val Cys Val
   1               5                   10                  15
   Gly Arg
<210> 65
   <211> 31
   <212> PRT
   <213> unknown
<220>
   <223> Cecropin P1 Mammal (pig)
<400> 65
   Ser Trp Leu Ser Lys Thr Ala Lys Lys Leu Glu Asn Ser Ala Lys Lys
   1               5                   10                  15
   Arg Ile Ser Glu Gly Ile Ala Ile Ala Ile Gln Gly Gly Pro Arg
               20                  25                  30
<210> 66
   <211> 23
   <212> PRT
   <213> unknown
<220>
   <223> Magainin frog
<400> 66
   Gly Ile Gly Lys Phe Leu His Ser Ala Lys Lys Phe Gly Lys Ala Phe
   1               5                   10                  15
   Val Gly Glu Ile Met Asn Ser
               20
<210> 67
   <211> 25
   <212> PRT
   <213> unknown
<220>
   <223> Pleurocidin fish
<400> 67
   Gly Trp Gly Ser Phe Phe Lys Lys Ala Ala His Val Gly Lys His Val
   1               5                   10                  15
   Gly Lys Ala Ala Leu Thr His Tyr Leu
               20                  25
<210> 68
   <211> 36
   <212> PRT
   <213> Aedes aegypti
<400> 68
   Gly Gly Leu Lys Lys Leu Gly Lys Lys Leu Glu Gly Ala Gly Lys Arg
   1               5                   10                  15
   Val Phe Asn Ala Ala Glu Lys Ala Leu Pro Val Val Ala Gly Ala Lys
               20                  25                  30
   Ala Leu Arg Lys
           35
<210> 69
   <211> 40
   <212> PRT
   <213> Drosophila melanogaster
<400> 69
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Pro Gln Gln Ala Ala
               20                  25                  30
   Asn Val Ala Ala Thr Ala Arg Gly
           35                  40
<210> 70
   <211> 21
   <212> PRT
   <213> unknown
<220>
   <223> Buforin II vertebrate
<400> 70
   Thr Arg Ser Ser Arg Ala Gly Leu Gln Phe Pro Val Gly Arg Val His
   1               5                   10                  15
   Arg Leu Leu Arg Lys
               20
<210> 71
   <211> 39
   <212> PRT
   <213> unknown
<220>
   <223> Sarcotoxin IA Fly
<400> 71
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Ala Gln Gln Ala Ala
               20                  25                  30
   Asn Val Ala Ala Thr Ala Arg
           35
<210> 72
   <211> 17
   <212> PRT
   <213> Apis mellifera
<400> 72
   Ala Asn Arg Pro Val Tyr Ile Pro Pro Pro Arg Pro Pro His Pro Arg
   1               5                   10                  15
   Leu
<210> 73
   <211> 24
   <212> PRT
   <213> unknown
<220>
   <223> Ascaphine 5 Frog
<400> 73
   Gly Ile Lys Asp Trp Ile Lys Gly Ala Ala Lys Lys Leu Ile Lys Thr
   1               5                   10                  15
   Val Ala Ser His Ile Ala Asn Gln
               20
<210> 74
   <211> 22
   <212> PRT
   <213> unknown
<220>
   <223> Nigrocine 2 Frog
<400> 74
   Gly Leu Leu Ser Lys Val Leu Gly Val Gly Lys Lys Val Leu Cys Gly
   1               5                   10                  15
   Val Ser Gly Leu Val Cys
               20
<210> 75
   <211> 24
   <212> PRT
   <213> unknown
<220>
   <223> Pseudin 1 Rana Frog
<400> 75
   Gly Leu Asn Thr Leu Lys Lys Val Phe Gln Gly Leu His Glu Ala Ile
   1               5                   10                  15
   Lys Leu Ile Asn Asn His Val Gln
               20
<210> 76
   <211> 18
   <212> PRT
   <213> unknown
<220>
   <223> Ranalexin Frog
<400> 76
   Phe Leu Gly Gly Leu Ile Val Pro Ala Met Ile Cys Ala Val Thr Lys
   1               5                   10                  15
   Lys Cys
<210> 77
   <211> 26
   <212> PRT
   <213> unknown
<220>
   <223> Melittin bee
<400> 77
   Gly Ile Gly Ala Val Leu Lys Val Leu Thr Thr Gly Leu Pro Ala Leu
   1               5                   10                  15
   Ile Ser Trp Ile Lys Arg Lys Arg Gln Gln
               20                  25
<210> 78
   <211> 25
   <212> PRT
   <213> unknown
<220>
   <223> Lycotoxin 1 Spider
<400> 78
   Ile Trp Leu Thr Ala Leu Lys Phe Leu Gly Lys His Ala Ala Lys Lys
   1               5                   10                  15
   Leu Ala Lys Gln Gln Leu Ser Lys Leu
               20                  25
<210> 79
   <211> 19
   <212> PRT
   <213> unknown
<220>
   <223> Parasin 1 Fish
<400> 79
   Lys Gly Arg Gly Lys Gln Gly Gly Lys Val Arg Ala Lys Ala Lys Thr
   1               5                   10                  15
   Arg Ser Ser
<210> 80
   <211> 39
   <212> PRT
   <213> unknown
<220>
   <223> Buforin I Toad
<400> 80
   Ala Gly Arg Gly Lys Gln Gly Gly Lys Val Arg Ala Lys Ala Lys Thr
   1               5                   10                  15
   Arg Ser Ser Arg Ala Gly Leu Gln Phe Pro Val Gly Arg Val His Arg
               20                  25                  30
   Leu Leu Arg Lys Gly Asn Tyr
           35
<210> 81
   <211> 34
   <212> PRT
   <213> unknown
<220>
   <223> Dermaseptin 1 Frog
<400> 81
   Ala Leu Trp Lys Thr Met Leu Lys Lys Leu Gly Thr Met Ala Leu His
   1               5                   10                  15
   Ala Gly Lys Ala Ala Leu Gly Ala Ala Ala Asp Thr Ile Ser Gln Gly
               20                  25                  30
   Thr Gln
<210> 82
   <211> 12
   <212> PRT
   <213> unknown
<220>
   <223> Bactenecin 1 Cow
<400> 82
   Arg Leu Cys Arg Ile Val Val Ile Arg Val Cys Arg
   1 5 10
<210> 83
   <211> 21
   <212> PRT
   <213> unknown
<220>
   <223> Thanatin Insect
<400> 83
   Gly Ser Lys Lys Pro Val Pro Ile Ile Tyr Cys Asn Arg Arg Thr Gly
   1 5 10 15
   Lys Cys Gln Arg Met
   20
<210> 84
   <211> 19
   <212> PRT
   <213> unknown
<220>
   <223> Brevinin 1T Rana frogs
<400> 84
   Val Asn Pro Ile Ile Leu Gly Val Leu Pro Lys Val Cys Leu Ile Thr
   1 5 10 15
   Lys Lys Cys
<210> 85
   <211> 26
   <212> PRT
   <213> unknown
<220>
   <223> Ranateurin 1 Rana frog
<400> 85
   Ser Met Leu Ser Val Leu Lys Asn Leu Gly Lys Val Gly Leu Gly Phe
   1 5 10 15
   Val Ala Cys Lys Ile Asn Ile Lys Gln Cys
   20 25
<210> 86
   <211> 46
   <212> PRT
   <213> unknown
<220>
   <223> Esculentin 1 Rana frogs
<400> 86
   Gly Ile Phe Ser Lys Leu Gly Arg Lys Lys Ile Lys Asn Leu Leu Ile
   1               5                   10                  15
   Ser Gly Leu Lys Asn Val Gly Lys Glu Val Gly Met Asp Val Val Arg
               20                  25                  30
   Thr Gly Ile Lys Ile Ala Gly Cys Lys Ile Lys Gly Glu Cys
           35                  40                  45
<210> 87
   <211> 17
   <212> PRT
   <213> Limulus polyphemus
<400> 87
   Arg Trp Cys Phe Arg Val Cys Tyr Arg Gly Ile Cys Tyr Arg Lys Cys
   1               5                   10                  15
   Arg
<210> 88
   <211> 25
   <212> PRT
   <213> unknown
<220>
   <223> Androctonin Scorpion
<400> 88
   Arg Ser Val Cys Arg Gln Ile Lys Ile Cys Arg Arg Arg Gly Gly Cys
   1               5                   10                  15
   Tyr Tyr Lys Cys Thr Asn Arg Pro Tyr
               20                  25
<210> 89
   <211> 30
   <212> PRT
   <213> Homo sapiens
<400> 89
   Asp Cys Tyr Cys Arg Ile Pro Ala Cys Ile Ala Gly Glu Arg Arg Tyr
   1               5                   10                  15
   Gly Thr Cys Ile Tyr Gln Gly Arg Leu Trp Ala Phe Cys Cys
               20                  25                  30
<210> 90
   <211> 38
   <212> PRT
   <213> unknown
<220>
   <223> beta-defensin cow
<400> 90
   Asn Pro Val Ser Cys Val Arg Asn Lys Gly Ile Cys Val Pro Ile Arg
   1               5                   10                  15
   Cys Pro Gly Ser Met Lys Gln Ile Gly Thr Cys Val Gly Arg Ala Val
                   20                  25                  30
   Lys Cys Cys Arg Lys Lys
           35
<210> 91
   <211> 18
   <212> PRT
   <213> unknown
<220>
   <223> theta-defensin monkey
<400> 91
   Gly Phe Cys Arg Cys Leu Cys Arg Arg Gly Val Cys Arg Cys Ile Cys
   1               5                   10                  15
   Thr Arg
<210> 92
   <211> 40
   <212> PRT
   <213> unknown
<220>
   <223> defensin (sapecin A) insect
<400> 92
   Ala Thr Cys Asp Leu Leu Ser Gly Thr Gly Ile Asn His Ser Ala Cys
   1               5                   10                  15
   Ala Ala His Cys Leu Leu Arg Gly Asn Arg Gly Gly Tyr Cys Asn Gly
               20                  25                  30
   Lys Ala Val Cys Val Cys Arg Asn
           35                  40
<210> 93
   <211> 46
   <212> PRT
   <213> unknown
<220>
   <223> Thionin (crambin) plant
<400> 93
   Thr Thr Cys Cys Pro Ser Ile Val Ala Arg Ser Asn Phe Asn Val Cys
   1               5                   10                  15
   Arg Ile Pro Gly Thr Pro Glu Ala Ile Cys Ala Thr Tyr Thr Gly Cys
               20                  25                  30
   Ile Ile Ile Pro Gly Ala Thr Cys Pro Gly Asp Tyr Ala Asn
           35                  40                  45
<210> 94
   <211> 50
   <212> PRT
   <213> unknown
<220>
   <223> defensin from radish
<400> 94
   Gln Lys Leu Cys Gln Arg Pro Ser Gly Thr Trp Ser Gly Val Cys Gly
   1               5                   10                  15
   Asn Asn Asn Ala Cys Lys Asn Gln Cys Ile Arg Leu Glu Lys Ala Arg
               20                  25                  30
   His Gly Ser Cys Asn Tyr Val Phe Pro Ala His Cys Ile Cys Tyr Phe
           35                  40                  45
   Pro Cys
       50
<210> 95
   <211> 44
   <212> PRT
   <213> Drosophila melanogaster
<400> 95
   Asp Cys Leu Ser Gly Arg Tyr Lys Gly Pro Cys Ala Val Trp Asp Asn
   1               5                   10                  15
   Glu Thr Cys Arg Arg Val Cys Lys Glu Glu Gly Arg Ser Ser Gly His
               20                  25                  30
   Cys Ser Pro Ser Leu Lys Cys Trp Cys Glu Gly Cys
           35                  40
<210> 96
   <211> 25
   <212> PRT
   <213> Homo sapiens
<400> 96
   Asp Thr His Phe Pro Ile Cys Ile Phe Cys Cys Gly Cys Cys His Arg
   1               5                   10                  15
   Ser Lys Cys Gly Met Cys Cys Lys Thr
                       20                  25
<210> 97
   <211> 44
   <212> PRT
   <213> unknown
<220>
   <223> Bac 5 Cow
<400> 97
   Arg Phe Arg Pro Pro Ile Arg Arg Pro Pro Ile Arg Pro Pro Phe Tyr
   1               5                   10                  15
   Pro Pro Phe Arg Pro Pro Ile Arg Pro Pro Ile Phe Pro Pro Ile Arg
               20                  25                  30
   Pro Pro Phe Arg Pro Pro Leu Gly Arg Pro Phe Pro
           35                  40
<210> 98
   <211> 39
   <212> PRT
   <213> unknown
<220>
   <223> PR-39 Pig
<400> 98
   Arg Arg Arg Pro Arg Pro Pro Tyr Leu Pro Arg Pro Arg Pro Pro Pro
   1               5                   10                  15
   Phe Phe Pro Pro Arg Leu Pro Pro Arg Ile Pro Pro Gly Phe Pro Pro
               20                  25                  30
   Arg Phe Pro Pro Arg Phe Pro
           35
<210> 99
   <211> 20
   <212> PRT
   <213> unknown
<220>
   <223> Pyrrhocoricin Insect
<400> 99
   Val Asp Lys Gly Ser Tyr Leu Pro Arg Pro Thr Pro Pro Arg Pro Ile
   1               5                   10                  15
   Tyr Asn Arg Asn
               20
<210> 100
   <211> 24
   <212> PRT
   <213> Homo sapiens
<400> 100
   Asp Ser His Ala Lys Arg His His Gly Tyr Lys Arg Lys Phe His Glu
   1               5                   10                  15
   Lys His His Ser His Arg Gly Tyr
               20
<210> 101
   <211> 34
   <212> PRT
   <213> Limulus polyphemus
<400> 101
   Gly Phe Lys Leu Lys Gly Met Ala Arg Ile Ser Cys Leu Pro Asn Gly
   1 5 10 15
   Gln Trp Ser Asn Phe Pro Pro Lys Cys Ile Arg Glu Cys Ala Met Val
   20 25 30
   Ser Ser
<210> 102
   <211> 18
   <212> PRT
   <213> artificial
<220>
   <223> synthetic sequence
<400> 102
   Gly Phe Phe Ile Pro Ala Val Ile Leu Pro Ser Ile Ala Phe Leu Ile
   1 5 10 15
   Val Pro
<210> 103
   <211> 5
   <212> PRT
   <213> artificial
<220>
   <223> synthetic sequence
<400> 103
   Phe Phe Val Ala Pro
   1 5
<210> 104
   <211> 13
   <212> PRT
   <213> unknown
<220>
   <223> alpha4-helix of T4 lysozyme
<400> 104
   Pro Asn Arg Ala Lys Arg Val Ile Thr Thr Phe Arg Thr
   1 5 10
<210> 105
   <211> 27
   <212> PRT
   <213> artificial
<220>
   <223> synthetic sequence
<400> 105
   Lys Arg Trp Val Lys Arg Val Lys Arg Val Lys Arg Trp Val Lys Arg
   1               5                   10                  15
   Val Val Arg Val Val Lys Arg Trp Val Lys Arg
               20                  25
<210> 106
   <211> 25
   <212> PRT
   <213> artificial
<220>
   <223> synthetic sequence
<400> 106
   Gly Lys Pro Gly Trp Leu Ile Lys Lys Ala Leu Val Phe Lys Lys Leu
   1               5                   10                  15
   Ile Arg Arg Pro Leu Lys Arg Leu Ala
               20                  25
<210> 107
   <211> 301
   <212> PRT
   <213> Artificial Sequence
<220>
   <223> Mutated EL188 with S120G plus KRKKRKKRK peptide
<400> 107
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Gly Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr
   290 295 300
<210> 108
   <211> 301
   <212> PRT
   <213> Artificial Sequence
<220>
   <223> Mutated EL188 with A126E and T128K plus KRKKRKKRK peptide
<400> 108
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Glu Phe Lys Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr
       290                 295                 300
<210> 109
   <211> 301
   <212> PRT
   <213> Artificial Sequence
<220>
   <223> Mutated EL188 with W134I plus KRKKRKKRK peptide
<400> 109
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Ile Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr
       290                 295                 300
<210> 110
   <211> 301
   <212> PRT
   <213> Artificial Sequence
<220>
   <223> Mutated EL188 with H138L plus KRKKRKKRK peptide
<400> 110
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val Leu Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr
       290                 295                 300
<210> 111
   <211> 301
   <212> PRT
   <213> Artificial Sequence
<220>
   <223> Mutated EL188 with E171G plus KRKKRKKRK peptide
<400> 111
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Gly Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr
       290                 295                 300
<210> 112
   <211> 301
   <212> PRT
   <213> Artificial Sequence
<220>
   <223> Mutated EL188 with Y173T plus KRKKRKKRK peptide
<400> 112
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Thr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr
   290 295 300
<210> 113
   <211> 301
   <212> PRT
   <213> Artificial Sequence
<220>
   <223> Mutated EL188 with A182T plus KRKKRKKRK peptide
<400> 113
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Thr Ala
               180                 185                 190
   Asn Asp Leu Asn Val Pro Leu Ser Val Ile Arg Ser Met Asp Gln Leu
                   195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr
       290                 295                 300
<210> 114
   <211> 301
   <212> PRT
   <213> Artificial Sequence
<220>
   <223> Mutated EL188 with N187G plus KRKKRKKRK peptide
<400> 114
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Gly Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr
       290                 295                 300
<210> 115
   <211> 301
   <212> PRT
   <213> Artificial Sequence
<220>
   <223> Mutated EL188 with D197S and Q198A plus KRKKRKKRK peptide
<400> 115
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Ser Ala Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr
   290 295 300
<210> 116
   <211> 301
   <212> PRT
   <213> Artificial Sequence
<220>
   <223> Mutated EL188 with L204Y plus KRKKRKKRK peptide
<400> 116
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Cys
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Tyr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Tyr Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr
   290 295 300
<210> 117
   <211> 301
   <212> PRT
   <213> Artificial Sequence
<220>
   <223> Mutated EL188 with F230L plus KRKKRKKRK peptide
<400> 117
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Leu His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr
       290                 295                 300
<210> 118
   <211> 301
   <212> PRT
   <213> Artificial Sequence
<220>
   <223> Mutated EL188 with H231Y plus KRKKRKKRK peptide
<400> 118
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe Tyr
   225                 230                 235                 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr
   290 295 300
<210> 119
   <211> 301
   <212> PRT
   <213> Artificial Sequence
<220>
   <223> Mutated EL188 with S234A plus KRKKRKKRK peptide
<400> 119
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ala Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr
       290                 295                 300
<210> 120
   <211> 301
   <212> PRT
   <213> Artificial Sequence
<220>
   <223> Mutated EL188 with C135S plus KRKKRKKRK peptide
<400> 120
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Ser
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Tyr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr
   290 295 300
<210> 121
   <211> 324
   <212> PRT
   <213> Artificial Sequence
<220>
   <223> Mutated EL188 with A182T plus SMAP peptide
<400> 121
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Thr Ala Asn Asp Leu Asn Val Pro Leu Ser Val
       210                 215                 220
   Ile Arg Ser Met Asp Gln Leu Thr Gln Leu Asp Leu Val Phe Lys Tyr
   225 230 235 240
   Phe Glu Met Trp Met Lys Arg Gly Lys Arg Tyr Thr Gln Leu Glu Asp
   245 250 255
   Phe Tyr Leu Thr Ile Phe His Pro Ala Ser Val Gly Lys Lys Ala Asp
   260 265 270
   Glu Val Leu Phe Leu Gln Gly Ser Lys Ala Tyr Leu Gln Asn Lys Gly
   275 280 285
   Phe Asp Val Asp Lys Asp Gly Lys Ile Thr Leu Gly Glu Ile Ser Ser
   290 295 300
   Thr Leu Tyr Thr Thr Tyr Tyr Lys Gly Leu Leu Pro Glu Asn Arg His
   305 310 315 320
   Val Ile Ser Tyr
<210> 122
   <211> 324
   <212> PRT
   <213> Artificial Sequence
<220>
   <223> Mutated EL188 with N187G plus SMAP peptide
<400> 122
   Met Arg Gly Leu Arg Arg Leu Gly Arg Lys Ile Ala His Gly Val Lys
   1 5 10 15
   Lys Tyr Gly Pro Thr Val Leu Arg Ile Ile Arg Ile Ala Gly Gly Ser
   20 25 30
   Met Asn Phe Arg Thr Lys Asn Gly Tyr Arg Asp Leu Gln Ala Leu Val
   35 40 45
   Lys Glu Leu Gly Leu Tyr Thr Gly Gln Ile Asp Gly Val Trp Gly Lys
   50 55 60
   Gly Thr Ser Ser Ser Thr Glu Thr Leu Leu Arg Gly Tyr Ala Glu Val
   65 70 75 80
   Val Gly Lys Asn Thr Gly Gly Ile Gly Leu Pro Thr Thr Ser Asp Ala
   85 90 95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Ala Ala Asn Asp Leu Gly Val Pro Leu Ser Val
       210                 215                 220
   Ile Arg Ser Met Asp Gln Leu Thr Gln Leu Asp Leu Val Phe Lys Tyr
   225                 230                 235                 240
   Phe Glu Met Trp Met Lys Arg Gly Lys Arg Tyr Thr Gln Leu Glu Asp
                   245                 250                 255
   Phe Tyr Leu Thr Ile Phe His Pro Ala Ser Val Gly Lys Lys Ala Asp
               260                 265                 270
   Glu Val Leu Phe Leu Gln Gly Ser Lys Ala Tyr Leu Gln Asn Lys Gly
           275                 280                 285
   Phe Asp Val Asp Lys Asp Gly Lys Ile Thr Leu Gly Glu Ile Ser Ser
       290                 295                 300
   Thr Leu Tyr Thr Thr Tyr Tyr Lys Gly Leu Leu Pro Glu Asn Arg His
   305                 310                 315                 320
   Val Ile Ser Tyr
<210> 123
   <211> 324
   <212> PRT
   <213> Artificial Sequence
<220>
   <223> Mutated EL188 with N187G and F230L plus SMAP peptide
<400> 123
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Ala Ala Asn Asp Leu Gly Val Pro Leu Ser Val
       210                 215                 220
   Ile Arg Ser Met Asp Gln Leu Thr Gln Leu Asp Leu Val Phe Lys Tyr
   225 230 235 240
   Phe Glu Met Trp Met Lys Arg Gly Lys Arg Tyr Thr Gln Leu Glu Asp
   245 250 255
   Phe Tyr Leu Thr Ile Leu His Pro Ala Ser Val Gly Lys Lys Ala Asp
   260 265 270
   Glu Val Leu Phe Leu Gln Gly Ser Lys Ala Tyr Leu Gln Asn Lys Gly
   275 280 285
   Phe Asp Val Asp Lys Asp Gly Lys Ile Thr Leu Gly Glu Ile Ser Ser
   290 295 300
   Thr Leu Tyr Thr Thr Tyr Tyr Lys Gly Leu Leu Pro Glu Asn Arg His
   305 310 315 320
   Val Ile Ser Tyr
<210> 124
   <211> 324
   <212> PRT
   <213> Artificial Sequence
<220>
   <223> Mutated EL188 with A182T, N187G and F230L plus SMAP peptide
<400> 124
   Met Arg Gly Leu Arg Arg Leu Gly Arg Lys Ile Ala His Gly Val Lys
   1 5 10 15
   Lys Tyr Gly Pro Thr Val Leu Arg Ile Ile Arg Ile Ala Gly Gly Ser
   20 25 30
   Met Asn Phe Arg Thr Lys Asn Gly Tyr Arg Asp Leu Gln Ala Leu Val
   35 40 45
   Lys Glu Leu Gly Leu Tyr Thr Gly Gln Ile Asp Gly Val Trp Gly Lys
   50 55 60
   Gly Thr Ser Ser Ser Thr Glu Thr Leu Leu Arg Gly Tyr Ala Glu Val
   65 70 75 80
   Val Gly Lys Asn Thr Gly Gly Ile Gly Leu Pro Thr Thr Ser Asp Ala
   85 90 95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Thr Ala Asn Asp Leu Gly Val Pro Leu Ser Val
       210                 215                 220
   Ile Arg Ser Met Asp Gln Leu Thr Gln Leu Asp Leu Val Phe Lys Tyr
   225                 230                 235                 240
   Phe Glu Met Trp Met Lys Arg Gly Lys Arg Tyr Thr Gln Leu Glu Asp
                   245                 250                 255
   Phe Tyr Leu Thr Ile Leu His Pro Ala Ser Val Gly Lys Lys Ala Asp
               260                 265                 270
   Glu Val Leu Phe Leu Gln Gly Ser Lys Ala Tyr Leu Gln Asn Lys Gly
           275                 280                 285
   Phe Asp Val Asp Lys Asp Gly Lys Ile Thr Leu Gly Glu Ile Ser Ser
       290                 295                 300
   Thr Leu Tyr Thr Thr Tyr Tyr Lys Gly Leu Leu Pro Glu Asn Arg His
   305                 310                 315                 320
   Val Ile Ser Tyr
<210> 125
   <211> 300
   <212> PRT
   <213> Artificial Sequence
<220>
   <223> Mutated EL188 with S120G plus KRKKRKKRK peptide, without N-terminal methionine
<400> 125
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Gly
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 126
   <211> 300
   <212> PRT
   <213> Artificial Sequence
<220>
   <223> Mutated EL188 with A126E and T128K plus KRKKRKKRK peptide, without N-terminal methionine
<400> 126
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Glu Phe Lys Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr
       290                 295                 300
<210> 127
   <211> 300
   <212> PRT
   <213> Artificial Sequence
<220>
   <223> Mutated EL188 with W134I plus KRKKRKKRK peptide, without N-terminal methionine
<400> 127
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Ile Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 128
   <211> 300
   <212> PRT
   <213> Artificial Sequence
<220>
   <223> Mutated EL188 with H138L plus KRKKRKKRK peptide, without N-terminal methionine
<400> 128
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
   130 135 140
   Val Leu Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 129
   <211> 300
   <212> PRT
   <213> Artificial Sequence
<220>
   <223> Mutated EL188 with E171G plus KRKKRKKRK peptide, without N-terminal methionine
<400> 129
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Gly Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 130
   <211> 300
   <212> PRT
   <213> Artificial Sequence
<220>
   <223> Mutated EL188 with Y173T plus KRKKRKKRK peptide, without N-terminal methionine
<400> 130
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Thr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 131
   <211> 300
   <212> PRT
   <213> Artificial Sequence
<220>
   <223> Mutated EL188 with A182T plus KRKKRKKRK peptide, without N-terminal methionine
<400> 131
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Thr Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr
       290                 295                 300
<210> 132
   <211> 300
   <212> PRT
   <213> Artificial Sequence
<220>
   <223> Mutated EL188 with N187G plus KRKKRKKRK peptide, without N-terminal methionine
<400> 132
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Gly Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr
       290                 295                 300
<210> 133
   <211> 300
   <212> PRT
   <213> Artificial Sequence
<220>
   <223> Mutated EL188 with D197S and Q198A plus KRKKRKKRK peptide, without N-terminal methionine
<400> 133
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Ser Ala Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr
       290                 295                 300
<210> 134
   <211> 300
   <212> PRT
   <213> Artificial Sequence
<220>
   <223> Mutated EL188 with L204Y plus KRKKRKKRK peptide, without N-terminal methionine
<400> 134
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Tyr Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 135
   <211> 300
   <212> PRT
   <213> Artificial Sequence
<220>
   <223> Mutated EL188 with F230L plus KRKKRKKRK peptide, without N-terminal methionine
<400> 135
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Leu His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 136
   <211> 300
   <212> PRT
   <213> Artificial Sequence
<220>
   <223> Mutated EL188 with H231Y plus KRKKRKKRK peptide, without N-terminal methionine
<400> 136
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe Tyr Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 137
   <211> 300
   <212> PRT
   <213> Artificial Sequence
<220>
   <223> Mutated EL188 with S234A plus KRKKRKKRK peptide, without N-terminal methionine
<400> 137
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ala Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 138
   <211> 300
   <212> PRT
   <213> Artificial Sequence
<220>
   <223> Mutated EL188 with C135S plus KRKKRKKRK peptide, without N-terminal methionine
<400> 138
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Ser Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr
   290 295 300
<210> 139
   <211> 323
   <212> PRT
   <213> Artificial Sequence
<220>
   <223> Mutated EL188 with A182T plus SMAP peptide, without N-terminal methionine
<400> 139
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Met
   20 25 30
   Asn Phe Arg Thr Lys Asn Gly Tyr Arg Asp Leu Gln Ala Leu Val Lys
   35 40 45
   Glu Leu Gly Leu Tyr Thr Gly Gln Ile Asp Gly Val Trp Gly Lys Gly
   50 55 60
   Thr Ser Ser Ser Thr Glu Thr Leu Leu Arg Gly Tyr Ala Glu Val Val
   65 70 75 80
   Gly Lys Asn Thr Gly Gly Ile Gly Leu Pro Thr Thr Ser Asp Ala Ser
   85 90 95
   Gly Tyr Asn Val Ile Thr Ala Leu Gln Arg Asn Leu Ala Phe Leu Gly
   100 105 110
   Leu Tyr Ser Leu Thr Val Asp Gly Ile Trp Gly Asn Gly Thr Leu Ser
   115 120 125
   Gly Leu Asp Lys Ala Phe Glu Val Tyr Lys Glu Arg Tyr Arg Thr Pro
   130 135 140
   Thr Tyr Asp Ile Ala Trp Ser Gly Lys Val Ser Pro Ala Phe Thr Ala
   145 150 155 160
   Lys Val Lys Asp Trp Cys Gly Val His Val Pro Asn His Arg Ala Pro
   165 170 175
   His Trp Leu Met Ala Cys Met Ala Phe Glu Thr Gly Gln Thr Phe Ser
   180 185 190
   Pro Ser Ile Lys Asn Ala Ala Gly Ser Glu Ala Tyr Gly Leu Ile Gln
   195 200 205
   Phe Met Ser Pro Thr Ala Asn Asp Leu Asn Val Pro Leu Ser Val Ile
   210 215 220
   Arg Ser Met Asp Gln Leu Thr Gln Leu Asp Leu Val Phe Lys Tyr Phe
   225 230 235 240
   Glu Met Trp Met Lys Arg Gly Lys Arg Tyr Thr Gln Leu Glu Asp Phe
   245 250 255
   Tyr Leu Thr Ile Phe His Pro Ala Ser Val Gly Lys Lys Ala Asp Glu
   260 265 270
   Val Leu Phe Leu Gln Gly Ser Lys Ala Tyr Leu Gln Asn Lys Gly Phe
   275 280 285
   Asp Val Asp Lys Asp Gly Lys Ile Thr Leu Gly Glu Ile Ser Ser Thr
   290 295 300
   Leu Tyr Thr Thr Tyr Tyr Lys Gly Leu Leu Pro Glu Asn Arg His Val
   305 310 315 320
   Ile Ser Tyr
<210> 140
   <211> 323
   <212> PRT
   <213> Artificial Sequence
<220>
   <223> Mutated EL188 with N187G plus SMAP peptide, without N-terminal methionine
<400> 140
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Met
               20                  25                  30
   Asn Phe Arg Thr Lys Asn Gly Tyr Arg Asp Leu Gln Ala Leu Val Lys
           35                  40                  45
   Glu Leu Gly Leu Tyr Thr Gly Gln Ile Asp Gly Val Trp Gly Lys Gly
       50                  55                  60
   Thr Ser Ser Ser Thr Glu Thr Leu Leu Arg Gly Tyr Ala Glu Val Val
   65                  70                  75                  80
   Gly Lys Asn Thr Gly Gly Ile Gly Leu Pro Thr Thr Ser Asp Ala Ser
                   85                  90                  95
   Gly Tyr Asn Val Ile Thr Ala Leu Gln Arg Asn Leu Ala Phe Leu Gly
               100                 105                 110
   Leu Tyr Ser Leu Thr Val Asp Gly Ile Trp Gly Asn Gly Thr Leu Ser
           115                 120                 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Ala Ala Asn Asp Leu Gly Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Phe His Pro Ala Ser Val Gly Lys Lys Ala Asp Glu
                   260                 265                 270
   Val Leu Phe Leu Gln Gly Ser Lys Ala Tyr Leu Gln Asn Lys Gly Phe
           275                 280                 285
   Asp Val Asp Lys Asp Gly Lys Ile Thr Leu Gly Glu Ile Ser Ser Thr
       290                 295                 300
   Leu Tyr Thr Thr Tyr Tyr Lys Gly Leu Leu Pro Glu Asn Arg His Val
   305                 310                 315                 320
   Ile Ser Tyr
<210> 141
   <211> 323
   <212> PRT
   <213> Artificial Sequence
<220>
   <223> Mutated EL188 with N187G and F230L plus SMAP peptide, without N-terminal methionine
<400> 141
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Met
               20                  25                  30
   Asn Phe Arg Thr Lys Asn Gly Tyr Arg Asp Leu Gln Ala Leu Val Lys
           35                  40                  45
   Glu Leu Gly Leu Tyr Thr Gly Gln Ile Asp Gly Val Trp Gly Lys Gly
       50                  55                  60
   Thr Ser Ser Ser Thr Glu Thr Leu Leu Arg Gly Tyr Ala Glu Val Val
   65                  70                  75                  80
   Gly Lys Asn Thr Gly Gly Ile Gly Leu Pro Thr Thr Ser Asp Ala Ser
                   85                  90                  95
   Gly Tyr Asn Val Ile Thr Ala Leu Gln Arg Asn Leu Ala Phe Leu Gly
               100                 105                 110
   Leu Tyr Ser Leu Thr Val Asp Gly Ile Trp Gly Asn Gly Thr Leu Ser
           115                 120                 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Ala Ala Asn Asp Leu Gly Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Leu His Pro Ala Ser Val Gly Lys Lys Ala Asp Glu
               260                 265                 270
   Val Leu Phe Leu Gln Gly Ser Lys Ala Tyr Leu Gln Asn Lys Gly Phe
           275                 280                 285
   Asp Val Asp Lys Asp Gly Lys Ile Thr Leu Gly Glu Ile Ser Ser Thr
       290                 295                 300
   Leu Tyr Thr Thr Tyr Tyr Lys Gly Leu Leu Pro Glu Asn Arg His Val
   305                 310                 315                 320
   Ile Ser Tyr
<210> 142
   <211> 323
   <212> PRT
   <213> Artificial Sequence
<220>
   <223> Mutated EL188 with A182T, N187G and F230 L plus SMAP peptide, without N-terminal methionine
<400> 142
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Met
               20                  25                  30
   Asn Phe Arg Thr Lys Asn Gly Tyr Arg Asp Leu Gln Ala Leu Val Lys
           35                  40                  45
   Glu Leu Gly Leu Tyr Thr Gly Gln Ile Asp Gly Val Trp Gly Lys Gly
       50                  55                  60
   Thr Ser Ser Ser Thr Glu Thr Leu Leu Arg Gly Tyr Ala Glu Val Val
   65                  70                  75                  80
   Gly Lys Asn Thr Gly Gly Ile Gly Leu Pro Thr Thr Ser Asp Ala Ser
                   85                  90                  95
   Gly Tyr Asn Val Ile Thr Ala Leu Gln Arg Asn Leu Ala Phe Leu Gly
               100                 105                 110
   Leu Tyr Ser Leu Thr Val Asp Gly Ile Trp Gly Asn Gly Thr Leu Ser
           115                 120                 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Thr Ala Asn Asp Leu Gly Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Leu His Pro Ala Ser Val Gly Lys Lys Ala Asp Glu
   260 265 270
   Val Leu Phe Leu Gln Gly Ser Lys Ala Tyr Leu Gln Asn Lys Gly Phe
   275 280 285
   Asp Val Asp Lys Asp Gly Lys Ile Thr Leu Gly Glu Ile Ser Ser Thr
   290 295 300
   Leu Tyr Thr Thr Tyr Tyr Lys Gly Leu Leu Pro Glu Asn Arg His Val
   305 310 315 320
   Ile Ser Tyr
<210> 143
   <211> 6
   <212> PRT
   <213> artificial sequence
<220>
   <223> His-Tag (6x)
<400> 143
   His His His His His His
   1 5
<210> 144
   <211> 309
   <212> PRT
   <213> Artificial Sequence
<220>
   <223> Mutated EL188 with S120G plus KRKKRKKRK peptide plus HIS-tag
<400> 144
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Gly Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu His
       290                 295                 300
   His His His His His
   305
<210> 145
   <211> 309
   <212> PRT
   <213> Artificial Sequence
<220>
   <223> Mutated EL188 with A126E and T128K plus KRKKRKKRK peptide plus HIS-tag
<400> 145
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Glu Phe Lys Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 146
   <211> 309
   <212> PRT
   <213> Artificial Sequence
<220>
   <223> Mutated EL188 with W134I plus KRKKRKKRK peptide plus HIS-tag
<400> 146
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Ile Cys
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Tyr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 147
   <211> 309
   <212> PRT
   <213> Artificial Sequence
<220>
   <223> Mutated EL188 with H138L plus KRKKRKKRK peptide plus HIS-tag
<400> 147
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val Leu Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 148
   <211> 309
   <212> PRT
   <213> Artificial Sequence
<220>
   <223> Mutated EL188 with E171G plus KRKKRKKRK peptide plus HIS-tag
<400> 148
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Cys
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Gly Ala Tyr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 149
   <211> 309
   <212> PRT
   <213> Artificial Sequence
<220>
   <223> Mutated EL188 with Y173T plus KRKKRKKRK peptide plus HIS-tag
<400> 149
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Cys
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Thr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 150
   <211> 309
   <212> PRT
   <213> Artificial Sequence
<220>
   <223> Mutated EL188 with A182T plus KRKKRKKRK peptide plus HIS-tag
<400> 150
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Thr Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 151
   <211> 309
   <212> PRT
   <213> Artificial Sequence
<220>
   <223> Mutated EL188 with N187G plus KRKKRKKRK peptide plus HIS-tag
<400> 151
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Gly Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu His
       290                 295                 300
   His His His His His
   305
<210> 152
   <211> 309
   <212> PRT
   <213> Artificial Sequence
<220>
   <223> Mutated EL188 with D197S and Q198A plus KRKKRKKRK peptide plus HIS-tag
<400> 152
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Ser Ala Leu
           195                 200                 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 153
   <211> 309
   <212> PRT
   <213> Artificial Sequence
<220>
   <223> Mutated EL188 with L204Y plus KRKKRKKRK peptide plus HIS-tag
<400> 153
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Cys
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Tyr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Tyr Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 154
   <211> 309
   <212> PRT
   <213> Artificial Sequence
<220>
   <223> Mutated EL188 with F230L plus KRKKRKKRK peptide plus HIS-tag
<400> 154
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Leu His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 155
   <211> 309
   <212> PRT
   <213> Artificial Sequence
<220>
   <223> Mutated EL188 with H231Y plus KRKKRKKRK peptide plus HIS-tag
<400> 155
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Cys
   130 135 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe Tyr
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu His
       290                 295                 300
   His His His His His
   305
<210> 156
   <211> 309
   <212> PRT
   <213> Artificial Sequence
<220>
   <223> Mutated EL188 with S234A plus KRKKRKKRK peptide plus HIS-tag
<400> 156
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Cys
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Tyr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ala Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
       290                 295                 300
   His His His His His
   305
<210> 157
   <211> 309
   <212> PRT
   <213> Artificial Sequence
<220>
   <223> Mutated EL188 with C135S plus KRKKRKKRK peptide plus HIS-tag
<400> 157
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Ser
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu His
       290                 295                 300
   His His His His His
   305
<210> 158
   <211> 332
   <212> PRT
   <213> Artificial Sequence
<220>
   <223> Mutated EL188 with A182T plus SMAP peptide plus HIS-tag
<400> 158
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Thr Ala Asn Asp Leu Asn Val Pro Leu Ser Val
       210                 215                 220
   Ile Arg Ser Met Asp Gln Leu Thr Gln Leu Asp Leu Val Phe Lys Tyr
   225                 230                 235                 240
   Phe Glu Met Trp Met Lys Arg Gly Lys Arg Tyr Thr Gln Leu Glu Asp
                   245                 250                 255
   Phe Tyr Leu Thr Ile Phe His Pro Ala Ser Val Gly Lys Lys Ala Asp
               260                 265                 270
   Glu Val Leu Phe Leu Gln Gly Ser Lys Ala Tyr Leu Gln Asn Lys Gly
           275                 280                 285
   Phe Asp Val Asp Lys Asp Gly Lys Ile Thr Leu Gly Glu Ile Ser Ser
       290                 295                 300
   Thr Leu Tyr Thr Thr Tyr Tyr Lys Gly Leu Leu Pro Glu Asn Arg His
   305                 310                 315                 320
   Val Ile Ser Tyr Leu Glu His His His His His His
   325 330
<210> 159
   <211> 332
   <212> PRT
   <213> Artificial Sequence
<220>
   <223> Mutated EL188 with N187G plus SMAP peptide plus HIS-tag
<400> 159
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
                   195                 200                 205
   Gln Phe Met Ser Pro Ala Ala Asn Asp Leu Gly Val Pro Leu Ser Val
       210                 215                 220
   Ile Arg Ser Met Asp Gln Leu Thr Gln Leu Asp Leu Val Phe Lys Tyr
   225                 230                 235                 240
   Phe Glu Met Trp Met Lys Arg Gly Lys Arg Tyr Thr Gln Leu Glu Asp
                   245                 250                 255
   Phe Tyr Leu Thr Ile Phe His Pro Ala Ser Val Gly Lys Lys Ala Asp
               260                 265                 270
   Glu Val Leu Phe Leu Gln Gly Ser Lys Ala Tyr Leu Gln Asn Lys Gly
           275                 280                 285
   Phe Asp Val Asp Lys Asp Gly Lys Ile Thr Leu Gly Glu Ile Ser Ser
       290                 295                 300
   Thr Leu Tyr Thr Thr Tyr Tyr Lys Gly Leu Leu Pro Glu Asn Arg His
   305                 310                 315                 320
   Val Ile Ser Tyr Leu Glu His His His His His His
                   325                 330
<210> 160
   <211> 332
   <212> PRT
   <213> Artificial Sequence
<220>
   <223> Mutated EL188 with N187G and F230L plus SMAP peptide plus HIS-tag
<400> 160
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Ala Ala Asn Asp Leu Gly Val Pro Leu Ser Val
       210                 215                 220
   Ile Arg Ser Met Asp Gln Leu Thr Gln Leu Asp Leu Val Phe Lys Tyr
   225                 230                 235                 240
   Phe Glu Met Trp Met Lys Arg Gly Lys Arg Tyr Thr Gln Leu Glu Asp
                   245                 250                 255
   Phe Tyr Leu Thr Ile Leu His Pro Ala Ser Val Gly Lys Lys Ala Asp
               260                 265                 270
   Glu Val Leu Phe Leu Gln Gly Ser Lys Ala Tyr Leu Gln Asn Lys Gly
           275                 280                 285
   Phe Asp Val Asp Lys Asp Gly Lys Ile Thr Leu Gly Glu Ile Ser Ser
       290                 295                 300
   Thr Leu Tyr Thr Thr Tyr Tyr Lys Gly Leu Leu Pro Glu Asn Arg His
   305                 310                 315                 320
   Val Ile Ser Tyr Leu Glu His His His His His His
   325 330
<210> 161
   <211> 332
   <212> PRT
   <213> Artificial Sequence
<220>
   <223> Mutated EL188 with A182T, N187G and F230L plus SMAP peptide plus HIS-tag
<400> 161
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Thr Ala Asn Asp Leu Gly Val Pro Leu Ser Val
       210                 215                 220
   Ile Arg Ser Met Asp Gln Leu Thr Gln Leu Asp Leu Val Phe Lys Tyr
   225                 230                 235                 240
   Phe Glu Met Trp Met Lys Arg Gly Lys Arg Tyr Thr Gln Leu Glu Asp
                   245                 250                 255
   Phe Tyr Leu Thr Ile Leu His Pro Ala Ser Val Gly Lys Lys Ala Asp
               260                 265                 270
   Glu Val Leu Phe Leu Gln Gly Ser Lys Ala Tyr Leu Gln Asn Lys Gly
           275                 280                 285
   Phe Asp Val Asp Lys Asp Gly Lys Ile Thr Leu Gly Glu Ile Ser Ser
       290                 295                 300
   Thr Leu Tyr Thr Thr Tyr Tyr Lys Gly Leu Leu Pro Glu Asn Arg His
   305                 310                 315                 320
   Val Ile Ser Tyr Leu Glu His His His His His His
                   325                 330
<210> 162
   <211> 308
   <212> PRT
   <213> Artificial Sequence
<220>
   <223> Mutated EL188 with S120G plus KRKKRKKRK peptide plus HIS-tag without N-terminal methionine
<400> 162
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Gly
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu His His
   290 295 300
   His His His His
   305
<210> 163
   <211> 308
   <212> PRT
   <213> Artificial Sequence
<220>
   <223> Mutated EL188 with A126E and T128K plus KRKKRKKRK peptide plus HIS-tag without N-terminal methionine
<400> 163
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Glu Phe Lys Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu His His
   290 295 300
   His His His His
   305
<210> 164
   <211> 308
   <212> PRT
   <213> Artificial Sequence
<220>
   <223> Mutated EL188 with W134I plus KRKKRKKRK peptide plus HIS-tag without N-terminal methionine
<400> 164
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Ile Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu His His
   290 295 300
   His His His His
   305
<210> 165
   <211> 308
   <212> PRT
   <213> Artificial Sequence
<220>
   <223> Mutated EL188 with H138L plus KRKKRKKRK peptide plus HIS-tag without N-terminal methionine
<400> 165
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val Leu Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu His His
   290 295 300
   His His His His
   305
<210> 166
   <211> 308
   <212> PRT
   <213> Artificial Sequence
<220>
   <223> Mutated EL188 with E171G plus KRKKRKKRK peptide plus HIS-tag without N-terminal methionine
<400> 166
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Gly Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu His His
       290                 295                 300
   His His His His
   305
<210> 167
   <211> 308
   <212> PRT
   <213> Artificial Sequence
<220>
   <223> Mutated EL188 with Y173T plus KRKKRKKRK peptide plus HIS-tag without N-terminal methionine
<400> 167
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Thr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu His His
       290                 295                 300
   His His His His
   305
<210> 168
   <211> 308
   <212> PRT
   <213> Artificial Sequence
<220>
   <223> Mutated EL188 with A182T plus KRKKRKKRK peptide plus HIS-tag without N-terminal methionine
<400> 168
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Thr Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu His His
       290                 295                 300
   His His His His
   305
<210> 169
   <211> 308
   <212> PRT
   <213> Artificial Sequence
<220>
   <223> Mutated EL188 with N187G plus KRKKRKKRK peptide plus HIS-tag without N-terminal methionine
<400> 169
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Gly Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu His His
   290 295 300
   His His His His
   305
<210> 170
   <211> 308
   <212> PRT
   <213> Artificial Sequence
<220>
   <223> Mutated EL188 with D197S and Q198A plus KRKKRKKRK peptide plus HIS-tag without N-terminal methionine
<400> 170
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
                   180                 185                 190
   Asp Leu Asn Val Pro Leu Ser Val Ile Arg Ser Met Ser Ala Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu His His
       290                 295                 300
   His His His His
   305
<210> 171
   <211> 308
   <212> PRT
   <213> Artificial Sequence
<220>
   <223> Mutated EL188 with L204Y plus KRKKRKKRK peptide plus HIS-tag without N-terminal methionine
<400> 171
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Tyr Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu His His
       290                 295                 300
   His His His His
   305
<210> 172
   <211> 308
   <212> PRT
   <213> Artificial Sequence
<220>
   <223> Mutated EL188 with F230L plus KRKKRKKRK peptide plus HIS-tag without N-terminal methionine
<400> 172
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Leu His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu His His
       290                 295                 300
   His His His His
   305
<210> 173
   <211> 308
   <212> PRT
   <213> Artificial Sequence
<220>
   <223> Mutated EL188 with H231Y plus KRKKRKKRK peptide plus HIS-tag without N-terminal methionine
<400> 173
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe Tyr Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu His His
   290 295 300
   His His His His
   305
<210> 174
   <211> 308
   <212> PRT
   <213> Artificial Sequence
<220>
   <223> Mutated EL188 with S234A plus KRKKRKKRK peptide plus HIS-tag without N-terminal methionine
<400> 174
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ala Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu His His
   290 295 300
   His His His His
   305
<210> 175
   <211> 308
   <212> PRT
   <213> Artificial Sequence
<220>
   <223> Mutated EL188 with C135S plus KRKKRKKRK peptide plus HIS-tag without N-terminal methionine
<400> 175
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Ser Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu His His
   290 295 300
   His His His His
   305
<210> 176
   <211> 331
   <212> PRT
   <213> Artificial Sequence
<220>
   <223> Mutated EL188 with A182T plus SMAP peptide plus HIS-tag without N-terminal methionine
<400> 176
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Met
               20                  25                  30
   Asn Phe Arg Thr Lys Asn Gly Tyr Arg Asp Leu Gln Ala Leu Val Lys
           35                  40                  45
   Glu Leu Gly Leu Tyr Thr Gly Gln Ile Asp Gly Val Trp Gly Lys Gly
       50                  55                  60
   Thr Ser Ser Ser Thr Glu Thr Leu Leu Arg Gly Tyr Ala Glu Val Val
   65                  70                  75                  80
   Gly Lys Asn Thr Gly Gly Ile Gly Leu Pro Thr Thr Ser Asp Ala Ser
                   85                  90                  95
   Gly Tyr Asn Val Ile Thr Ala Leu Gln Arg Asn Leu Ala Phe Leu Gly
               100                 105                 110
   Leu Tyr Ser Leu Thr Val Asp Gly Ile Trp Gly Asn Gly Thr Leu Ser
           115                 120                 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Thr Ala Asn Asp Leu Asn Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Phe His Pro Ala Ser Val Gly Lys Lys Ala Asp Glu
               260                 265                 270
   Val Leu Phe Leu Gln Gly Ser Lys Ala Tyr Leu Gln Asn Lys Gly Phe
           275                 280                 285
   Asp Val Asp Lys Asp Gly Lys Ile Thr Leu Gly Glu Ile Ser Ser Thr
       290                 295                 300
   Leu Tyr Thr Thr Tyr Tyr Lys Gly Leu Leu Pro Glu Asn Arg His Val
   305                 310                 315                 320
   Ile Ser Tyr Leu Glu His His His His His His
                   325                 330
<210> 177
   <211> 331
   <212> PRT
   <213> Artificial Sequence
<220>
   <223> Mutated EL188 with N187G plus SMAP peptide plus HIS-tag without N-terminal methionine
<400> 177
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Met
               20                  25                  30
   Asn Phe Arg Thr Lys Asn Gly Tyr Arg Asp Leu Gln Ala Leu Val Lys
           35                  40                  45
   Glu Leu Gly Leu Tyr Thr Gly Gln Ile Asp Gly Val Trp Gly Lys Gly
       50                  55                  60
   Thr Ser Ser Ser Thr Glu Thr Leu Leu Arg Gly Tyr Ala Glu Val Val
   65                  70                  75                  80
   Gly Lys Asn Thr Gly Gly Ile Gly Leu Pro Thr Thr Ser Asp Ala Ser
                   85                  90                  95
   Gly Tyr Asn Val Ile Thr Ala Leu Gln Arg Asn Leu Ala Phe Leu Gly
               100                 105                 110
   Leu Tyr Ser Leu Thr Val Asp Gly Ile Trp Gly Asn Gly Thr Leu Ser
           115                 120                 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Ala Ala Asn Asp Leu Gly Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Phe His Pro Ala Ser Val Gly Lys Lys Ala Asp Glu
               260                 265                 270
   Val Leu Phe Leu Gln Gly Ser Lys Ala Tyr Leu Gln Asn Lys Gly Phe
           275                 280                 285
   Asp Val Asp Lys Asp Gly Lys Ile Thr Leu Gly Glu Ile Ser Ser Thr
       290                 295                 300
   Leu Tyr Thr Thr Tyr Tyr Lys Gly Leu Leu Pro Glu Asn Arg His Val
   305                 310                 315                 320
   Ile Ser Tyr Leu Glu His His His His His His
                   325                 330
<210> 178
   <211> 331
   <212> PRT
   <213> Artificial Sequence
<220>
   <223> Mutated EL188 with N187G and F230L plus SMAP peptide plus HIS-tag without N-terminal methionine
<400> 178
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Met
               20                  25                  30
   Asn Phe Arg Thr Lys Asn Gly Tyr Arg Asp Leu Gln Ala Leu Val Lys
           35                  40                  45
   Glu Leu Gly Leu Tyr Thr Gly Gln Ile Asp Gly Val Trp Gly Lys Gly
       50                  55                  60
   Thr Ser Ser Ser Thr Glu Thr Leu Leu Arg Gly Tyr Ala Glu Val Val
   65                  70                  75                  80
   Gly Lys Asn Thr Gly Gly Ile Gly Leu Pro Thr Thr Ser Asp Ala Ser
                   85                  90                  95
   Gly Tyr Asn Val Ile Thr Ala Leu Gln Arg Asn Leu Ala Phe Leu Gly
               100                 105                 110
   Leu Tyr Ser Leu Thr Val Asp Gly Ile Trp Gly Asn Gly Thr Leu Ser
           115                 120                 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Ala Ala Asn Asp Leu Gly Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Leu His Pro Ala Ser Val Gly Lys Lys Ala Asp Glu
                   260                 265                 270
   Val Leu Phe Leu Gln Gly Ser Lys Ala Tyr Leu Gln Asn Lys Gly Phe
           275                 280                 285
   Asp Val Asp Lys Asp Gly Lys Ile Thr Leu Gly Glu Ile Ser Ser Thr
       290                 295                 300
   Leu Tyr Thr Thr Tyr Tyr Lys Gly Leu Leu Pro Glu Asn Arg His Val
   305                 310                 315                 320
   Ile Ser Tyr Leu Glu His His His His His His
                   325                 330
<210> 179
   <211> 331
   <212> PRT
   <213> Artificial Sequence
<220>
   <223> Mutated EL188 with A182T, N187G and F230L plus SMAP peptide plus HIS-tag without N-terminal methionine
<400> 179
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Met
               20                  25                  30
   Asn Phe Arg Thr Lys Asn Gly Tyr Arg Asp Leu Gln Ala Leu Val Lys
           35                  40                  45
   Glu Leu Gly Leu Tyr Thr Gly Gln Ile Asp Gly Val Trp Gly Lys Gly
       50                  55                  60
   Thr Ser Ser Ser Thr Glu Thr Leu Leu Arg Gly Tyr Ala Glu Val Val
   65                  70                  75                  80
   Gly Lys Asn Thr Gly Gly Ile Gly Leu Pro Thr Thr Ser Asp Ala Ser
                   85                  90                  95
   Gly Tyr Asn Val Ile Thr Ala Leu Gln Arg Asn Leu Ala Phe Leu Gly
               100                 105                 110
   Leu Tyr Ser Leu Thr Val Asp Gly Ile Trp Gly Asn Gly Thr Leu Ser
           115                 120                 125
   Gly Leu Asp Lys Ala Phe Glu Val Tyr Lys Glu Arg Tyr Arg Thr Pro
   130 135 140
   Thr Tyr Asp Ile Ala Trp Ser Gly Lys Val Ser Pro Ala Phe Thr Ala
   145 150 155 160
   Lys Val Lys Asp Trp Cys Gly Val His Val Pro Asn His Arg Ala Pro
   165 170 175
   His Trp Leu Met Ala Cys Met Ala Phe Glu Thr Gly Gln Thr Phe Ser
   180 185 190
   Pro Ser Ile Lys Asn Ala Ala Gly Ser Glu Ala Tyr Gly Leu Ile Gln
   195 200 205
   Phe Met Ser Pro Thr Ala Asn Asp Leu Gly Val Pro Leu Ser Val Ile
   210 215 220
   Arg Ser Met Asp Gln Leu Thr Gln Leu Asp Leu Val Phe Lys Tyr Phe
   225 230 235 240
   Glu Met Trp Met Lys Arg Gly Lys Arg Tyr Thr Gln Leu Glu Asp Phe
   245 250 255
   Tyr Leu Thr Ile Leu His Pro Ala Ser Val Gly Lys Lys Ala Asp Glu
   260 265 270
   Val Leu Phe Leu Gln Gly Ser Lys Ala Tyr Leu Gln Asn Lys Gly Phe
   275 280 285
   Asp Val Asp Lys Asp Gly Lys Ile Thr Leu Gly Glu Ile Ser Ser Thr
   290 295 300
   Leu Tyr Thr Thr Tyr Tyr Lys Gly Leu Leu Pro Glu Asn Arg His Val
   305 310 315 320
   Ile Ser Tyr Leu Glu His His His His His His
   325 330
<210> 180
   <211> 309
   <212> PRT
   <213> Artificial Sequence
<220>
   <223> EL188 plus KRKKRKKRK peptide plus HIS-tag
<400> 180
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu His
   290 295 300
   His His His His His
   305
<210> 181
   <211> 326
   <212> PRT
   <213> Artificial Sequence
<220>
   <223> EL188 plus SMAP peptide plus HIS-tag without linker
<400> 181
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
   165 170 175
   Pro His Trp Leu Met Ala Cys Met Ala Phe Glu Thr Gly Gln Thr Phe
   180 185 190
   Ser Pro Ser Ile Lys Asn Ala Ala Gly Ser Glu Ala Tyr Gly Leu Ile
   195 200 205
   Gln Phe Met Ser Pro Ala Ala Asn Asp Leu Asn Val Pro Leu Ser Val
   210 215 220
   Ile Arg Ser Met Asp Gln Leu Thr Gln Leu Asp Leu Val Phe Lys Tyr
   225 230 235 240
   Phe Glu Met Trp Met Lys Arg Gly Lys Arg Tyr Thr Gln Leu Glu Asp
   245 250 255
   Phe Tyr Leu Thr Ile Phe His Pro Ala Ser Val Gly Lys Lys Ala Asp
   260 265 270
   Glu Val Leu Phe Leu Gln Gly Ser Lys Ala Tyr Leu Gln Asn Lys Gly
   275 280 285
   Phe Asp Val Asp Lys Asp Gly Lys Ile Thr Leu Gly Glu Ile Ser Ser
   290 295 300
   Thr Leu Tyr Thr Thr Tyr Tyr Lys Gly Leu Leu Pro Glu Asn Arg His
   305 310 315 320
   Val Ile Ser Tyr Leu Glu
   325
<210> 182
   <211> 339
   <212> PRT
   <213> Artificial Sequence
<220>
   <223> EL188 plus SMAP peptide plus HIS-tag with GAGAGAGA-linker
<400> 182
   Met Arg Gly Leu Arg Arg Leu Gly Arg Lys Ile Ala His Gly Val Lys
   1 5 10 15
   Lys Tyr Gly Pro Thr Val Leu Arg Ile Ile Arg Ile Ala Gly Gly Ser
   20 25 30
   Gly Ala Gly Ala Gly Ala Gly Ala Asn Phe Arg Thr Lys Asn Gly Tyr
           35                  40                  45
   Arg Asp Leu Gln Ala Leu Val Lys Glu Leu Gly Leu Tyr Thr Gly Gln
       50                  55                  60
   Ile Asp Gly Val Trp Gly Lys Gly Thr Ser Ser Ser Thr Glu Thr Leu
   65                  70                  75                  80
   Leu Arg Gly Tyr Ala Glu Val Val Gly Lys Asn Thr Gly Gly Ile Gly
                   85                  90                  95
   Leu Pro Thr Thr Ser Asp Ala Ser Gly Tyr Asn Val Ile Thr Ala Leu
               100                 105                 110
   Gln Arg Asn Leu Ala Phe Leu Gly Leu Tyr Ser Leu Thr Val Asp Gly
           115                 120                 125
   Ile Trp Gly Asn Gly Thr Leu Ser Gly Leu Asp Lys Ala Phe Glu Val
       130                 135                 140
   Tyr Lys Glu Arg Tyr Arg Thr Pro Thr Tyr Asp Ile Ala Trp Ser Gly
   145                 150                 155                 160
   Lys Val Ser Pro Ala Phe Thr Ala Lys Val Lys Asp Trp Cys Gly Val
                   165                 170                 175
   His Val Pro Asn His Arg Ala Pro His Trp Leu Met Ala Cys Met Ala
               180                 185                 190
   Phe Glu Thr Gly Gln Thr Phe Ser Pro Ser Ile Lys Asn Ala Ala Gly
           195                 200                 205
   Ser Glu Ala Tyr Gly Leu Ile Gln Phe Met Ser Pro Ala Ala Asn Asp
       210                 215                 220
   Leu Asn Val Pro Leu Ser Val Ile Arg Ser Met Asp Gln Leu Thr Gln
   225                 230                 235                 240
   Leu Asp Leu Val Phe Lys Tyr Phe Glu Met Trp Met Lys Arg Gly Lys
                   245                 250                 255
   Arg Tyr Thr Gln Leu Glu Asp Phe Tyr Leu Thr Ile Phe His Pro Ala
               260                 265                 270
   Ser Val Gly Lys Lys Ala Asp Glu Val Leu Phe Leu Gln Gly Ser Lys
           275                 280                 285
   Ala Tyr Leu Gln Asn Lys Gly Phe Asp Val Asp Lys Asp Gly Lys Ile
       290                 295                 300
   Thr Leu Gly Glu Ile Ser Ser Thr Leu Tyr Thr Thr Tyr Tyr Lys Gly
   305                 310                 315                 320
   Leu Leu Pro Glu Asn Arg His Val Ile Ser Tyr Leu Glu His His His
                   325                 330                 335
   His His His
<210> 183
   <211> 290
   <212> PRT
   <213> Artificial Sequence
<220>
   <223> EL188 with L99 deletion without N-terminal Met
<400> 183
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Asp Lys Ala Phe Glu Val Tyr Lys Glu Arg Tyr Arg Thr Pro Thr
               100                 105                 110
   Tyr Asp Ile Ala Trp Ser Gly Lys Val Ser Pro Ala Phe Thr Ala Lys
           115                 120                 125
   Val Lys Asp Trp Cys Gly Val His Val Pro Asn His Arg Ala Pro His
       130                 135                 140
   Trp Leu Met Ala Cys Met Ala Phe Glu Thr Gly Gln Thr Phe Ser Pro
   145 150 155 160
   Ser Ile Lys Asn Ala Ala Gly Ser Glu Ala Tyr Gly Leu Ile Gln Phe
   165 170 175
   Met Ser Pro Ala Ala Asn Asp Leu Asn Val Pro Leu Ser Val Ile Arg
   180 185 190
   Ser Met Asp Gln Leu Thr Gln Leu Asp Leu Val Phe Lys Tyr Phe Glu
   195 200 205
   Met Trp Met Lys Arg Gly Lys Arg Tyr Thr Gln Leu Glu Asp Phe Tyr
   210 215 220
   Leu Thr Ile Phe His Pro Ala Ser Val Gly Lys Lys Ala Asp Glu Val
   225 230 235 240
   Leu Phe Leu Gln Gly Ser Lys Ala Tyr Leu Gln Asn Lys Gly Phe Asp
   245 250 255
   Val Asp Lys Asp Gly Lys Ile Thr Leu Gly Glu Ile Ser Ser Thr Leu
   260 265 270
   Tyr Thr Thr Tyr Tyr Lys Gly Leu Leu Pro Glu Asn Arg His Val Ile
   275 280 285
   Ser Tyr
   290
<210> 184
   <211> 294
   <212> PRT
   <213> Artificial Sequence
<220>
   <223> Mutated EL188 with S120G
<400> 184
   Met Asn Phe Arg Thr Lys Asn Gly Tyr Arg Asp Leu Gln Ala Leu Val
   1 5 10 15
   Lys Glu Leu Gly Leu Tyr Thr Gly Gln Ile Asp Gly Val Trp Gly Lys
   20 25 30
   Gly Thr Ser Ser Ser Thr Glu Thr Leu Leu Arg Gly Tyr Ala Glu Val
   35 40 45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Gly Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 185
   <211> 294
   <212> PRT
   <213> Artificial Sequence
<220>
   <223> Mutated EL188 with A126E and T128K
<400> 185
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Glu Phe Lys
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 186
   <211> 294
   <212> PRT
   <213> Artificial Sequence
<220>
   <223> Mutated EL188 with W134I
<400> 186
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
               115                 120                 125
   Ala Lys Val Lys Asp Ile Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 187
   <211> 294
   <212> PRT
   <213> Artificial Sequence
<220>
   <223> Mutated EL188 with H138L
<400> 187
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val Leu Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
   275 280 285
   Val Ile Ser Tyr Leu Glu
   290
<210> 188
   <211> 294
   <212> PRT
   <213> Artificial Sequence
<220>
   <223> Mutated EL188 with E171G
<400> 188
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Gly Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr Leu Glu
   290
<210> 189
   <211> 294
   <212> PRT
   <213> Artificial Sequence
<220>
   <223> Mutated EL188 with Y173T
<400> 189
   Met Asn Phe Arg Thr Lys Asn Gly Tyr Arg Asp Leu Gln Ala Leu Val
   1 5 10 15
   Lys Glu Leu Gly Leu Tyr Thr Gly Gln Ile Asp Gly Val Trp Gly Lys
   20 25 30
   Gly Thr Ser Ser Ser Thr Glu Thr Leu Leu Arg Gly Tyr Ala Glu Val
   35 40 45
   Val Gly Lys Asn Thr Gly Gly Ile Gly Leu Pro Thr Thr Ser Asp Ala
   50 55 60
   Ser Gly Tyr Asn Val Ile Thr Ala Leu Gln Arg Asn Leu Ala Phe Leu
   65 70 75 80
   Gly Leu Tyr Ser Leu Thr Val Asp Gly Ile Trp Gly Asn Gly Thr Leu
   85 90 95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Thr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 190
   <211> 294
   <212> PRT
   <213> Artificial Sequence
<220>
   <223> Mutated EL188 with A182T
<400> 190
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Thr Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 191
   <211> 294
   <212> PRT
   <213> Artificial Sequence
<220>
   <223> Mutated EL188 with N187G
<400> 191
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Gly Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 192
   <211> 294
   <212> PRT
   <213> Artificial Sequence
<220>
   <223> Mutated EL188 with D197S and Q198A
<400> 192
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65 70 75 80
   Gly Leu Tyr Ser Leu Thr Val Asp Gly Ile Trp Gly Asn Gly Thr Leu
   85 90 95
   Ser Gly Leu Asp Lys Ala Phe Glu Val Tyr Lys Glu Arg Tyr Arg Thr
   100 105 110
   Pro Thr Tyr Asp Ile Ala Trp Ser Gly Lys Val Ser Pro Ala Phe Thr
   115 120 125
   Ala Lys Val Lys Asp Trp Cys Gly Val His Val Pro Asn His Arg Ala
   130 135 140
   Pro His Trp Leu Met Ala Cys Met Ala Phe Glu Thr Gly Gln Thr Phe
   145 150 155 160
   Ser Pro Ser Ile Lys Asn Ala Ala Gly Ser Glu Ala Tyr Gly Leu Ile
   165 170 175
   Gln Phe Met Ser Pro Ala Ala Asn Asp Leu Asn Val Pro Leu Ser Val
   180 185 190
   Ile Arg Ser Met Ser Ala Leu Thr Gln Leu Asp Leu Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr Leu Glu
   290
<210> 193
   <211> 294
   <212> PRT
   <213> Artificial Sequence
<220>
   <223> Mutated EL188 with L204Y
<400> 193
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Tyr Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr Leu Glu
   290
<210> 194
   <211> 294
   <212> PRT
   <213> Artificial Sequence
<220>
   <223> Mutated EL188 with F230L
<400> 194
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Leu His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 195
   <211> 294
   <212> PRT
   <213> Artificial Sequence
<220>
   <223> Mutated EL188 with H231Y
<400> 195
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe Tyr Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 196
   <211> 294
   <212> PRT
   <213> Artificial Sequence
<220>
   <223> Mutated EL188 with S234A
<400> 196
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ala Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 197
   <211> 294
   <212> PRT
   <213> Artificial Sequence
<220>
   <223> Mutated EL188 with C135S
<400> 197
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
   115 120 125
   Ala Lys Val Lys Asp Trp Ser Gly Val His Val Pro Asn His Arg Ala
   130 135 140
   Pro His Trp Leu Met Ala Cys Met Ala Phe Glu Thr Gly Gln Thr Phe
   145 150 155 160
   Ser Pro Ser Ile Lys Asn Ala Ala Gly Ser Glu Ala Tyr Gly Leu Ile
   165 170 175
   Gln Phe Met Ser Pro Ala Ala Asn Asp Leu Asn Val Pro Leu Ser Val
   180 185 190
   Ile Arg Ser Met Asp Gln Leu Thr Gln Leu Asp Leu Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Phe His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr Leu Glu
   290
<210> 198
   <211> 294
   <212> PRT
   <213> Artificial Sequence
<220>
   <223> Mutated EL188 with N187G and F230L
<400> 198
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Gly Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Leu His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
               260                 265                 270
   Thr Leu Tyr Thr Thr Tyr Tyr Lys Gly Leu Leu Pro Glu Asn Arg His
           275                 280                 285
   Val Ile Ser Tyr Leu Glu
       290
<210> 199
   <211> 294
   <212> PRT
   <213> Artificial Sequence
<220>
   <223> Mutated EL188 with A182T, N187G and F230L
<400> 199
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Thr Ala Asn Asp Leu Gly Val Pro Leu Ser Val
   180 185 190
   Ile Arg Ser Met Asp Gln Leu Thr Gln Leu Asp Leu Val Phe Lys Tyr
   195 200 205
   Phe Glu Met Trp Met Lys Arg Gly Lys Arg Tyr Thr Gln Leu Glu Asp
   210 215 220
   Phe Tyr Leu Thr Ile Leu His Pro Ala Ser Val Gly Lys Lys Ala Asp
   225 230 235 240
   Glu Val Leu Phe Leu Gln Gly Ser Lys Ala Tyr Leu Gln Asn Lys Gly
   245 250 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr Leu Glu
   290
<210> 200
   <211> 293
   <212> PRT
   <213> Artificial Sequence
<220>
   <223> Mutated EL188 with S120G and without N-terminal methionine
<400> 200
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
   100 105 110
   Thr Tyr Asp Ile Ala Trp Gly Gly Lys Val Ser Pro Ala Phe Thr Ala
   115 120 125
   Lys Val Lys Asp Trp Cys Gly Val His Val Pro Asn His Arg Ala Pro
   130 135 140
   His Trp Leu Met Ala Cys Met Ala Phe Glu Thr Gly Gln Thr Phe Ser
   145 150 155 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Leu Glu
   290
<210> 201
   <211> 293
   <212> PRT
   <213> Artificial Sequence
<220>
   <223> Mutated EL188 with A126E and T128K and without N-terminal methionine
<400> 201
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Glu Phe Lys Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Leu Glu
   290
<210> 202
   <211> 293
   <212> PRT
   <213> Artificial Sequence
<220>
   <223> Mutated EL188 with W134I and without N-terminal methionine
<400> 202
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Ile Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr Leu Glu
       290
<210> 203
   <211> 293
   <212> PRT
   <213> Artificial Sequence
<220>
   <223> Mutated EL188 with H138L and without N-terminal methionine
<400> 203
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val Leu Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr Leu Glu
       290
<210> 204
   <211> 293
   <212> PRT
   <213> Artificial Sequence
<220>
   <223> Mutated EL188 with E171G and without N-terminal methionine
<400> 204
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Gly Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Leu Glu
   290
<210> 205
   <211> 293
   <212> PRT
   <213> Artificial Sequence
<220>
   <223> Mutated EL188 with Y173T and without N-terminal methionine
<400> 205
   Asn Phe Arg Thr Lys Asn Gly Tyr Arg Asp Leu Gln Ala Leu Val Lys
   1 5 10 15
   Glu Leu Gly Leu Tyr Thr Gly Gln Ile Asp Gly Val Trp Gly Lys Gly
   20 25 30
   Thr Ser Ser Ser Thr Glu Thr Leu Leu Arg Gly Tyr Ala Glu Val Val
   35 40 45
   Gly Lys Asn Thr Gly Gly Ile Gly Leu Pro Thr Thr Ser Asp Ala Ser
   50 55 60
   Gly Tyr Asn Val Ile Thr Ala Leu Gln Arg Asn Leu Ala Phe Leu Gly
   65 70 75 80
   Leu Tyr Ser Leu Thr Val Asp Gly Ile Trp Gly Asn Gly Thr Leu Ser
   85 90 95
   Gly Leu Asp Lys Ala Phe Glu Val Tyr Lys Glu Arg Tyr Arg Thr Pro
   100 105 110
   Thr Tyr Asp Ile Ala Trp Ser Gly Lys Val Ser Pro Ala Phe Thr Ala
   115 120 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Thr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr Leu Glu
       290
<210> 206
   <211> 293
   <212> PRT
   <213> Artificial Sequence
<220>
   <223> Mutated EL188 with A182T and without N-terminal methionine
<400> 206
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
               35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Thr Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr Leu Glu
   290
<210> 207
   <211> 293
   <212> PRT
   <213> Artificial Sequence
<220>
   <223> Mutated EL188 with N187G and without N-terminal methionine
<400> 207
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Gly Val Pro Leu Ser Val Ile
               180                 185                 190
   Arg Ser Met Asp Gln Leu Thr Gln Leu Asp Leu Val Phe Lys Tyr Phe
               195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr Leu Glu
       290
<210> 208
   <211> 293
   <212> PRT
   <213> Artificial Sequence
<220>
   <223> Mutated EL188 with D197S and Q198A and without N-terminal methionine
<400> 208
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Ser Ala Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr Leu Glu
       290
<210> 209
   <211> 293
   <212> PRT
   <213> Artificial Sequence
<220>
   <223> Mutated EL188 with L204Y and without N-terminal methionine
<400> 209
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Tyr Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Leu Glu
   290
<210> 210
   <211> 293
   <212> PRT
   <213> Artificial Sequence
<220>
   <223> Mutated EL188 with F230L and without N-terminal methionine
<400> 210
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Leu His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Leu Glu
   290
<210> 211
   <211> 293
   <212> PRT
   <213> Artificial Sequence
<220>
   <223> Mutated EL188 with H231Y and without N-terminal methionine
<400> 211
   Asn Phe Arg Thr Lys Asn Gly Tyr Arg Asp Leu Gln Ala Leu Val Lys
   1 5 10 15
   Glu Leu Gly Leu Tyr Thr Gly Gln Ile Asp Gly Val Trp Gly Lys Gly
   20 25 30
   Thr Ser Ser Ser Thr Glu Thr Leu Leu Arg Gly Tyr Ala Glu Val Val
   35 40 45
   Gly Lys Asn Thr Gly Gly Ile Gly Leu Pro Thr Thr Ser Asp Ala Ser
   50 55 60
   Gly Tyr Asn Val Ile Thr Ala Leu Gln Arg Asn Leu Ala Phe Leu Gly
   65 70 75 80
   Leu Tyr Ser Leu Thr Val Asp Gly Ile Trp Gly Asn Gly Thr Leu Ser
   85 90 95
   Gly Leu Asp Lys Ala Phe Glu Val Tyr Lys Glu Arg Tyr Arg Thr Pro
   100 105 110
   Thr Tyr Asp Ile Ala Trp Ser Gly Lys Val Ser Pro Ala Phe Thr Ala
   115 120 125
   Lys Val Lys Asp Trp Cys Gly Val His Val Pro Asn His Arg Ala Pro
   130 135 140
   His Trp Leu Met Ala Cys Met Ala Phe Glu Thr Gly Gln Thr Phe Ser
   145 150 155 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe Tyr Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Leu Glu
   290
<210> 212
   <211> 293
   <212> PRT
   <213> Artificial Sequence
<220>
   <223> Mutated EL188 with S234A and without N-terminal methionine
<400> 212
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ala Val Gly Lys Lys Ala Asp Glu
   225                 230                 235                 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Leu Glu
   290
<210> 213
   <211> 293
   <212> PRT
   <213> Artificial Sequence
<220>
   <223> Mutated EL188 with C135S and without N-terminal methionine
<400> 213
   Asn Phe Arg Thr Lys Asn Gly Tyr Arg Asp Leu Gln Ala Leu Val Lys
   1 5 10 15
   Glu Leu Gly Leu Tyr Thr Gly Gln Ile Asp Gly Val Trp Gly Lys Gly
   20 25 30
   Thr Ser Ser Ser Thr Glu Thr Leu Leu Arg Gly Tyr Ala Glu Val Val
   35 40 45
   Gly Lys Asn Thr Gly Gly Ile Gly Leu Pro Thr Thr Ser Asp Ala Ser
   50 55 60
   Gly Tyr Asn Val Ile Thr Ala Leu Gln Arg Asn Leu Ala Phe Leu Gly
   65 70 75 80
   Leu Tyr Ser Leu Thr Val Asp Gly Ile Trp Gly Asn Gly Thr Leu Ser
   85 90 95
   Gly Leu Asp Lys Ala Phe Glu Val Tyr Lys Glu Arg Tyr Arg Thr Pro
   100 105 110
   Thr Tyr Asp Ile Ala Trp Ser Gly Lys Val Ser Pro Ala Phe Thr Ala
   115 120 125
   Lys Val Lys Asp Trp Ser Gly Val His Val Pro Asn His Arg Ala Pro
   130 135 140
   His Trp Leu Met Ala Cys Met Ala Phe Glu Thr Gly Gln Thr Phe Ser
   145 150 155 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Leu Glu
   290
<210> 214
   <211> 293
   <212> PRT
   <213> Artificial Sequence
<220>
   <223> Mutated EL188 with N187G and F230L and without N-terminal methionine
<400> 214
   Asn Phe Arg Thr Lys Asn Gly Tyr Arg Asp Leu Gln Ala Leu Val Lys
   1 5 10 15
   Glu Leu Gly Leu Tyr Thr Gly Gln Ile Asp Gly Val Trp Gly Lys Gly
   20 25 30
   Thr Ser Ser Ser Thr Glu Thr Leu Leu Arg Gly Tyr Ala Glu Val Val
   35 40 45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Gly Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Leu His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr Leu Glu
       290
<210> 215
   <211> 293
   <212> PRT
   <213> Artificial Sequence
<220>
   <223> Mutated EL188 with A182T, N187G and F230L and without N-terminal methionine
<400> 215
   Asn Phe Arg Thr Lys Asn Gly Tyr Arg Asp Leu Gln Ala Leu Val Lys
   1               5                   10                  15
   Glu Leu Gly Leu Tyr Thr Gly Gln Ile Asp Gly Val Trp Gly Lys Gly
               20                  25                  30
   Thr Ser Ser Ser Thr Glu Thr Leu Leu Arg Gly Tyr Ala Glu Val Val
           35                  40                  45
   Gly Lys Asn Thr Gly Gly Ile Gly Leu Pro Thr Thr Ser Asp Ala Ser
       50                  55                  60
   Gly Tyr Asn Val Ile Thr Ala Leu Gln Arg Asn Leu Ala Phe Leu Gly
   65                  70                  75                  80
   Leu Tyr Ser Leu Thr Val Asp Gly Ile Trp Gly Asn Gly Thr Leu Ser
                   85                  90                  95
   Gly Leu Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Thr Ala Asn Asp Leu Gly Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
   210 215 220
   Tyr Leu Thr Ile Leu His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Leu Glu
   290
<210> 216
   <211> 303
   <212> PRT
   <213> Artificial Sequence
<220>
   <223> Mutated EL188 with S120G plus KRKKRKKRK peptide
<400> 216
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
           115                 120                 125
   Gly Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu
       290                 295                 300
<210> 217
   <211> 303
   <212> PRT
   <213> Artificial Sequence
<220>
   <223> Mutated EL188 with A126E and T128K plus KRKKRKKRK peptide
<400> 217
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Glu Phe Lys Ala Lys Val Lys Asp Trp Cys
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Tyr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu
   290 295 300
<210> 218
   <211> 303
   <212> PRT
   <213> Artificial Sequence
<220>
   <223> Mutated EL188 with W134I plus KRKKRKKRK peptide
<400> 218
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Ile Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
                   180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu
       290                 295                 300
<210> 219
   <211> 303
   <212> PRT
   <213> Artificial Sequence
<220>
   <223> Mutated EL188 with H138L plus KRKKRKKRK peptide
<400> 219
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Cys
   130 135 140
   Gly Val Leu Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Tyr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu
   290 295 300
<210> 220
   <211> 303
   <212> PRT
   <213> Artificial Sequence
<220>
   <223> Mutated EL188 with E171G plus KRKKRKKRK peptide
<400> 220
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Gly Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu
   290 295 300
<210> 221
   <211> 303
   <212> PRT
   <213> Artificial Sequence
<220>
   <223> Mutated EL188 with Y173T plus KRKKRKKRK peptide
<400> 221
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Cys
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Thr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu
   290 295 300
<210> 222
   <211> 303
   <212> PRT
   <213> Artificial Sequence
<220>
   <223> Mutated EL188 with A182T plus KRKKRKKRK peptide
<400> 222
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Thr Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu
       290                 295                 300
<210> 223
   <211> 303
   <212> PRT
   <213> Artificial Sequence
<220>
   <223> Mutated EL188 with N187G plus KRKKRKKRK peptide
<400> 223
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Gly Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu
       290                 295                 300
<210> 224
   <211> 303
   <212> PRT
   <213> Artificial Sequence
<220>
   <223> Mutated EL188 with D197S and Q198A plus KRKKRKKRK peptide
<400> 224
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Ser Ala Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu
       290                 295                 300
<210> 225
   <211> 303
   <212> PRT
   <213> Artificial Sequence
<220>
   <223> Mutated EL188 with L204Y plus KRKKRKKRK peptide
<400> 225
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Tyr Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu
   290 295 300
<210> 226
   <211> 303
   <212> PRT
   <213> Artificial Sequence
<220>
   <223> Mutated EL188 with F230L plus KRKKRKKRK peptide
<400> 226
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Leu His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu
   290 295 300
<210> 227
   <211> 303
   <212> PRT
   <213> Artificial Sequence
<220>
   <223> Mutated EL188 with H231Y plus KRKKRKKRK peptide
<400> 227
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe Tyr
   225                 230                 235                 240
   Pro Ala Ser Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
           275                 280                 285
   Lys Gly Leu Leu Pro Glu Asn Arg His Val Ile Ser Tyr Leu Glu
       290                 295                 300
<210> 228
   <211> 303
   <212> PRT
   <213> Artificial Sequence
<220>
   <223> Mutated EL188 with S234A plus KRKKRKKRK peptide
<400> 228
   Met Lys Arg Lys Lys Arg Lys Lys Arg Lys Asn Phe Arg Thr Lys Asn
   1               5                   10                  15
   Gly Tyr Arg Asp Leu Gln Ala Leu Val Lys Glu Leu Gly Leu Tyr Thr
               20                  25                  30
   Gly Gln Ile Asp Gly Val Trp Gly Lys Gly Thr Ser Ser Ser Thr Glu
           35                  40                  45
   Thr Leu Leu Arg Gly Tyr Ala Glu Val Val Gly Lys Asn Thr Gly Gly
       50                  55                  60
   Ile Gly Leu Pro Thr Thr Ser Asp Ala Ser Gly Tyr Asn Val Ile Thr
   65                  70                  75                  80
   Ala Leu Gln Arg Asn Leu Ala Phe Leu Gly Leu Tyr Ser Leu Thr Val
                   85                  90                  95
   Asp Gly Ile Trp Gly Asn Gly Thr Leu Ser Gly Leu Asp Lys Ala Phe
               100                 105                 110
   Glu Val Tyr Lys Glu Arg Tyr Arg Thr Pro Thr Tyr Asp Ile Ala Trp
           115                 120                 125
   Ser Gly Lys Val Ser Pro Ala Phe Thr Ala Lys Val Lys Asp Trp Cys
       130                 135                 140
   Gly Val His Val Pro Asn His Arg Ala Pro His Trp Leu Met Ala Cys
   145                 150                 155                 160
   Met Ala Phe Glu Thr Gly Gln Thr Phe Ser Pro Ser Ile Lys Asn Ala
                   165                 170                 175
   Ala Gly Ser Glu Ala Tyr Gly Leu Ile Gln Phe Met Ser Pro Ala Ala
               180                 185                 190
   Asn Asp Leu Asn Val Pro Leu Ser Val Ile Arg Ser Met Asp Gln Leu
           195                 200                 205
   Thr Gln Leu Asp Leu Val Phe Lys Tyr Phe Glu Met Trp Met Lys Arg
       210                 215                 220
   Gly Lys Arg Tyr Thr Gln Leu Glu Asp Phe Tyr Leu Thr Ile Phe His
   225                 230                 235                 240
   Pro Ala Ala Val Gly Lys Lys Ala Asp Glu Val Leu Phe Leu Gln Gly
                   245                 250                 255
   Ser Lys Ala Tyr Leu Gln Asn Lys Gly Phe Asp Val Asp Lys Asp Gly
               260                 265                 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu
   290 295 300
<210> 229
   <211> 303
   <212> PRT
   <213> Artificial Sequence
<220>
   <223> Mutated EL188 with C135S plus KRKKRKKRK peptide
<400> 229
   Met Lys Arg Lys Lys Arg Lys Lys Arg Lys Asn Phe Arg Thr Lys Asn
   1 5 10 15
   Gly Tyr Arg Asp Leu Gln Ala Leu Val Lys Glu Leu Gly Leu Tyr Thr
   20 25 30
   Gly Gln Ile Asp Gly Val Trp Gly Lys Gly Thr Ser Ser Ser Thr Glu
   35 40 45
   Thr Leu Leu Arg Gly Tyr Ala Glu Val Val Gly Lys Asn Thr Gly Gly
   50 55 60
   Ile Gly Leu Pro Thr Thr Ser Asp Ala Ser Gly Tyr Asn Val Ile Thr
   65 70 75 80
   Ala Leu Gln Arg Asn Leu Ala Phe Leu Gly Leu Tyr Ser Leu Thr Val
   85 90 95
   Asp Gly Ile Trp Gly Asn Gly Thr Leu Ser Gly Leu Asp Lys Ala Phe
   100 105 110
   Glu Val Tyr Lys Glu Arg Tyr Arg Thr Pro Thr Tyr Asp Ile Ala Trp
   115 120 125
   Ser Gly Lys Val Ser Pro Ala Phe Thr Ala Lys Val Lys Asp Trp Ser
   130 135 140
   Gly Val His Val Pro Asn His Arg Ala Pro His Trp Leu Met Ala Cys
   145 150 155 160
   Met Ala Phe Glu Thr Gly Gln Thr Phe Ser Pro Ser Ile Lys Asn Ala
   165 170 175
   Ala Gly Ser Glu Ala Tyr Gly Leu Ile Gln Phe Met Ser Pro Ala Ala
   180 185 190
   Asn Asp Leu Asn Val Pro Leu Ser Val Ile Arg Ser Met Asp Gln Leu
   195 200 205
   Thr Gln Leu Asp Leu Val Phe Lys Tyr Phe Glu Met Trp Met Lys Arg
   210 215 220
   Gly Lys Arg Tyr Thr Gln Leu Glu Asp Phe Tyr Leu Thr Ile Phe His
   225 230 235 240
   Pro Ala Ser Val Gly Lys Lys Ala Asp Glu Val Leu Phe Leu Gln Gly
   245 250 255
   Ser Lys Ala Tyr Leu Gln Asn Lys Gly Phe Asp Val Asp Lys Asp Gly
   260 265 270
   Lys Ile Thr Leu Gly Glu Ile Ser Ser Thr Leu Tyr Thr Thr Tyr Tyr
   275 280 285
   Lys Gly Leu Leu Pro Glu Asn Arg His Val Ile Ser Tyr Leu Glu
   290 295 300
<210> 230
   <211> 326
   <212> PRT
   <213> Artificial Sequence
<220>
   <223> Mutated EL188 with A182T plus SMAP peptide
<400> 230
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Thr Ala Asn Asp Leu Asn Val Pro Leu Ser Val
       210                 215                 220
   Ile Arg Ser Met Asp Gln Leu Thr Gln Leu Asp Leu Val Phe Lys Tyr
   225                 230                 235                 240
   Phe Glu Met Trp Met Lys Arg Gly Lys Arg Tyr Thr Gln Leu Glu Asp
                   245                 250                 255
   Phe Tyr Leu Thr Ile Phe His Pro Ala Ser Val Gly Lys Lys Ala Asp
               260                 265                 270
   Glu Val Leu Phe Leu Gln Gly Ser Lys Ala Tyr Leu Gln Asn Lys Gly
           275                 280                 285
   Phe Asp Val Asp Lys Asp Gly Lys Ile Thr Leu Gly Glu Ile Ser Ser
       290                 295                 300
   Thr Leu Tyr Thr Thr Tyr Tyr Lys Gly Leu Leu Pro Glu Asn Arg His
   305                 310                 315                 320
   Val Ile Ser Tyr Leu Glu
                   325
<210> 231
   <211> 326
   <212> PRT
   <213> Artificial Sequence
<220>
   <223> Mutated EL188 with N187G plus SMAP peptide
<400> 231
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Ala Ala Asn Asp Leu Gly Val Pro Leu Ser Val
       210                 215                 220
   Ile Arg Ser Met Asp Gln Leu Thr Gln Leu Asp Leu Val Phe Lys Tyr
   225                 230                 235                 240
   Phe Glu Met Trp Met Lys Arg Gly Lys Arg Tyr Thr Gln Leu Glu Asp
                   245                 250                 255
   Phe Tyr Leu Thr Ile Phe His Pro Ala Ser Val Gly Lys Lys Ala Asp
               260                 265                 270
   Glu Val Leu Phe Leu Gln Gly Ser Lys Ala Tyr Leu Gln Asn Lys Gly
           275                 280                 285
   Phe Asp Val Asp Lys Asp Gly Lys Ile Thr Leu Gly Glu Ile Ser Ser
       290                 295                 300
   Thr Leu Tyr Thr Thr Tyr Tyr Lys Gly Leu Leu Pro Glu Asn Arg His
   305                 310                 315                 320
   Val Ile Ser Tyr Leu Glu
                   325
<210> 232
   <211> 326
   <212> PRT
   <213> Artificial Sequence
<220>
   <223> Mutated EL188 with N187G and F230L plus SMAP peptide
<400> 232
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Ala Ala Asn Asp Leu Gly Val Pro Leu Ser Val
       210                 215                 220
   Ile Arg Ser Met Asp Gln Leu Thr Gln Leu Asp Leu Val Phe Lys Tyr
   225                 230                 235                 240
   Phe Glu Met Trp Met Lys Arg Gly Lys Arg Tyr Thr Gln Leu Glu Asp
                   245                 250                 255
   Phe Tyr Leu Thr Ile Leu His Pro Ala Ser Val Gly Lys Lys Ala Asp
               260                 265                 270
   Glu Val Leu Phe Leu Gln Gly Ser Lys Ala Tyr Leu Gln Asn Lys Gly
           275                 280                 285
   Phe Asp Val Asp Lys Asp Gly Lys Ile Thr Leu Gly Glu Ile Ser Ser
       290                 295                 300
   Thr Leu Tyr Thr Thr Tyr Tyr Lys Gly Leu Leu Pro Glu Asn Arg His
   305                 310                 315                 320
   Val Ile Ser Tyr Leu Glu
                   325
<210> 233
   <211> 326
   <212> PRT
   <213> Artificial Sequence
<220>
   <223> Mutated EL188 with A182T, N187G and F230L plus SMAP peptide
<400> 233
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Asn Phe Arg Thr Lys Asn Gly Tyr Arg Asp Leu Gln Ala Leu Val
           35                  40                  45
   Lys Glu Leu Gly Leu Tyr Thr Gly Gln Ile Asp Gly Val Trp Gly Lys
       50                  55                  60
   Gly Thr Ser Ser Ser Thr Glu Thr Leu Leu Arg Gly Tyr Ala Glu Val
   65                  70                  75                  80
   Val Gly Lys Asn Thr Gly Gly Ile Gly Leu Pro Thr Thr Ser Asp Ala
                   85                  90                  95
   Ser Gly Tyr Asn Val Ile Thr Ala Leu Gln Arg Asn Leu Ala Phe Leu
               100                 105                 110
   Gly Leu Tyr Ser Leu Thr Val Asp Gly Ile Trp Gly Asn Gly Thr Leu
           115                 120                 125
   Ser Gly Leu Asp Lys Ala Phe Glu Val Tyr Lys Glu Arg Tyr Arg Thr
       130                 135                 140
   Pro Thr Tyr Asp Ile Ala Trp Ser Gly Lys Val Ser Pro Ala Phe Thr
   145                 150                 155                 160
   Ala Lys Val Lys Asp Trp Cys Gly Val His Val Pro Asn His Arg Ala
                   165                 170                 175
   Pro His Trp Leu Met Ala Cys Met Ala Phe Glu Thr Gly Gln Thr Phe
               180                 185                 190
   Ser Pro Ser Ile Lys Asn Ala Ala Gly Ser Glu Ala Tyr Gly Leu Ile
           195                 200                 205
   Gln Phe Met Ser Pro Thr Ala Asn Asp Leu Gly Val Pro Leu Ser Val
       210                 215                 220
   Ile Arg Ser Met Asp Gln Leu Thr Gln Leu Asp Leu Val Phe Lys Tyr
   225 230 235 240
   Phe Glu Met Trp Met Lys Arg Gly Lys Arg Tyr Thr Gln Leu Glu Asp
   245 250 255
   Phe Tyr Leu Thr Ile Leu His Pro Ala Ser Val Gly Lys Lys Ala Asp
   260 265 270
   Glu Val Leu Phe Leu Gln Gly Ser Lys Ala Tyr Leu Gln Asn Lys Gly
   275 280 285
   Phe Asp Val Asp Lys Asp Gly Lys Ile Thr Leu Gly Glu Ile Ser Ser
   290 295 300
   Thr Leu Tyr Thr Thr Tyr Tyr Lys Gly Leu Leu Pro Glu Asn Arg His
   305 310 315 320
   Val Ile Ser Tyr Leu Glu
   325
<210> 234
   <211> 302
   <212> PRT
   <213> Artificial Sequence
<220>
   <223> Mutated EL188 with S120G plus KRKKRKKRK peptide, without N-terminal methionine
<400> 234
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Gly
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu
   290 295 300
<210> 235
   <211> 302
   <212> PRT
   <213> Artificial Sequence
<220>
   <223> Mutated EL188 with A126E and T128K plus KRKKRKKRK peptide, without N-terminal methionine
<400> 235
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Glu Phe Lys Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu
   290 295 300
<210> 236
   <211> 302
   <212> PRT
   <213> Artificial Sequence
<220>
   <223> Mutated EL188 with W134I plus KRKKRKKRK peptide, without N-terminal methionine
<400> 236
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Ile Cys Gly
       130                 135                 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu
   290 295 300
<210> 237
   <211> 302
   <212> PRT
   <213> Artificial Sequence
<220>
   <223> Mutated EL188 with H138L plus KRKKRKKRK peptide, without N-terminal methionine
<400> 237
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val Leu Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu
       290                 295                 300
<210> 238
   <211> 302
   <212> PRT
   <213> Artificial Sequence
<220>
   <223> Mutated EL188 with E171G plus KRKKRKKRK peptide, without N-terminal methionine
<400> 238
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Gly Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu
       290                 295                 300
<210> 239
   <211> 302
   <212> PRT
   <213> Artificial Sequence
<220>
   <223> Mutated EL188 with Y173T plus KRKKRKKRK peptide, without N-terminal methionine
<400> 239
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Thr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu
       290                 295                 300
<210> 240
   <211> 302
   <212> PRT
   <213> Artificial Sequence
<220>
   <223> Mutated EL188 with A182T plus KRKKRKKRK peptide, without N-terminal methionine
<400> 240
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Thr Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu
   290 295 300
<210> 241
   <211> 302
   <212> PRT
   <213> Artificial Sequence
<220>
   <223> Mutated EL188 with N187G plus KRKKRKKRK peptide, without N-terminal methionine
<400> 241
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Gly Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu
   290 295 300
<210> 242
   <211> 302
   <212> PRT
   <213> Artificial Sequence
<220>
   <223> Mutated EL188 with D197S and Q198A plus KRKKRKKRK peptide, without N-terminal methionine
<400> 242
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Ser Ala Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu
   290 295 300
<210> 243
   <211> 302
   <212> PRT
   <213> Artificial Sequence
<220>
   <223> Mutated EL188 with L204Y plus KRKKRKKRK peptide, without N-terminal methionine
<400> 243
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Tyr Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu
   290 295 300
<210> 244
   <211> 302
   <212> PRT
   <213> Artificial Sequence
<220>
   <223> Mutated EL188 with F230L plus KRKKRKKRK peptide, without N-terminal methionine
<400> 244
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
   50 55 60
   Gly Leu Pro Thr Thr Ser Asp Ala Ser Gly Tyr Asn Val Ile Thr Ala
   65 70 75 80
   Leu Gln Arg Asn Leu Ala Phe Leu Gly Leu Tyr Ser Leu Thr Val Asp
   85 90 95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Cys Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Leu His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu
   290 295 300
<210> 245
   <211> 302
   <212> PRT
   <213> Artificial Sequence
<220>
   <223> Mutated EL188 with H231Y plus KRKKRKKRK peptide, without N-terminal methionine
<400> 245
   Lys Arg Lys Lys Arg Lys Lys Arg Lys Asn Phe Arg Thr Lys Asn Gly
   1 5 10 15
   Tyr Arg Asp Leu Gln Ala Leu Val Lys Glu Leu Gly Leu Tyr Thr Gly
   20 25 30
   Gln Ile Asp Gly Val Trp Gly Lys Gly Thr Ser Ser Ser Thr Glu Thr
   35 40 45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe Tyr Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu
   290 295 300
<210> 246
   <211> 302
   <212> PRT
   <213> Artificial Sequence
<220>
   <223> Mutated EL188 with S234A plus KRKKRKKRK peptide, without N-terminal methionine
<400> 246
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
               195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ala Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr Leu Glu
       290                 295                 300
<210> 247
   <211> 302
   <212> PRT
   <213> Artificial Sequence
<220>
   <223> Mutated EL188 with C135S plus KRKKRKKRK peptide, without N-terminal methionine
<400> 247
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
   100 105 110
   Val Tyr Lys Glu Arg Tyr Arg Thr Pro Thr Tyr Asp Ile Ala Trp Ser
   115 120 125
   Gly Lys Val Ser Pro Ala Phe Thr Ala Lys Val Lys Asp Trp Ser Gly
   130 135 140
   Val His Val Pro Asn His Arg Ala Pro His Trp Leu Met Ala Cys Met
   145 150 155 160
   Ala Phe Glu Thr Gly Gln Thr Phe Ser Pro Ser Ile Lys Asn Ala Ala
   165 170 175
   Gly Ser Glu Ala Tyr Gly Leu Ile Gln Phe Met Ser Pro Ala Ala Asn
   180 185 190
   Asp Leu Asn Val Pro Leu Ser Val Ile Arg Ser Met Asp Gln Leu Thr
   195 200 205
   Gln Leu Asp Leu Val Phe Lys Tyr Phe Glu Met Trp Met Lys Arg Gly
   210 215 220
   Lys Arg Tyr Thr Gln Leu Glu Asp Phe Tyr Leu Thr Ile Phe His Pro
   225 230 235 240
   Ala Ser Val Gly Lys Lys Ala Asp Glu Val Leu Phe Leu Gln Gly Ser
   245 250 255
   Lys Ala Tyr Leu Gln Asn Lys Gly Phe Asp Val Asp Lys Asp Gly Lys
   260 265 270
   Ile Thr Leu Gly Glu Ile Ser Ser Thr Leu Tyr Thr Thr Tyr Tyr Lys
   275 280 285
   Gly Leu Leu Pro Glu Asn Arg His Val Ile Ser Tyr Leu Glu
   290 295 300
<210> 248
   <211> 325
   <212> PRT
   <213> Artificial Sequence
<220>
   <223> Mutated EL188 with A182T plus SMAP peptide, without N-terminal methionine
<400> 248
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Met
               20                  25                  30
   Asn Phe Arg Thr Lys Asn Gly Tyr Arg Asp Leu Gln Ala Leu Val Lys
           35                  40                  45
   Glu Leu Gly Leu Tyr Thr Gly Gln Ile Asp Gly Val Trp Gly Lys Gly
       50                  55                  60
   Thr Ser Ser Ser Thr Glu Thr Leu Leu Arg Gly Tyr Ala Glu Val Val
   65                  70                  75                  80
   Gly Lys Asn Thr Gly Gly Ile Gly Leu Pro Thr Thr Ser Asp Ala Ser
                   85                  90                  95
   Gly Tyr Asn Val Ile Thr Ala Leu Gln Arg Asn Leu Ala Phe Leu Gly
               100                 105                 110
   Leu Tyr Ser Leu Thr Val Asp Gly Ile Trp Gly Asn Gly Thr Leu Ser
           115                 120                 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Thr Ala Asn Asp Leu Asn Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Phe His Pro Ala Ser Val Gly Lys Lys Ala Asp Glu
   260 265 270
   Val Leu Phe Leu Gln Gly Ser Lys Ala Tyr Leu Gln Asn Lys Gly Phe
   275 280 285
   Asp Val Asp Lys Asp Gly Lys Ile Thr Leu Gly Glu Ile Ser Ser Thr
   290 295 300
   Leu Tyr Thr Thr Tyr Tyr Lys Gly Leu Leu Pro Glu Asn Arg His Val
   305 310 315 320
   Ile Ser Tyr Leu Glu
   325
<210> 249
   <211> 325
   <212> PRT
   <213> Artificial Sequence
<220>
   <223> Mutated EL188 with N187G plus SMAP peptide, without N-terminal methionine
<400> 249
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Met
   20 25 30
   Asn Phe Arg Thr Lys Asn Gly Tyr Arg Asp Leu Gln Ala Leu Val Lys
   35 40 45
   Glu Leu Gly Leu Tyr Thr Gly Gln Ile Asp Gly Val Trp Gly Lys Gly
   50 55 60
   Thr Ser Ser Ser Thr Glu Thr Leu Leu Arg Gly Tyr Ala Glu Val Val
   65 70 75 80
   Gly Lys Asn Thr Gly Gly Ile Gly Leu Pro Thr Thr Ser Asp Ala Ser
   85 90 95
   Gly Tyr Asn Val Ile Thr Ala Leu Gln Arg Asn Leu Ala Phe Leu Gly
   100 105 110
   Leu Tyr Ser Leu Thr Val Asp Gly Ile Trp Gly Asn Gly Thr Leu Ser
   115 120 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Ala Ala Asn Asp Leu Gly Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Phe His Pro Ala Ser Val Gly Lys Lys Ala Asp Glu
               260                 265                 270
   Val Leu Phe Leu Gln Gly Ser Lys Ala Tyr Leu Gln Asn Lys Gly Phe
           275                 280                 285
   Asp Val Asp Lys Asp Gly Lys Ile Thr Leu Gly Glu Ile Ser Ser Thr
       290                 295                 300
   Leu Tyr Thr Thr Tyr Tyr Lys Gly Leu Leu Pro Glu Asn Arg His Val
   305                 310                 315                 320
   Ile Ser Tyr Leu Glu
                   325
<210> 250
   <211> 325
   <212> PRT
   <213> Artificial Sequence
<220>
   <223> Mutated EL188 with N187G and F230L plus SMAP peptide, without N-terminal methionine
<400> 250
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Met
               20                  25                  30
   Asn Phe Arg Thr Lys Asn Gly Tyr Arg Asp Leu Gln Ala Leu Val Lys
           35                  40                  45
   Glu Leu Gly Leu Tyr Thr Gly Gln Ile Asp Gly Val Trp Gly Lys Gly
       50                  55                  60
   Thr Ser Ser Ser Thr Glu Thr Leu Leu Arg Gly Tyr Ala Glu Val Val
   65                  70                  75                  80
   Gly Lys Asn Thr Gly Gly Ile Gly Leu Pro Thr Thr Ser Asp Ala Ser
                   85                  90                  95
   Gly Tyr Asn Val Ile Thr Ala Leu Gln Arg Asn Leu Ala Phe Leu Gly
               100                 105                 110
   Leu Tyr Ser Leu Thr Val Asp Gly Ile Trp Gly Asn Gly Thr Leu Ser
           115                 120                 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Ala Ala Asn Asp Leu Gly Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Leu His Pro Ala Ser Val Gly Lys Lys Ala Asp Glu
   260 265 270
   Val Leu Phe Leu Gln Gly Ser Lys Ala Tyr Leu Gln Asn Lys Gly Phe
   275 280 285
   Asp Val Asp Lys Asp Gly Lys Ile Thr Leu Gly Glu Ile Ser Ser Thr
   290 295 300
   Leu Tyr Thr Thr Tyr Tyr Lys Gly Leu Leu Pro Glu Asn Arg His Val
   305 310 315 320
   Ile Ser Tyr Leu Glu
   325
<210> 251
   <211> 325
   <212> PRT
   <213> Artificial Sequence
<220>
   <223> Mutated EL188 with A182T, N187G and F230 L plus SMAP peptide, without N-terminal methionine
<400> 251
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Met
   20 25 30
   Asn Phe Arg Thr Lys Asn Gly Tyr Arg Asp Leu Gln Ala Leu Val Lys
   35 40 45
   Glu Leu Gly Leu Tyr Thr Gly Gln Ile Asp Gly Val Trp Gly Lys Gly
   50 55 60
   Thr Ser Ser Ser Thr Glu Thr Leu Leu Arg Gly Tyr Ala Glu Val Val
   65 70 75 80
   Gly Lys Asn Thr Gly Gly Ile Gly Leu Pro Thr Thr Ser Asp Ala Ser
   85 90 95
   Gly Tyr Asn Val Ile Thr Ala Leu Gln Arg Asn Leu Ala Phe Leu Gly
   100 105 110
   Leu Tyr Ser Leu Thr Val Asp Gly Ile Trp Gly Asn Gly Thr Leu Ser
   115 120 125
   Gly Leu Asp Lys Ala Phe Glu Val Tyr Lys Glu Arg Tyr Arg Thr Pro
       130                 135                 140
   Thr Tyr Asp Ile Ala Trp Ser Gly Lys Val Ser Pro Ala Phe Thr Ala
   145                 150                 155                 160
   Lys Val Lys Asp Trp Cys Gly Val His Val Pro Asn His Arg Ala Pro
                   165                 170                 175
   His Trp Leu Met Ala Cys Met Ala Phe Glu Thr Gly Gln Thr Phe Ser
               180                 185                 190
   Pro Ser Ile Lys Asn Ala Ala Gly Ser Glu Ala Tyr Gly Leu Ile Gln
           195                 200                 205
   Phe Met Ser Pro Thr Ala Asn Asp Leu Gly Val Pro Leu Ser Val Ile
       210                 215                 220
   Arg Ser Met Asp Gln Leu Thr Gln Leu Asp Leu Val Phe Lys Tyr Phe
   225                 230                 235                 240
   Glu Met Trp Met Lys Arg Gly Lys Arg Tyr Thr Gln Leu Glu Asp Phe
                   245                 250                 255
   Tyr Leu Thr Ile Leu His Pro Ala Ser Val Gly Lys Lys Ala Asp Glu
               260                 265                 270
   Val Leu Phe Leu Gln Gly Ser Lys Ala Tyr Leu Gln Asn Lys Gly Phe
           275                 280                 285
   Asp Val Asp Lys Asp Gly Lys Ile Thr Leu Gly Glu Ile Ser Ser Thr
       290                 295                 300
   Leu Tyr Thr Thr Tyr Tyr Lys Gly Leu Leu Pro Glu Asn Arg His Val
   305                 310                 315                 320
   Ile Ser Tyr Leu Glu
                   325

## Description

The present invention relates to polypeptides comprising an amino acid sequence exhibiting at least 90% sequence identity with the sequence of SEQ ID NO: 4, wherein the polypeptide does neither comprise the amino acid sequence of SEQ ID NO: 2 nor of SEQ ID NO: 183, wherein the polypeptide does not comprise an E155A mutation at position 155 of SEQ ID NO: 4, wherein the polypeptide exhibits in the amino acid sequence exhibiting at least 90% sequence identity with the sequence of SEQ ID NO: 4 at least one of the following: the amino acid at position 120 is G, the amino acid at position 126 is E, the amino acid at position 128 is K, the amino acid at position 134 is I, the amino acid at position 135 is not C, the amino acid at position 138 is L, the amino acid at position 171 is G, the amino acid at position 173 is T, the amino acid at position 182 is T, the amino acid at position 187 is G, the amino acid at position 197 is S, the amino acid at position 198 is A, the amino acid at position 204 is Y, the amino acid at position 230 is L, the amino acid at position 231 is Y, the amino acid at position 234 is A, and wherein the said amino acid sequence exhibiting at least 90% sequence identity with the sequence of SEQ ID NO: 4 exhibits improved temperature stability and/or muralytic activity as compared to the amino acid sequence of SEQ ID NO: 2. Said polypeptides preferably degrade the peptidoglycan of Gram-negative bacteria, in particular of *Pseudomonas* and/or *Campylobacter* bacteria. In addition, the present invention relates to nucleic acids encoding such polypeptides, vectors comprising such nucleic acids, and corresponding host cells. Finally, the present invention relates to compositions comprising such polypeptides, nucleic acids, vectors, and/or host cells according to the present invention.

The giant, lytic Myoviridae bacteriophage EL (211 215 bp) infects *Pseudomonas aeruginosa,* an important opportunistic nosocomial pathogen resistant to many commonly used antibiotics, and is therefore the cause of considerable concern in hospital environments. In 2007, Briers et al. (Molecular Microbiology (2007) 65(5), 1334-1344) sequenced the genome of said bacteriophage and identified the endolysin EL188, a highly lytic peptidoglycan hydrolase. In WO 2010/149792, a fusion protein comprising the sequence of said endolysin as enzymatic element has been proposed for use in degrading the cell wall of Gram-negative bacteria. Similarly, WO 2012/085259 discloses fusion proteins composed of an enzyme having the activity of degrading the cell wall of Gram-negative bacteria and/or Gram-positive bacteria and at least two peptide stretches fused to the enzyme. The enzyme may be inter alia endolysin EL188. Sequences 141, 150 and 151 are examples of such sequence, however with an inadvertent deletion of L99. In the same context, WO 2011/134998 teaches methods of eliminating, reducing or preventing bacterial biofilms by means of fusion proteins comprising an endolysin, an autolysin or a bacteriocin to which a peptide with membrane or LPS disrupting activity is fused. Sequences 40 and 42 are examples for such fusion protein on basis of EL188 endolysin.

While said endolysin and fusion proteins are effective in general, it turned out, that for some technical applications the endolysin polypeptide exhibits suboptimal characteristics, in particular in terms of stability and processing. Thus, there was a need in the art for a further endolysin enzyme, which exhibits preferably improved characteristics in this respect. The problem of the present invention was thus to provide such polypeptide.

The problem is solved by the subject-matter as set forth in the appended claims.

In the following a brief description of the appended figures will be given. The figures are intended to illustrate the present invention in more detail. However, they are not intended to limit the subject matter of the invention to any extent.

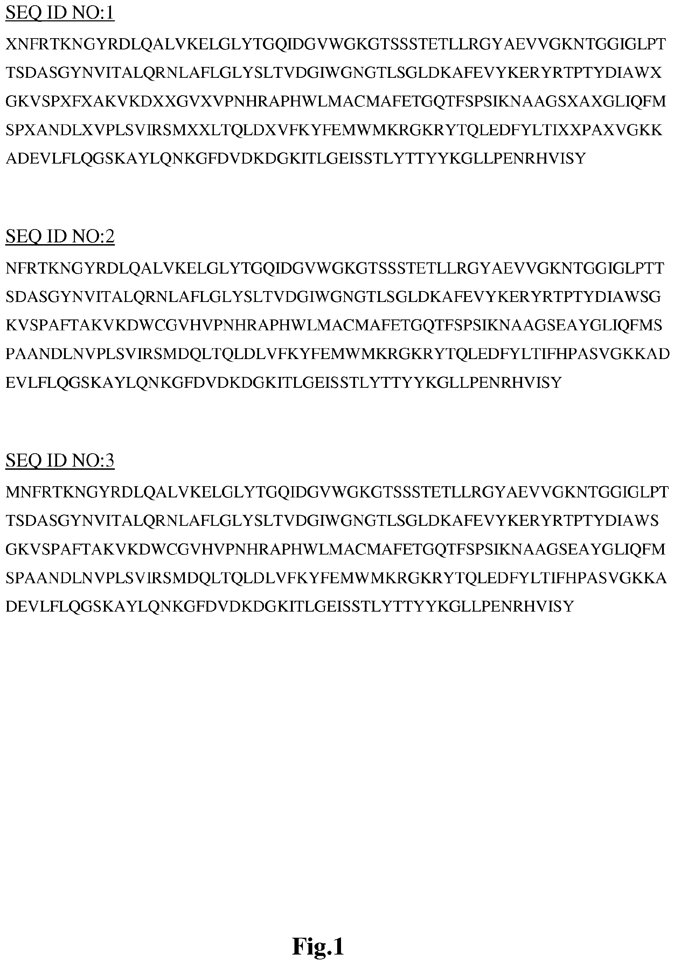

Fig. 1: illustrates:

| | |
|---|---|
| SEQ ID NO: 1, SEQ ID NO: 2 | EL188 endolysin without N-terminal methionine, |
| SEQ ID NO: 3 | EL188 endoylsin. |

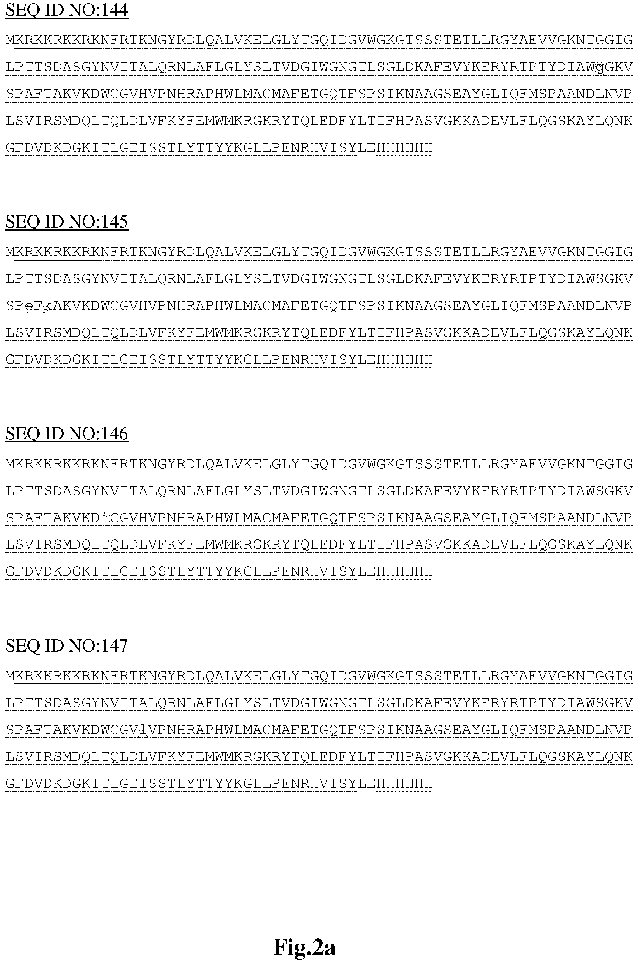

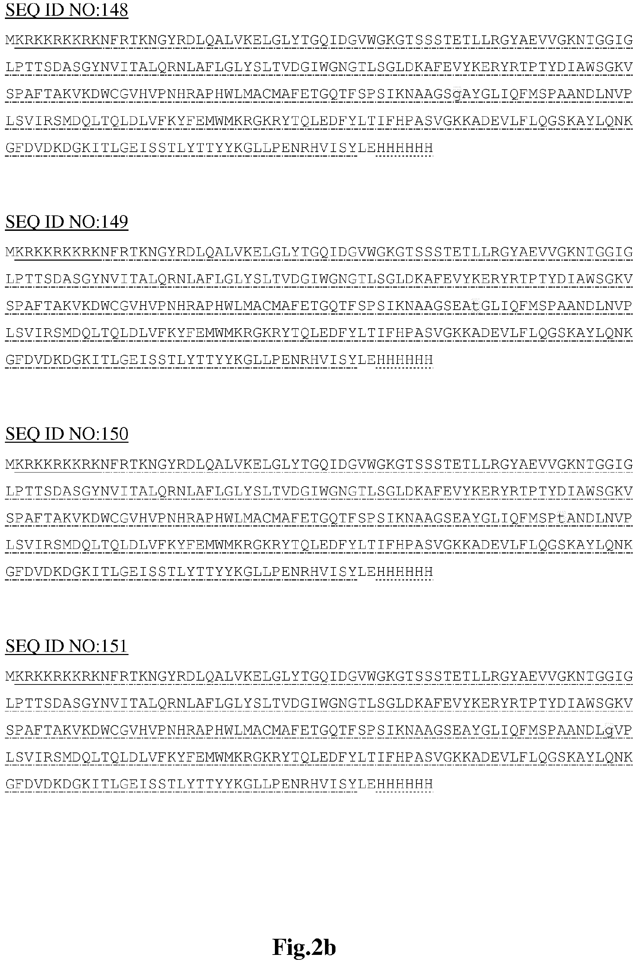

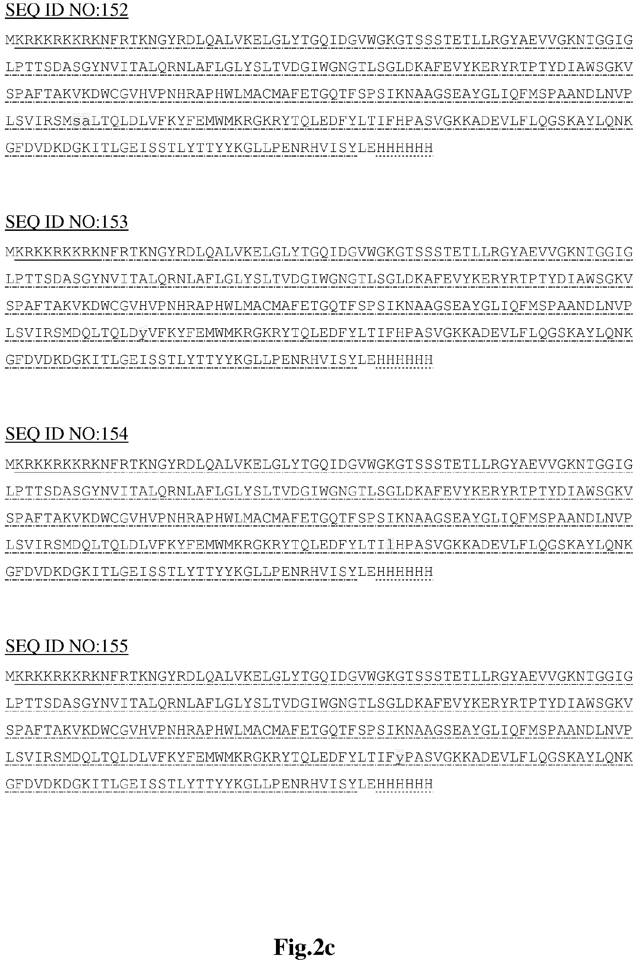

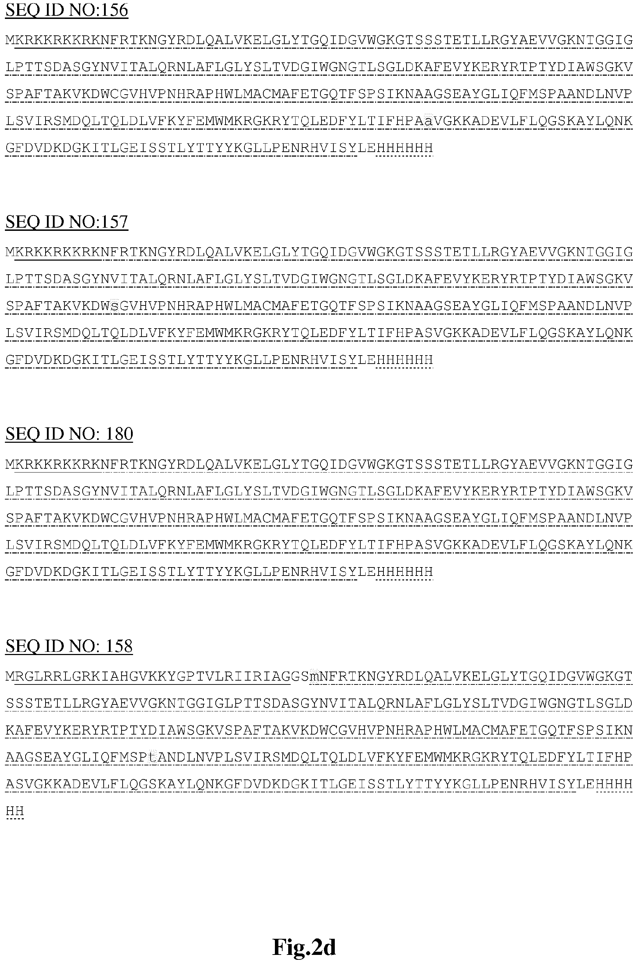

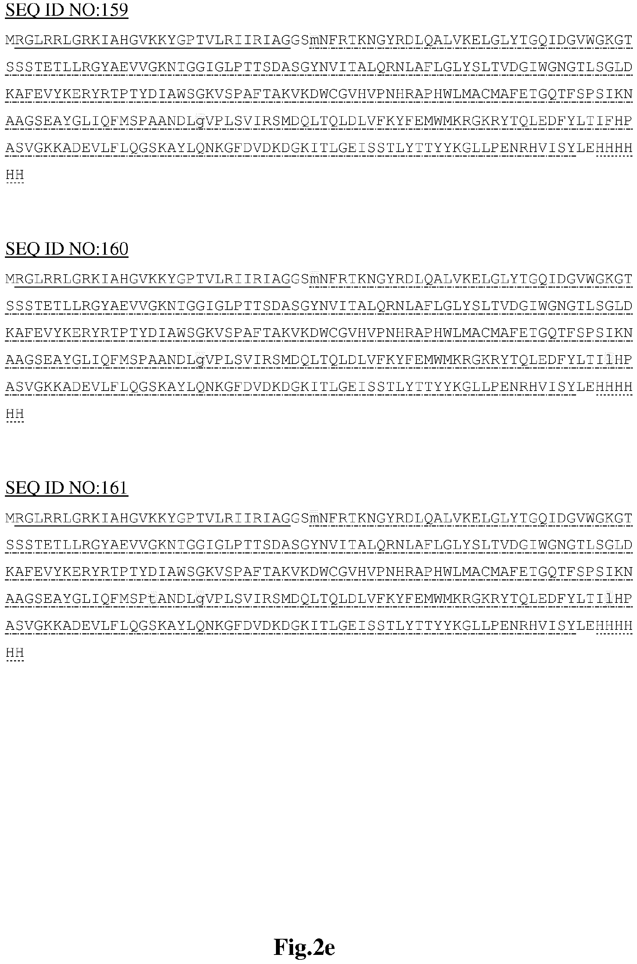

Fig. 2: illustrates:

| | |
|---|---|
| SEQ ID NO: 144 | Fusion protein of KRKKRKKRK (underlined with solid line; SEQ ID NO: 40), modified EL188 without N-terminal methionine and with S120G (underlined with semi-dotted/semi-solid line; SEQ ID NO: 20) and His-tag (underlined with dotted line, SEQ ID NO: 143). |
| SEQ ID NO: 145 | Fusion protein of KRKKRKKRK (underlined with solid line; SEQ ID NO:40), modified EL188 without N-terminal methionine and with A126E and T128K (underlined with semi-dotted/semi-solid line; SEQ ID NO: 21) and His-tag (underlined with dotted line, SEQ ID NO: 143). |
| SEQ ID NO: 146 | Fusion protein of KRKKRKKRK (underlined with solid line; SEQ ID NO:40), modified EL188 without N-terminal methionine and with W134I (underlined with semi-dotted/semi-solid line; SEQ ID NO: 22) and His-tag (underlined with dotted line, SEQ ID NO: 143). |
| SEQ ID NO: 147 | Fusion protein of KRKKRKKRK (underlined with solid line; SEQ ID NO:40), modified EL188 without N-terminal methionine and with H138L (underlined with semi-dotted/semi-solid line; SEQ ID NO: 23) and His-tag (underlined with dotted line, SEQ ID NO: 143). |
| SEQ ID NO: 148 | Fusion protein of KRKKRKKRK (underlined with solid line; SEQ ID NO:40), modified EL188 without N-terminal methionine and with E171G (underlined with semi-dotted/semi-solid line; SEQ ID NO: 24) and His-tag (underlined with dotted line, SEQ ID NO: 143). |
| SEQ ID NO: 149 | Fusion protein of KRKKRKKRK (underlined with solid line; SEQ ID NO:40), modified EL188 without N-terminal methionine and with Y173T (underlined with semi-dotted/semi-solid line; SEQ ID NO: 25) and His-tag (underlined with dotted line, SEQ ID NO: 143). |
| SEQ ID NO: 150 | Fusion protein of KRKKRKKRK (underlined with solid line; SEQ ID NO:40), modified EL188 without N-terminal methionine and with A182T (underlined with semi-dotted/semi-solid line; SEQ ID NO: 26) and His-tag (underlined with dotted line, SEQ ID NO: 143). |
| SEQ ID NO: 151 | Fusion protein of KRKKRKKRK (underlined with solid line; SEQ ID NO:40), modified EL188 without N-terminal methionine and with N187G (underlined with semi-dotted/semi-solid line; SEQ ID NO: 27) and His-tag (underlined with dotted line, SEQ ID NO: 143). |
| SEQ ID NO: 152 | Fusion protein of KRKKRKKRK (underlined with solid line; SEQ ID NO:40), modified EL188 without N-terminal methionine and with D197S and Q198A (underlined with semi-dotted/semi-solid line; SEQ ID NO: 28) and His-tag (underlined with dotted line, SEQ ID NO: 143). |
| SEQ ID NO: 153 | Fusion protein of KRKKRKKRK (underlined with solid line; SEQ ID NO:40), modified EL188 without N-terminal methionine and with L204Y (underlined with semi-dotted/semi-solid line; SEQ ID NO: 29) and His-tag (underlined with dotted line, SEQ ID NO: 143). |
| SEQ ID NO: 154 | Fusion protein of KRKKRKKRK (underlined with solid line; SEQ ID NO:40), modified EL188 without N-terminal methionine and with F230L (underlined with semi-dotted/semi-solid line; SEQ ID NO: 30) and His-tag (underlined with dotted line, SEQ ID NO: 143). |
| SEQ ID NO: 155 | Fusion protein of KRKKRKKRK (underlined with solid line; SEQ ID NO:40), modified EL188 without N-terminal methionine and with H231Y (underlined with semi-dotted/semi-solid line; SEQ ID NO: 31) and His-tag (underlined with dotted line, SEQ ID NO: 143). |
| SEQ ID NO: 156 | Fusion protein of KRKKRKKRK (underlined with solid line; SEQ ID NO:40), modified EL188 without N-terminal methionine and with S234A (underlined with semi-dotted/semi-solid line; SEQ ID NO: 32) and His-tag (underlined with dotted line, SEQ ID NO: 143). |
| SEQ ID NO: 157 | Fusion protein of KRKKRKKRK (underlined with solid line; SEQ ID NO:40), modified EL188 without N-terminal methionine and with C135S (underlined with semi-dotted/semi-solid line; SEQ ID NO: 33) and His-tag (underlined with dotted line, SEQ ID NO: 143). |
| SEQ ID NO: 180 | Fusion protein of KRKKRKKRK (underlined with solid line; SEQ ID NO:40), unmodified EL188 without N-terminal methionine (underlined with semi-dotted/semi-solid line; SEQ ID NO: 2) and His-tag (underlined with dotted line, SEQ ID NO: 143). |
| SEQ ID NO: 158 | Fusion protein of SMAP29 (underlined with solid line; SEQ ID NO:62), modified EL188 with N-terminal methionine and with A182T (underlined with semi-dotted/semi-solid line; SEQ ID NO: 10) and His-tag (underlined with dotted line, SEQ ID NO: 143). |
| SEQ ID NO: 159 | Fusion protein of SMAP29 (underlined with solid line; SEQ ID NO:62), modified EL188 with N-terminal methionine and with N187G (underlined with semi-dotted/semi-solid line; SEQ ID NO: 11) and His-tag (underlined with dotted line, SEQ ID NO: 143). |
| SEQ ID NO: 160 | Fusion protein of SMAP29 (underlined with solid line; SEQ ID NO:62), modified EL188 with N-terminal methionine and with N187G and F230L (underlined with semi-dotted/semi-solid line; SEQ ID NO: 18) and His-tag (underlined with dotted line, SEQ ID NO: 143). |
| SEQ ID NO: 161 | Fusion protein of SMAP29 (underlined with solid line; SEQ ID NO:62), modified EL188 with N-terminal methionine and with A82T, N187G and F230L (underlined with semi-dotted/semisolid line; SEQ ID NO: 19) and His-tag (underlined with dotted line, SEQ ID NO: 143). |

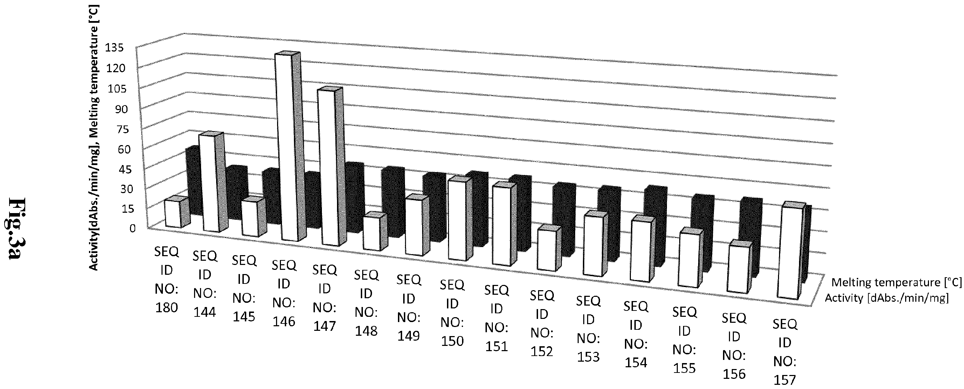

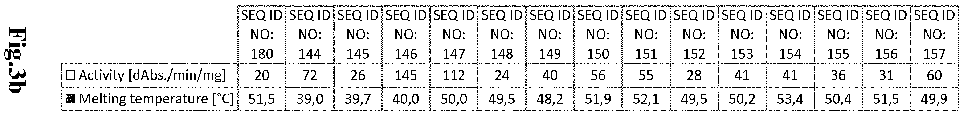

Fig. 3: a) illustrates in white bars the muralytic activity of the various polypeptides according to the present invention and of the non-mutated control (SEQ ID NO: 180) (in delta Absorption units at 600nm per minute per milligram of protein) determined in muralytic activity assays. In black bars the melting temperature of the respective poylpeptides (in °C) is presented, determined by CD-spectroscopy. b) Table indicating the numeric values for a).

In a first aspect the present invention relates to a polypeptide comprising an amino acid sequence exhibiting at least 90% sequence identity with the sequence of SEQ ID NO: 4,
wherein the polypeptide does neither comprise the amino acid sequence of SEQ ID NO: 2 nor of SEQ ID NO: 183, and wherein the polypeptide does not comprise an E155A mutation at position 155 of SEQ ID NO: 4,
wherein the polypeptide exhibits in the amino acid sequence exhibiting at least 90% sequence identity with the sequence of SEQ ID NO: 4 at least one of the following:

| | |
|---|---|
| the amino acid at position 120 | is G |
| the amino acid at position 126 | is E |
| the amino acid at position 128 | is K |
| the amino acid at position 134 | is I |
| the amino acid at position 135 | is not C |
| the amino acid at position 138 | is L |
| the amino acid at position 171 | is G |
| the amino acid at position 173 | is T |
| the amino acid at position 182 | is T |
| the amino acid at position 187 | is G |
| the amino acid at position 197 | is S |
| the amino acid at position 198 | is A |
| the amino acid at position 204 | is Y |
| the amino acid at position 230 | is L |
| the amino acid at position 231 | is Y |
| the amino acid at position 234 | is A, and |

wherein the said amino acid sequence exhibiting at least 90% sequence identity with the sequence of SEQ ID NO: 4 exhibits improved temperature stability and/or muralytic activity as compared to the amino acid sequence of SEQ ID NO: 2.

The term "polypeptide" as used herein refers in particular to a polymer of amino acid residues linked by peptide bonds in a specific sequence. The amino acid residues of a polypeptide may be modified by e.g. covalent attachments of various groups such as carbohydrates and phosphate. Other substances may be more loosely associated with the polypeptide, such as heme or lipid, giving rise to conjugated polypeptides which are also comprised by the term "polypeptide" as used herein. The term as used herein is intended to encompass also proteins. Thus, the term "polypeptide" also encompasses for example complexes of two or more amino acid polymer chains. The term "polypeptide " does encompass embodiments of polypeptides which exhibit optionally modifications typically used in the art, e.g. biotinylation, acetylation, pegylation, chemical changes of the amino-, SH- or carboxyl-groups (e.g. protecting groups) etc.. As will become apparent from the description below, the polypeptide according to the present invention may also be a fusion protein, i.e. linkage of at least two amino acid sequences which do not occur in this combination in nature. The term " polypeptide " as used herein is not limited to a specific length of the amino acid polymer chain, but typically the polypeptide will exhibit a length of more than about 50 amino acids, more than about 100 amino acids or even more than about 150 amino acids. Usually, but not necessarily, a typical polypeptide of the present invention will not exceed about 750 amino acids in length.

As used herein, the term "% sequence identity", has to be understood as follows: Two sequences to be compared are aligned to give a maximum correlation between the sequences. This may include inserting "gaps" in either one or both sequences, to enhance the degree of alignment. A % identity may then be determined over the whole length of each of the sequences being compared (so-called global alignment), that is particularly suitable for sequences of the same or similar length, or over shorter, defined lengths (so-called local alignment), that is more suitable for sequences of unequal length. In the above context, an amino acid sequence having a "sequence identity" of at least, for example, 95% to a query amino acid sequence, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted. Methods for comparing the identity and homology of two or more sequences are well known in the art. The percentage to which two sequences are identical can for example be determined by using a mathematical algorithm. A preferred, but not limiting, example of a mathematical algorithm which can be used is the algorithm of Karlin et a/. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1 997), Nucleic Acids Res, 25:3389-3402), accessible through the home page of the NCBI at world wide web site ncbi.nlm.nih.gov) and FASTA (Pearson (1 990), Methods Enzymol. 83, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al, 1984, Nucleic Acids Res., 387-395), for example the programs BESTFIT and GAP, may be used to determine the % identity between two polypeptide sequences. BESTFIT uses the "local homology" algorithm of (Smith and Waterman (1981), J. Mol. Biol. 147, 195-197.) and finds the best single region of similarity between two sequences. If herein reference is made to an amino acid sequence sharing a particular extent of sequence identity to a reference sequence, then said difference in sequence is preferably due to conservative amino acid substitutions. Preferably, such sequence retains the activity of the reference sequence, e.g. albeit maybe at a slower rate. In addition, if reference is made herein to a sequence sharing "at least" at certain percentage of sequence identity, then 100% sequence identity are preferably not encompassed.

"Conservative amino acid substitutions", as used herein, may occur within a group of amino acids which have sufficiently similar physicochemical properties, so that a substitution between members of the group will preserve the biological activity of the molecule (see e.g. Grantham, R. (1974), Science 185, 862-864). Particularly, conservative amino acid substitutions are preferably substitutions in which the amino acids originate from the same class of amino acids (e.g. basic amino acids, acidic amino acids, polar amino acids, amino acids with aliphatic side chains, amino acids with positively or negatively charged side chains, amino acids with aromatic groups in the side chains, amino acids the side chains of which can enter into hydrogen bridges, e.g. side chains which have a hydroxyl function, etc.). Conservative substitutions are in the present case for example substituting a basic amino acid residue (Lys, Arg, His) for another basic amino acid residue (Lys, Arg, His), substituting an aliphatic amino acid residue (Gly, Ala, Val, Leu, lie) for another aliphatic amino acid residue, substituting an aromatic amino acid residue (Phe, Tyr, Trp) for another aromatic amino acid residue, substituting threonine by serine or leucine by isoleucine. Further conservative amino acid exchanges will be known to the person skilled in the art.

The term "deletion" as used herein refers preferably to the absence of 1, 2, 3, 4, 5 (or even more than 5) continuous amino acid residues in the derivative sequence in comparison to the respective reference sequence, either intrasequentially or at the N- or C-terminus.

The term "insertion" as used herein refers preferably to the additional intrasequential presence of 1, 2, 3, 4, 5 (or even more than 5) continuous amino acid residues in the derivative sequence in comparison to the respective reference sequence.

The term "addition" as used herein refers preferably to the additional presence of 1, 2, 3, 4, 5 (or even more than 5) continuous amino acid residues at the N- and/or C-terminus of the derivative sequence in comparison to the respective reference sequence.

The term "substitution" as used herein refers to the presence of an amino acid residue at a certain position of the derivative sequence which is different from the amino acid residue which is present or absent at the corresponding position in the reference sequence. As mentioned above, preferably such substitutions are conservative substitutions.

The term "cell wall" as used herein refers to all components that form the outer cell enclosure of Gram-negative bacteria and thus guarantee their integrity. In particular, the term "cell wall" as used herein refers to peptidoglycan, the outer membrane of the Gram-negative bacteria with the lipopolysaccharide, the bacterial cell membrane, but also to additional layers deposited on the peptidoglycan as e.g. capsules, outer protein layers or slimes.

The term "amino acid sequence stretch" as used herein refers to a particular stretch of amino acid sequence in the amino acid sequence of the polypeptide of the invention. Said sequence refers to a sequence of a cationic peptide, a polycationic peptide, an amphiphatic peptide, a hydrophobic peptide, a sushi peptide and/or an antimicrobial peptide. The term does not refer to conventional tags like His-tags, such as His5-tags, His6-tags, His7-tags, His8-tags, His9-tags, His10-tags, His11-tags, His12-tags, His16-tags and His20-tags, Strep-tags, Avi-tags, Myc-tags, Gst-tags, JS-tags, cystein-tags, FLAG-tags or other tags known in the art, thioredoxin or maltose binding proteins (MBP). Preferably an amino acid sequence stretch as used herein as a length of about 6 to about 39 amino acid residues.

As used herein, the term "cationic peptide" refers preferably to a peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides, but also includes cationic peptides which comprise for example less than 20%, preferably less than 10% positively charged amino acid residues.

The term "polycationic peptide" as used herein refers preferably to a peptide composed of mostly positively charged amino acid residues, in particular lysine and/or arginine residues. A peptide is composed of mostly positively charged amino acid residues if at least about 20, 30, 40, 50, 60, 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers preferably to any naturally occurring peptide that has microbicidal and/or microbistatic activity on for example bacteria, viruses, fungi, yeasts, mycoplasma and protozoa. Thus, the term "antimicrobial peptide" as used herein refers in particular to any peptide having anti-bacterial, anti-fungal, anti-mycotic, anti-parasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties. Preferred are anti-bacterial peptides. The antimicrobial peptide may be a member of the RNase A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in insects, fish, plants, arachnids, vertebrates or mammals. Preferably the antimicrobial peptide may be naturally occurring in radish, silk moth, wolf spider, frog, preferably in Xenopus laevis, Rana frogs, more preferably in Rana catesbeiana, toad, preferably Asian toad Bufo bufo gargarizans, fly, preferably in Drosophila, more preferably in Drosophila melanogaster, in Aedes aegypti, in honey bee, bumblebee, preferably in Bombus pascuorum, flesh fly, preferably in Sarcophaga peregrine, scorpion, horseshoe crab, catfish, preferably in Parasilurus asotus, cow, pig, sheep, porcine, bovine, monkey and human. As used herein, an "antimicrobial peptide" (AMP) may in particular be a peptide which is not a cationic peptide, polycationic peptide, amphiphatic peptide, sushi peptide, defensins, and hydrophobic peptide, but nevertheless exhibits antimicrobial activity.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "amphiphatic peptide" as used herein refers to synthetic peptides having both hydrophilic and hydrophobic functional groups. Preferably, the term "amphiphatic peptide" as used herein refers to a peptide having a defined arrangement of hydrophilic and hydrophobic groups e.g. amphiphatic peptides may be e.g. alpha helical, having predominantly non polar side chains along one side of the helix and polar residues along the rest of its surface.

The term "hydrophobic group" as used herein refers preferably to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a non-aqueous environment. Examples of amino acid residues with hydrophobic side chains are valine, isoleucine, leucine, methionine, phenylalanine, tryptophan, cysteine, alanine, tyrosine, and proline residues

The term "hydrophobic peptide" as used herein refers to a hydrophobic peptide, which is preferably composed of mostly amino acid residues with hydrophobic groups. Such peptide is preferably composed of mostly hydrophobic amino acid residues, i.e. at least about 20, 30, 40, 50, 60, 70, 75, 80, 85, 90, 95 or at least about 100 % of the amino acid residues are hydrophobic amino acid residues. The amino acid residues being not hydrophobic are preferably neutral and preferably not hydrophilic.

As used herein, the term "tag" refers to an amino acid sequence, which is typically in the art fused to or included in another amino acid sequence for a) improving expression of the overall amino acid sequence or polypeptide, b) facilitating purification of the overall amino acid sequence or polypeptide, c) facilitating immobilisation of the overall amino acid sequence or polypeptide, and/or d) facilitating detection of the overall amino acid sequence or polypeptide. Examples for tags are His tags, such as His5-tags, His6-tags, His7-tags, His8-tags, His9-tags, His10-tags, His11-tags, His12-tags, His16-tags and His20-tags, Strep-tags, Avi-tags, Myc-tags, GST-tags, JS-tags, cystein-tags, FLAG-tags, HA-tags, thioredoxin or maltose binding proteins (MBP), CAT, GFP, YFP, etc. The person skilled in the art will know a vast number of tags suitable for different technical applications. The tag may for example make such tagged polypeptide suitable for e.g. antibody binding in different ELISA assay formats or other technical applications.

The term "comprising" as used herein shall not be construed as being limited to the meaning "consisting of" (i.e. excluding the presence of additional other matter). Rather, "comprising" implies that optionally additional matter may be present. The term "comprising" encompasses as particularly envisioned embodiments falling within its scope "consisting of" (i.e. excluding the presence of additional other matter) and "comprising but not consisting of" (i.e. requiring the presence of additional other matter), with the former being more preferred.

The polypeptide according to the present invention exhibits in the amino acid sequence exhibiting at least 90% sequence identity with the sequence of SEQ ID NO: 4 at least one of the following: X120 is G; X126 is E; X128 is K; X134 is I; X135 is not C; X138 is L; X171 is G; X173 is T; X182 is T; X187 is G; X197 is S; X198 is A; X204 is Y; X230 is L; X231 is Y; and/or X234 is A. It is understood that the number indicating the position of the respective amino acid residue indicates the relative position in the sequence corresponding to SEQ ID NO: 4, and not to the overall amino acid sequence of the polypeptide according to the present invention, which may be longer.

The inventive polypeptide exhibits said at least 90% sequence identity. The inventive polypeptide may thus for example exhibit a higher level of sequence identity, e.g. may exhibit at least 95%, at least 97%, at least 98%, at least 98,5%, at least 98,75%, at least 99% (e.g. less than 3 amino acids deviation), at least 99,5% (e.g. less than 2 amino acids deviation) or even 100% sequence identity with the sequence of SEQ ID NO: 4.

An inventive polypeptide comprising a sequence sharing a given level of sequence identity with the sequence of SEQ ID NO: 4 can for example deviate from the reference sequence by addition, substitution, insertion or deletion of one or more amino acid residues and all possible combinations thereof. Only for the sake of clarity it is pointed out that such combinations refer to distinct positions in the sequence. A "deletion" followed by "addition", or "addition" followed by "deletion", of one or more amino acids, at the same relative position, is not an combination of an "addition" and "deletion" (or vice versa) but falls under the term "substitution". Preferably, the deviations in sequence from the sequence of SEQ ID NO: 4 (or more specific sequences thereof, see below) will be of conservative nature, e.g. conservative substitutions. Even more preferably the deviation in sequence is limited to those positions in SEQ ID NO: 4 (or more specific sequences thereof, see below), which have been identified to be non-critical for the enzymatic activity, i.e. X1, X120, X126, X128, X134, X135, X138, X171, X173, X182, X187, X197, X198, X204, X230, X231, and/or X234.

The polypeptide according to the present invention does not exhibit in the amino acid sequence exhibiting at least 90% sequence identity with the sequence of SEQ ID NO: 4 an alanine residue at position 155. As shown in the publication Briers et al. (Molecular Microbiology (2007) 65(5), 1334-1344), the mutation E155A led to a loss in activity. Thus, such mutation is clearly not desired in the inventive polypeptide. Other E155 mutations may be tolerable, but preferably said residue is unmutated, i.e. is E in an inventive polypeptide.

The inventors of the present invention have found out that a cysteine residues in the amino acid sequence of SEQ ID NO: 3 (EL188 endolysin sequence) is not essential for the enzymatic activity. Thus, in the sequence corresponding to SEQ ID NO: 4 of the inventive polypeptide (or sharing at least 90% sequence identity therewith), in some embodiments X135 is not C. In principle said amino acid residue can be deleted or substituted by any other amino acid. However, a conservative amino acid substitution is preferred. Particularly preferred is a substitute of a serine residue for the cysteine residue. Thus, in particularly preferred examples of the present invention X135 is S. Absence of this cysteine residue has the advantage that the risk of aggregation of the polypeptide according to the present invention, e.g. by undesired disulfide bridge formation, is reduced.

Aside of the dispensability of the above referenced cysteine residues, the inventors of the present invention have also elucidated that various other residues in the sequence of SEQ ID NO: 3 are also not essential and, moreover, may be replaced by other residues, thereby increasing in some cases even the temperature stability of the inventive polypeptide. Examples for such substitutions are X120G, X126E, X128K, X134I, X138L, X171G, X173T, X182T, X187G, X197S, X198A, X204Y, X230L, X231Y, X234A, and/or X241H.. These substitutions may be present alone or in any combination. A typical combination is the combination of X126E and X128K. Other examples of combinations are, without being limited thereto, X197S and X198A; X187G and X230L; and X182T and X187G and X230L. Of course, this second type of amino acid modifications may be combined with the above mentioned cysteine replacement in any type of combination conceivable.

In SEQ ID NO: 1 the first amino acid residue is indicated as being either absent or any amino acid, in particular M. The results of the inventors, and of previous work (see WO 2010/149792) show, that the N-terminal methionine of EL188 is dispensable. Thus, in some embodiments of the present invention the position of X1 in the sequence corresponding to SEQ ID NO: 4 in the inventive polypeptide is not M. If the polypeptide of the present invention exhibits for example N-terminally of the sequence corresponding to SEQ ID NO: 4 further sequence elements, it may for instance for the purpose of effective expression in a host cell be useful, if the methionine at position 4 of SEQ ID NO: 1 is eliminated or replaced by another amino acid in order to avoid a starting codon in the corresponding nucleic acid sequence, potentially leading to parallel expression of a polypeptide lacking the further sequence elements located more N-terminally. On the other hand, if there are no further N-terminal sequence elements in the inventive polypeptide, X1 is of course preferably methionine (e.g. for expression purposes). For the enzymatic activity X1 is however never required.

Sequences falling under the definition of SEQ ID NO: 1, which have been particularly tested by the inventors, are for instance SEQ ID NOs: 4-19 (and corresponding sequences without N-terminal methionine, SEQ ID NOs: 20-35).

The polypeptide according to the present invention may comprise aside of the enzymatic amino acid sequence, e.g. the sequence exhibiting at least 90% sequence identity with the sequence of SEQ ID NO: 4, further amino acid sequence stretches, e.g. as already disclosed in similar fashion in WO 2010/149792. The polypeptide according to the present invention may for example comprise additionally at least one amino acid sequence stretch selected from the group consisting of amphiphatic peptide, cationic peptide, polycationic peptide, hydrophobic peptide, or naturally occurring antimicrobial peptide, like sushi peptide and defensin. Such additional amino acid sequence stretches may improve the antibacterial properties of the inventive polypeptide. In some embodiments, the inventive polypeptide may comprise at least two distinct amino acid sequence stretches selected from the group of amphiphatic peptide, cationic peptide, polycationic peptide, hydrophobic peptide, or naturally occurring antimicrobial peptide, like sushi peptide and defensin.

These one or more additional amino acid sequence stretches may be present N-terminally or C-terminally of the sequence exhibiting at least 90% sequence identity with the sequence of SEQ ID NO: 4. They may for example be located at the N- or C-terminus of the inventive polypeptide. Preferred examples of such additional amino acid sequence stretches (without being limited thereto), are the sequence KRK and SEQ ID NOs: 36-106, as set out in more detail below. The polypeptide according to the present invention may comprise at least one additional amino acid sequence stretch selected from this group. For further guidance, in particular with respect to the generic and specific nature of possible additional amino acid sequence stretches, see for example also WO 2010/023207, WO 2010/149792, WO 2010/149795 and WO 2012/085259.

Examples for cationic and polycationic amino acid sequence stretches are listed in the following table.

**Table 1:**

| **amino acid sequence stretch** | **length** | **SEQ ID NO:** |
|---|---|---|
| KRKKRK | 6 | SEQ ID NO: 36 |
| KRXKR | 5 | SEQ ID NO: 37 |
| KRSKR | 5 | SEQ ID NO: 38 |
| KRGSG | 5 | SEQ ID NO: 39 |
| KRKKRKKRK | 9 | SEQ ID NO: 40 |
| RRRRRRRRR | 9 | SEQ ID NO: 41 |
| KKKKKKKK | 8 | SEQ ID NO: 42 |
| KRKKRKKRKK | 10 | SEQ ID NO: 43 |
| KRKKRKKRKKRK | 12 | SEQ ID NO: 44 |
| KRKKRKKRKKRKKR | 14 | SEQ ID NO: 45 |
| KKKKKKKKKKKKKKKK | 16 | SEQ ID NO: 46 |
| KRKKRKKRKKRKKRKKRK | 18 | SEQ ID NO: 47 |
| KRKKRKKRKKRKKRKKRKK | 19 | SEQ ID NO: 48 |
| RRRRRRRRRRRRRRRRRRR | 19 | SEQ ID NO: 49 |
| KKKKKKKKKKKKKKKKKKK | 19 | SEQ ID NO: 50 |
| KRKKRKKRKRSKRKKRKKRK | 20 | SEQ ID NO: 51 |
| KRKKRKKRKRSKRKKRKKRKK | 21 | SEQ ID NO: 52 |
| KRKKRKKRKKRKKRKKRKKRK | 21 | SEQ ID NO: 53 |
| KRKKRKKRKRGSGKRKKRKKRK | 22 | SEQ ID NO: 54 |
| KRKKRKKRKRGSGSGKRKKRKKRK | 24 | SEQ ID NO: 55 |
| KRKKRKKRKKRKKRKKRKKRKKRKK | 25 | SEQ ID NO: 56 |
| KRKKRKKRKRSKRKKRKKRKRSKRKKRKKRK | 31 | SEQ ID NO: 57 |
| KRKKRKKRKRGSGSGKRKKRKKRKGSGSGKRKKRKKRK | 38 | SEQ ID NO: 58 |
| KRKKRKKRKKRKKRKKRKKRKKRKKRKKRKKRKKRKKRK | 39 | SEQ ID NO: 59 |
| KRKKRKKRKRSKRKKRKKRKRSKRKKRKKRKRSKRKKRKKRK | 42 | SEQ ID NO: 60 |

Examples for antimicrobial amino acid sequences which may be used in carrying out the present invention are listed in the following table.

**Table 2:**

| **Peptide** | **Sequence** | **SEQ ID NO** |
|---|---|---|
| LL-37 | LLGDFFRKSKEKIGKEFKRIVQRIKDFLRNLVPRTES | SEQ ID NO: 61 |
| SMAP-29 | RGLRRLGRKIAHGVKKYGPTVLRIIRIAG | SEQ ID NO: 62 |
| Indolicidin | ILPWKWPWWPWRR | SEQ ID NO: 63 |
| Protegrin | RGGRLCYCRRRFCVCVGR | SEQ ID NO: 64 |
| Cecropin P1 | SWLSKTAKKLENSAKKRISEGIAIAIQGGPR | SEQ ID NO: 65 |
| Magainin | GIGKFLHSAKKFGKAFVGEIMNS | SEQ ID NO: 66 |
| Pleurocidin | GWGSFFKKAAHVGKHVGKAALTHYL | SEQ ID NO: 67 |
| Cecropin A (A.aegypti) | GGLKKLGKKLEGAGKRVFNAAEKALPVVAGAKALRK | SEQ ID NO: 68 |
| Cecropin A (D. melanogaster) | | SEQ 10 NO: 69 |
| Buforin II | TRSSRAGLQFPVGRVHRLLRK | SEQ ID NO: 70 |
| Sarcotoxin IA | | SEQ ID NO: 71 |
| Apidaecin | ANRPVYIPPPRPPHPRL | SEQ ID NO: 72 |
| Ascaphine 5 | GIKDWIKGAAKKLIKTVASHIANQ | SEQ ID NO: 73 |
| Nigrocine 2 | GLLSKVLGVGKKVLCGVSGLVC | SEQ ID NO: 74 |
| Pseudin 1 | GLNTLKKVFQGLHEAIKLINNHVQ | SEQ ID NO: 75 |
| Ranalexin | FLGGLIVPAMICAVTKKC | SEQ ID NO: 76 |
| Melittin | GIGAVLKVLTTGLPALISWIKRKRQQ | SEQ ID NO: 77 |
| Lycotoxin 1 | IWLTALKFLGKHAAKKLAKQQLSKL | SEQ ID NO: 78 |
| Parasin 1 | KGRGKQGGKVRAKAKTRSS | SEQ ID NO: 79 |
| Buforin I | | SEQ ID NO: 80 |
| Dermaseptin 1 | AL WKTMLKKLGTMALHAGKAALGAAADTISQGTQ | SEQ ID NO: 81 |
| Bactenecin 1 | RLCRIVVIRVCR | SEQ ID NO: 82 |
| Thanatin | GSKKPVPIIYCNRRTGKCQRM | SEQ ID NO: 83 |
| Brevinin 1T | VNPIILGVLPKVCLITKKC | SEQ ID NO: 84 |
| Ranateurin 1 | SMLSVLKNLGKVGLGFVACKINIKQC | SEQ ID NO: 85 |
| Esculentin 1 | | SEQ ID NO: 86 |
| Tachyplesin | RWCFRVCYRGICYRKCR | SEQ ID NO: 87 |
| Androctonin | RSVCRQIKICRRRGGCYYKCTNRPY | SEQ ID NO: 88 |
| alpha-defensin | DCYCRIPACIAGERRYGTCIYQGRLWAFCC | SEQ ID NO: 89 |
| beta-defensin | NPVSCVRNKGICVPIRCPGSMKQIGTCVGRAVKCCRKK | SEQ ID NO: 90 |
| theta-defensin | GFCRCLCRRGVCRCICTR | SEQ ID NO: 91 |
| defensin (sapecin A) | | SEQ ID NO: 92 |
| Thionin (crambin) | | SEQ ID NO: 93 |
| defensin from radish | | SEQ ID NO: 94 |
| Drosomycin | | SEQ ID NO: 95 |
| Hepcidin | DTHFPICIFCCGCCHRSKCGMCCKT | SEQ ID NO: 96 |
| Bac 5 | | SEQ ID NO: 97 |
| PR-39 | | SEQ ID NO: 98 |
| Pyrrhocoricin | VDKGSYLPRPTPPRPIYNRN | SEQ ID NO: 99 |
| Histatin 5 | DSHAKRHHGYKRKFHEKHHSHRGY | SEQ ID NO: 100 |

The at least one additional amino acid sequence stretch may be a sushi peptide which is described by Ding JL, Li P, Ho B Cell Mol Life Sci. 2008 Apr;65(7-8):1202-19. The Sushi peptides: structural characterization and mode of action against Gram-negative bacteria. Especially preferred is the sushi 1 peptide according to SEQ ID NO: 101. Other preferred sushi peptides are sushi peptides S1 and S3 and multiples thereof; FASEB J. 2000 Sep;14(12):1801-13.

Preferred hydrophobic peptides are Walmagh1 having the amino acid sequence according to SEQ ID NO: 102 and the hydrophobic peptide having the amino acid sequence Phe-Phe-Val-Ala-Pro (SEQ ID NO: 103).

Preferred amphiphatic peptides are α4-helix of T4 lysozyme according to SEQ ID NO: 104 and WLBU2-Variant having the amino acid sequence according to SEQ ID NO: 105 and Walmagh 2 according to SEQ ID NO: 106.

As mentioned above, a polypeptide according to the present invention may comprise at least one additional amino acid sequence stretch selected from the group consisting of: KRK and SEQ ID NOs: 36-106. Corresponding examples are for instance polypeptides comprising a sequence selected from the group consisting of SEQ ID NOs: 107-124 (and corresponding sequences without N-terminal methionine, SEQ ID NOs: 125-142.

A polypeptide according to the present invention comprises an amino acid sequence exhibiting at least 90% sequence identity with the sequence of SEQ ID NO: 4, wherein the polypeptide does neither comprise the amino acid sequence of SEQ ID NO: 2 nor of SEQ ID NO: 183, and wherein the polypeptide does not comprise an E155A mutation at position 155 of SEQ ID NO: 4. Thus, a polypeptide of the present invention may also comprise an amino acid sequence exhibiting at least 91,5 % sequence identity with an amino acid sequence selected from any of SEQ ID NOs: 107 - 142, wherein the polypeptide does neither comprise the amino acid sequence of SEQ ID NO: 2 nor of SEQ ID NO: 183, and wherein the polypeptide does not comprise an E155A mutation at position 155 of SEQ ID NO: 4.

Such inventive polypeptide may thus for example comprise a sequence exhibiting a higher level of sequence identity than 91,5% with an amino acid sequence selected from any of SEQ ID NOs: 107 - 142, e.g. may exhibit at least 95%, at least 97%, at least 98%, at least 98,5%, at least 99%, at least 99,25% (e.g. less than 3 amino acids deviation), or at least 99,5% (e.g. less than 2 amino acids deviation) sequence identity with an amino acid sequence selected from any of SEQ ID NOs: 107 - 142.

In addition, and irrespective whether or not one or more additional amino acid sequence stretches as set out above are present in the inventive polypeptide, the polypeptide may comprise additionally one or more tag sequences. Such tag sequence may be present N-terminally or C-terminally of the sequence exhibiting at least 90% sequence identity with the sequence of SEQ ID NO: 4. They may for example be located at the N- or C-terminus of the inventive polypeptide. In a preferred embodiment, the one or more tag sequence is located C-terminally of the amino acid sequence exhibiting at least 90% sequence identity with the sequence of SEQ ID NO: 4.

The one or more tag sequences may for example be linked to the amino acid sequence exhibiting at least 90% sequence identity with the sequence of SEQ ID NO: 4 directly or via a short linker of 1 to 10 amino acid residues, preferably 1 to 5 amino acid residues, even more preferably 1 to 2 amino acids. Linker sequences are preferably flexible sequences, comprising one or more glycine residues. Exemplary sequences, which may be comprised in the polypeptides of the invention, with a C-terminal Leu-Glu linker, e.g. for fusion to a tag or further sequence are given in SEQ ID NOs: 184 to 251 as non-limiting examples.

Numerous examples for tags are known in the art, some of which have already been mentioned above. In the context of the present invention a particularly preferred tag sequence is a His-tag, preferably a His tag according to SEQ ID NO. 143.

The length of the polypeptide according to present invention is in principle not limited, but preferably the length will not be excessively large. Preferably, a polypeptide according to the present invention has an overall length not exceeding about 360 amino acids, preferably not exceeding about 340 amino acids.

Specific examples of polypeptides according to the present invention can be selected from the group consisting of SEQ ID NOs: 144-161 (and corresponding sequences without N-terminal methionine, SEQ ID NOs: 162-179).

A polypeptide according to the present invention comprises an amino acid sequence exhibiting at least 90% sequence identity with the sequence of SEQ ID NO: 41, wherein the polypeptide does neither comprise the amino acid sequence of SEQ ID NO: 2 nor of SEQ ID NO: 183, and wherein the polypeptide does not comprise an E155A mutation at position 155 of SEQ ID NO: 4. Thus, a polypeptide of the present invention may also comprise an amino acid sequence exhibiting at least 91,5 % sequence identity with an amino acid sequence selected from any of SEQ ID NOs: 144 - 179, wherein the polypeptide does neither comprise the amino acid sequence of SEQ ID NO: 2 nor of SEQ ID NO: 183, and wherein the polypeptide does not comprise an E155A mutation at position 155 of SEQ ID NO: 4.

Such inventive polypeptide may thus for example comprise a sequence exhibiting a higher level of sequence identity than 91,5% with an amino acid sequence selected from any of SEQ ID NOs: 144 - 179, e.g. may exhibit at least 95%, at least 97%, at least 98%, at least 98,5%, at least 99%, at least 99,25% (e.g. less than 3 amino acids deviation),or at least 99,5% (e.g. less than 2 amino acids deviation) or even 100% sequence identity with an amino acid sequence selected from any of SEQ ID NOs: 144 - 179. Deviations from SEQ ID NOs: 144 - 179 may in particular occur in the two sequences linking the components addtionial amio acid sequence stretch (i.e. KRKKRKKRK, SEQ ID NO: 40, or SMAP29 peptide; SEQ ID NO: 62), modified EL188 endolysin and His-tag.

A polypeptide according to the present invention is preferably characterized by the ability to degrade the peptidoglycan of Gram-negative bacteria, in particular of *Pseudomonas* and/or *Campylobacter* bacteria. In particular, the polypeptide according to the present invention is preferably capable of degrading the peptidoglycan of *Pseudomonas aeroginosa,* in particular *Pseudomonas aeroginosa* PAO1, *Campylobacter jejuni* and/or *Campylobacter coli.*

The peptidoglycan degrading activity on gram negative bacteria can be measured by assays well known in the art, e.g. by muralytic assays in which the outer membrane of gram negative bacteria is permeabilized or removed (e.g. with chloroform) to allow the putative enzyme access to the peptidoglycan layer. If the enzyme is active, degradation of the peptidoglycan layer will lead to a drop of turbidity, which can be measured photometrically (see for example Briers et al., J. Biochem. Biophys Methods 70: 531-533, (2007*).*

In a further aspect the present invention relates to a nucleic acid encoding a polypeptide according to the present invention. A person skilled in the art, having the degeneracy of the genetic code in mind, will be aware of means to generate such nucleic acid.

In a further aspect, the present invention relates to a vector, such as an expression or cloning vector, which comprises a nucleic acid according to the present invention.

In a further aspect, the present invention relates to a host cell comprising a polypeptide according to the present invention, a nucleic acid according to the present invention, and/or a vector according to the present invention.

In a further aspect, the present invention relates to composition comprising a polypeptide according to the present invention, a nucleic acid according to the present invention, a vector according to the present invention, and/or a host cell according to the present invention. Preferably, said composition is a pharmaceutical composition comprising a pharmaceutical acceptable diluent, excipient or carrier.

### Examples

In the following, specific examples illustrating various embodiments and aspects of the invention are presented. However, the present invention shall not to be limited in scope by the specific embodiments described herein. Indeed, various modifications of the invention in addition to those described herein will become readily apparent to those skilled in the art from the foregoing description, accompanying figures and the examples below. All such modifications fall within the scope of the appended claims.

### Example 1: Identification of tolerable mutations in EL188 endolysin

For the construction of polypeptides according to the present invention the lytic enzyme (gp188) of the Pseudomonas aeruginosa phage EL was used as starting point (SEQ ID NO: 3. This endolysin consists of 292 amino acids (molecular weight: 32 kDa).

In some embodiments, KRKKRKKRK (SEQ ID NO: 40) was used as fusion partner.

In other embodiments, SMAP-29 was chosen as fusion partner. SMAP-29 was found in sheep leukocytes and consists of 29 amino acids (RGLRRLGRKIAHGVKKYGPTVLRIIRIAG; molecular weight: 3.3 kDa: SEQ ID NO: 62).

Respective fusions of modified EL188 with KRKKRKKRK (SEQ ID NOs: 144-157) and with SMAP-29 (SEQ ID NOs: 158-161 were created. As controls served fusions of unmodified EL188 3 with KRKKRKKRK (SEQ ID NO: 180) and with SMAP-29 (SEQ ID NOs: 181-182).

The corresponding nucleic acid constructs were constructed using standard cloning techniques as described e.g. by Sambrook et al. 2001, Molecular Cloning: A Laboratory Manual and quick change mutagenesis.

### Mutations:

The following modified EL188 endolysins were constructed:

**Table 3:**

| **SEQ ID NO:** | **Mutation*** |
|---|---|
| 180 | --- |
| 144 | S120G |
| 145 | A126E |
| | T128K |
| 146 | W134I |
| 147 | H138L |
| 148 | E171G |
| 149 | Y173T |
| 150 | A182T |
| 151 | N187G |
| 152 | D197S |
| | Q198A |
| 153 | L204Y |
| 154 | F230L |
| 155 | H231Y |
| 156 | S234A |
| 157 | C135S |
| 158 | A182T |
| 159 | N187G |
| 160 | N187G |
| | F230L |
| 161 | A182T |
| | N187G |
| | F230L |

| | |
|---|---|
| *Please note, the position indicated refers to the position in the sequence of EL188 sequence, SEQ ID NO: 3, and not to the position in the respective SEQ ID NO: indicated in the first column. | |

### Purification

Recombinant expression of the fusion proteins was done in *E. coli* BL21(DE3)pLysS cells (Novagen, Darmstadt, Germany). The cells were grown until an optical density of OD₆₀₀ₙₘ = 0.5-0.8 was reached. Then the expression of the fusion protein was induced with 0.5 mM IPTG (isopropylthiogalactoside) and the expression was performed at 16-18°C overnight, but at least 12h.

Cells were harvested by centrifugation for 20 min at 6000g and disrupted via sonication on ice. Soluble and insoluble fraction of the *E.coli* crude extract were separated by centrifugation (Sorvall, SS34, 30 min, 15 000 rpm). All proteins were purified by Ni²⁺ affinity chromatography (Äkta FPLC, GE Healthcare) using the C-terminal His₆ tag, encoded by the pET21b vector. Samples were microfiltrated (0.2 µm) before every chromatographic step.

The Ni²⁺ affinity chromatography is performed in 4 subsequent steps, all at room temperature:
1. *Equilibration* of the Histrap FF 5 ml column (GE Healthcare) with up to 10 column volumes of Washing Buffer (20 mM imidazole, 1 M NaCl and 20 mM HEPES on pH 7.4) at a flow rate of 3-5 ml/min.
2. *Loading* of the total lysate with wanted target protein on the Histrap FF 5 ml column at a flow rate of 3-5 ml/min.
3. *Washing* of the column with up to 10 column volumes of Washing Buffer to remove unbound protein.
4. Elution of bounded target protein from the column with an increasing linear gradient of 15 column volumes of Elution Buffer (500 mM imidazole, 1M NaCl and 20 mM HEPES on pH 7.4) to 100% at a flow rate of 3-5 ml/min.

The *Hydrophobic Interaction Chromatography* (HIC) is performed in 5 subsequent steps, all at room temperature:
1. *Equilibration* of the HiScreen Phenyl HP 5 ml column (GE Healthcare) with up to 5 column volumes of Washing Buffer (500mM ammonium sulfate, 1M NaCl and 20mM HEPES; pH 7.4) at a flow rate of 1-2ml/min
*2. Preparation* of the sample (5mg per 1ml column volume of the protein pool from Ni²⁺ affinity step) starts by first setting the protein concentration to 0.5mg/ml by adding a predefined amount of Washing Buffer from the Ni²⁺ Affinity step. Followed by adjusting the ammonium sulfate concentration by stepwise adding of a predefined amount of ammonium sulfate stock solution (3.8M) to a final concentration of approx. 500mM.
3. *Loading* of the prepared sample on the *HiScreen Phenyl HP 5 ml* column at a flowrate of 1-2ml/min.
4. *Washing* of the column with 5 column volumes of Washing Buffer to remove unbound protein.
5. *Elution* of the target protein from the column with a decreasing linear gradient of 15 column volumes of Elution Buffer (500mM NaCl and 20mM HEPES; pH 7.4) to 0% at a flow rate of 1-2 ml/min. The target protein is eluted in the second peak close to the end of the decreasing gradient.

### Buffer change by membrane dialysis:

The elution pool of the HIC step is dialyzed (membrane: regenerated cellulose with MWCO: 6000-8000D) into storage buffer (750mM NaCl and 20mM HEPES; pH7.4) at 4°C. Dialysis factor is 160 - 250.

### Characterisation

Activity of the polypeptides of SEQ ID NOs: 144 - 161 as well as the control of SEQ ID NO: 180 was characterized in a muralytic activity assay.

### Muralytic Assay

*Ps. aeruginosa* cells were prepared from overnight culture as follows:
1. *Ps. aeruginosa* cells were grown to OD600=0.5-0.8. in 50ml LB
2. The cells were pelleted by centrifugation at 4°C 5min, 6000g, supernatant was removed.
3. The pellet was washed in 50ml ChCl3-buffer (same volume as liquid culture) 20mM HEPES, 150mM NaCl, ChCl3 saturated, pH=7.4
   the following way: the cell pellet was resuspended by pipetting up and down and incubated at room temperature 45minutes. Afterwards, the cells were pelleted by centrifugation at 4°C 10min, 3000g to remove the supernatant.
4. The resulting pellet was washed again with 50ml of sample buffer (same volume as liquid culture).
   20mM HEPES, 150mM NaCl, pH=7.4
   The cell pellet was resuspended by pipetting up and down, afterwards the cells were pelleted again by centrifugation at 4°C 10min, 3000g to remove the supernatant.
5 In the last step the pellet was resuspended in X ml of sample buffer 20mM HEPES, 150mM NaCl, pH=7.4.
The volume X was adjusted so, that 1ml of the suspension has OD600 ∼ 1.
The cells were pelleted by centrifugation at 4°C 10min, 3000g, to remove the supernatant frozen and stored at -20°C.

For the measurement of muralytic activity the following procedure was applied:
1. Cell pellets were resuspended in 1ml 20 mM HEPES, pH 7,4 to yield an OD600 of ca. 1.0 and transferred into a measurement cuvette.
2. The polypeptide of interest was added to the cell suspension in small volume and mixed with the cells.
3. The drop of optical density at 600nm was recorded for 10 minutes, starting immediately.
4. The muralytic activity of the protein was calculated as optical density drop at 600nm per minute per milligram of added polypeptide (dAbs/min/mg).

Additionally, melting temperature values for the polypeptides of SEQ ID NOs:_144 - 161 as well as the controls of SEQ ID NOs: 180-182 were determined.

The protein melting temperature was determined by circular dichroism (CD). Changes of ellipticity for the proteins were recorded at 220nm as a function of temperature using Jasco J-815 CD spectrometer and fitted to a simple sigmoid unfolding model using JASCO analysis software. The protein melting temperatures (Tmelt) were determined as midpoint of unfolding transition. The spectra were recorded at protein concentrations of 5.0-5.8µM with a heating rate of 1°C/min and incubation time of 3s in 410µl volume in a 1mm light path Hellma quartz cuvette. Measurements were performed in 50mM NaPh buffer, 300mM NaCl at pH of 7.4.

For the polypeptides of SEQ ID NOs:_144 - 157 and the control of SEQ ID NO: 180 the results are indicated in Fig.3. For the polypeptides of SEQ ID NOs: 158 - 161 and the controls of 181 and 182 the results are given in the table below:

| **SEQ ID NO:** | **Concentration** | **Buffer** | **Tm [°C]** |
|---|---|---|---|
| 158 | 5,65µM | 50mM NaPh, pH 7.45 | 51°C |
| | | 300mM NaCl | 51,2°C |
| 159 | 5,4µM | 50mM NaPh, pH 7.44 | 50,32°C |
| | | 300mM NaCl | 50,37°C |
| 160 | 5,7µM | 50mM NaPh, pH 7.45 | 51,48°C |
| | | 300mM NaCl | |
| 161 | 5,65µM | 50mM NaPh, pH 7.45 | 51,58°C |
| | | 300mM NaCl | |
| 181 | 5,1 µM | 50mM NaPh, pH 7.45 | 49,44 |
| | | 300mM NaCl | |
| 182 | 5,32 µM | 50mM NaPh, pH 7.45 | 49.6°C |
| | | 300mM NaCl | |

The results showed that the mutations introduced did thus not negatively affect activity of the polypeptide. Rather they did even enhance the activity. Moreover, some of the polypeptides increased favorably the melting temperature of the endolysin.

### Example 2: Temperature stability of the polypeptide according to SEQ ID NO: 161

In order to illustrate that the increased melting temperature does indeed affect temperature stability, the polypeptide of SEQ ID NO: 161 was subjected to prolonged direct heating at temperatures of 51°C and 52°C. Activity tests were performed on *Pseudomonas aeruginosa* strain as a model system at adapted conditions.

### Determination of the Minimal Inhibitory Concentration (MIC)

In analogy to the determination of the "Minimum inhibitory concentration (MIC)" for antibiotics, the MIC was determined as a microdilution test.

The setup of the experiment is the following:
The respective overnight culture was diluted 1:10. *Ps. aeruginosa* was incubated at 37°C up to OD600=0.6 (approx. 10⁹ cells/ml). The bacterial culture was diluted to a concentration of 2x10⁵ to 8x10⁵ colony-forming-units per ml in Mueller-Hinton-broth (not cation-adjusted Mueller-Hinton-broth) and split in the required amount of tubes.

The polypeptide of interest was added in different concentrations (determined as µg/ml final concentration in the Mueller-Hinton-broth). EDTA was added to a final concentration of 2 mM.

The mixture was incubated overnight at 37°C. Bacterial growth was visibly determined by turbidity (in comparison to negative control). The MIC was defined as the concentration in the tube where no bacterial growth was observed. Positive (without polypeptide of interest and/or EDTA) and negative control (Mueller-Hinton-broth without bacteria) were included in the experiment.

Activity of the polypeptide of SEQ ID NO: 161, measured as minimal inhibitory concentration (MIC), shows that SEQ ID NO: 161 retains 80% of its initial activity even after direct heating at 51°C for 1min.

| | **Heating** | SEQ ID NO: 161 **MIC µg/ml** |
|---|---|---|
| ***P.aeruginosa PAO1*** | not heated | 12,5 |
| | 1min. 51°C | 15 |
| | 2min. 51°C | >20 |
| | 2min. 52°C | >20 |

## Claims

1. Polypeptide comprising an amino acid sequence exhibiting at least 90% sequence identity with the sequence of SEQ ID NO: 4,
wherein the polypeptide does neither comprise the amino acid sequence of SEQ ID NO: 2 nor of SEQ ID NO: 183, wherein the polypeptide does not comprise an E155A mutation at position 155 of SEQ ID NO: 4,
wherein the polypeptide exhibits in the amino acid sequence exhibiting at least 90% sequence identity with the sequence of SEQ ID NO: 4 at least one of the following:
| | |
|---|---|
| the amino acid at position 120 | is G |
| the amino acid at position 126 | is E |
| the amino acid at position 128 | is K |
| the amino acid at position 134 | is I |
| the amino acid at position 135 | is not C |
| the amino acid at position 138 | is L |
| the amino acid at position 171 | is G |
| the amino acid at position 173 | is T |
| the amino acid at position 182 | is T |
| the amino acid at position 187 | is G |
| the amino acid at position 197 | is S |
| the amino acid at position 198 | is A |
| the amino acid at position 204 | is Y |
| the amino acid at position 230 | is L |
| the amino acid at position 231 | is Y |
| the amino acid at position 234 | is A, and |
wherein the said amino acid sequence exhibiting at least 90% sequence identity with the sequence of SEQ ID NO: 4 exhibits improved temperature stability and/or muralytic activity as compared to the amino acid sequence of SEQ ID NO: 2.

2. The polypeptide according to claim 1, wherein the polypeptide exhibits in the amino acid sequence exhibiting at least 90% sequence identity with the sequence of SEQ ID NO: 4 a glutamic acid residue at position 155.

3. The polypeptide according to claim 1 or 2, wherein the sequence exhibiting at least 90% sequence identity with the sequence of SEQ ID NO: 4 deviates from the sequence of SEQ ID NO: 4 only at residues 1, 120, 126, 128, 134, 135, 138, 171, 173, 182, 187, 197, 198, 204, 230, 231, and/or 234.

4. The polypeptide according to claim 1 or 2, wherein the polypeptide comprises a sequence selected from the group consisting of SEQ ID NOs: 4-35.

5. The polypeptide according to any one of the preceding claims, wherein the polypeptide comprises at least one additional amino acid sequence stretch selected from the group consisting of: KRK and SEQ ID NOs: 36 - 106.

6. The polypeptide according to any one of the preceding claims, wherein the polypeptide comprises at least one additional amino acid sequence stretch having the amino acid sequence of SMAP-29, SEQ ID NO: 62 or the amino acid sequence of KRKKRKKRK, SEQ ID NO: 40.

7. The polypeptide according to any one of the preceding claims, wherein the polypeptide comprises an amino acid sequence exhibiting at least 91,5% sequence identity with an amino acid sequence selected from any of SEQ ID NOs: 107-142, and wherein the polypeptide does neither comprise the amino acid sequence of SEQ ID NO: 2 nor of SEQ ID NO: 183, and wherein the polypeptide does not comprise an E155A mutation at the respective position corresponding to position 155 of SEQ ID NO: 4, but exhibits preferably an glutamic acid residue at said position.

8. The polypeptide according to claim 7, wherein the polypeptide comprises an amino acid sequence exhibiting at least 95%, at least 97%, at least 98%, at least 98,5%, at least 99%, at least 99,25%,or at least 99,5% sequence identity with an amino acid sequence selected from any of SEQ ID NOs: 107-142.

9. The polypeptide according to any one of the preceding claims, wherein the polypeptide comprises a sequence selected from the group consisting of SEQ ID NOs: 107-142.

10. The polypeptide according to any one of the preceding claims, wherein the polypeptide comprises a sequence selected from the group consisting of SEQ ID NOs: 144-179.

11. Nucleic acid encoding a polypeptide according to any one of claims 1 to 10.

12. Vector comprising a nucleic acid according to claim 11.

13. Host cell comprising a polypeptide according to any one of claims 1 to 10, a nucleic acid according to claim 11, and/or a vector according to claim 12.

14. Composition comprising a polypeptide according to any one of claims 1 to 10, a nucleic acid according to claim 11, a vector according to claim 12 and/or a host cell according to claim 13.

15. Composition according to claim 14, wherein the composition is a pharmaceutical composition comprising a pharmaceutical acceptable diluent, excipient or carrier.

## Patentansprüche

1. Polypeptid umfassend eine Aminosäuresequenz, die wenigstens 90% Sequenzidentität mit der Sequenz von SEQ ID NO: 4 zeigt,
wobei das Polypeptid weder die Aminosäuresequenz von SEQ ID NO: 2 noch von SEQ ID NO: 183 umfasst, wobei das Polypeptid keine E155A-Mutation an Position 155 von SEQ ID NO: 4 umfasst,
wobei das Polypeptid in der Aminosäuresequenz, die wenigstens 90% Sequenzidentität mit der Sequenz von SEQ ID NO: 4 zeigt, wenigstens eines des nachfolgenden zeigt:
| | |
|---|---|
| die Aminosäure an Position 120 | G ist |
| die Aminosäure an Position 126 | E ist |
| die Aminosäure an Position 128 | K ist |
| die Aminosäure an Position 134 | I ist |
| die Aminosäure an Position 135 | nicht C ist |
| die Aminosäure an Position 138 | L ist |
| die Aminosäure an Position 171 | G ist |
| die Aminosäure an Position 173 | T ist |
| die Aminosäure an Position 182 | T ist |
| die Aminosäure an Position 187 | G ist |
| die Aminosäure an Position 197 | S ist |
| die Aminosäure an Position 198 | A ist |
| die Aminosäure an Position 204 | Y ist |
| die Aminosäure an Position 230 | L ist |
| die Aminosäure an Position 231 | Y ist |
| die Aminosäure an Position 234 | A ist, und |
wobei besagte Aminosäuresequenz, die wenigstens 90% Sequenzidentität mit der Sequenz von SEQ ID NO: 4 zeigt, eine verbesserte Temperaturstabilität und/oder muralytische Aktivität verglichen mit der Aminosäuresequenz von SEQ ID NO: 2 zeigt.

2. Das Polypeptid gemäß Anspruch 1, wobei das Polypeptid in der Aminosäuresequenz, die wenigstens 90% Sequenzidentität mit der Sequenz von SEQ ID NO: 4 zeigt, einen Glutaminsäure-Rest an Position 155 zeigt.

3. Das Polypeptid gemäß Anspruch 1 oder 2, wobei die Sequenz, die wenigstens 90% Sequenzidentität mit der Sequenz von SEQ ID NO: 4 zeigt, nur bei Resten 1, 120, 126, 128, 134, 135, 138, 171, 173, 182, 187, 197, 198, 204, 230, 231 und/oder 234 von der SEQ ID NO: 4 abweicht.

4. Das Polypeptid gemäß Anspruch 1 oder 2, wobei das Polypeptid eine Sequenz umfasst, ausgewählt aus der Gruppe, bestehend aus SEQ ID NO: 4 bis 35.

5. Das Polypeptid gemäß einem der vorhergehenden Ansprüche, wobei das Polypeptid wenigstens einen weiteren Aminosäuresequenzabschnitt umfasst, ausgewählt aus der Gruppe, bestehend aus: KRK und SEQ ID NOs: 36 bis 106.

6. Das Polypeptid gemäß einem der vorhergehenden Ansprüche, wobei das Polypeptid wenigstens einen weiteren Aminosäuresequenzabschnitt mit der Aminosäuresequenz von SMAP-29, SEQ ID NO: 62 oder der Aminosäuresequenz von KRKKRKKRK, SEQ ID NO: 40, umfasst.

7. Das Polypeptid gemäß einem der vorhergehenden Ansprüche, wobei das Polypeptid eine Aminosäuresequenz umfasst, die wenigstens 91,5% Sequenzidentität mit einer Aminosäuresequenz, ausgewählt aus einer beliebigen von SEQ ID NOs: 107 bis 142, zeigt, und wobei das Polypeptid weder die Aminosäuresequenz von SEQ ID NO: 2 noch von SEQ ID NO: 183 umfasst, und wobei das Polypeptid keine E155A-Mutation an der jeweiligen Position, die Position 155 von SEQ ID NO: 4 entspricht, sondern vorzugsweise einen Glutaminsäure-Rest an besagter Position umfasst.

8. Das Polypeptid gemäß Anspruch 7, wobei das Polypeptid eine Aminosäure umfasst, die wenigstens 95%, wenigstens 97%, wenigstens 98%, wenigstens 98,5%, wenigstens 99%, wenigstens 99,25%, wenigstens 99,5% Sequenzidentität mit einer Aminosäuresequenz zeigt, ausgewählt aus einer beliebigen von SEQ ID NOs: 107 bis 142.

9. Das Polypeptid gemäß einem der vorhergehenden Ansprüche, wobei das Polypeptid eine Sequenz umfasst, ausgewählt aus der Gruppe, bestehend aus SEQ ID NOs: 107 bis 142.

10. Das Polypeptid gemäß einem der vorhergehenden Ansprüche, wobei das Polypeptid eine Sequenz umfasst, ausgewählt aus der Gruppe, bestehend aus SEQ ID NOs: 144 bis 179.

11. Nukleinsäure codierend ein Polypeptid gemäß einem der Ansprüche 1 bis 10.

12. Vektor umfassend eine Nukleinsäure gemäß Anspruch 11.

13. Wirtszelle umfassend ein Polypeptid gemäß einem der Ansprüche 1 bis 10, eine Nukleinsäure gemäß Anspruch 11 und/oder einen Vektor gemäß Anspruch 12.

14. Zusammensetzung umfassend ein Polypeptid gemäß einem der Ansprüche 1 bis 10, eine Nukleinsäure gemäß Anspruch 11, einen Vektor gemäß Anspruch 12 und/oder eine Wirtszelle gemäß Anspruch 13.

15. Zusammensetzung gemäß Anspruch 14, wobei die Zusammensetzung eine pharmazeutische Zusammensetzung ist, umfassend ein(en) pharmazeutisch verträgliches/n Verdünnungsmittel, Exzipienten oder Träger.

## Revendications

1. Polypeptide comprenant une séquence d'acides aminés présentant une identité de séquence d'au moins 90 % avec la séquence de la SEQ ID NO : 4,
lequel polypeptide ne comprend ni la séquence d'acides aminés de la SEQ ID NO : 2 ni de la SEQ ID NO : 183, lequel polypeptide ne comprend pas une mutation E155A à la position 155 de la SEQ ID NO : 4,
lequel polypeptide présente, dans la séquence d'acides aminés présentant une identité de séquence d'au moins 90 % avec la séquence de la SEQ ID NO : 4, au moins l'un des suivants :
| | |
|---|---|
| l'acide aminé à la position 120 | est G |
| l'acide aminé à la position 126 | est E |
| l'acide aminé à la position 128 | est K |
| l'acide aminé à la position 134 | est I |
| l'acide aminé à la position 135 | n'est pas C |
| l'acide aminé à la position 138 | est L |
| l'acide aminé à la position 171 | est G |
| l'acide aminé à la position 173 | est T |
| l'acide aminé à la position 182 | est T |
| l'acide aminé à la position 187 | est G |
| l'acide aminé à la position 197 | est S |
| l'acide aminé à la position 198 | est A |
| l'acide aminé à la position 204 | est Y |
| l'acide aminé à la position 230 | est L |
| l'acide aminé à la position 231 | est Y |
| l'acide aminé à la position 234 | est A, et |
dans lequel ladite séquence d'acides aminés présentant une identité de séquence d'au moins 90 % avec la séquence de la SEQ ID NO : 4 présente une amélioration de la stabilité à la température et/ou de l'activité muralytique en comparaison avec la séquence d'acides aminés de la SEQ ID NO : 2.

2. Polypeptide selon la revendication 1, lequel polypeptide présente, dans la séquence d'acides aminés présentant une identité de séquence d'au moins 90 % avec la séquence de la SEQ ID NO : 4, un résidu d'acide glutamique à la position 155.

3. Polypeptide selon la revendication 1 ou 2, dans lequel la séquence présentant une identité de séquence d'au moins 90 % avec la séquence de la SEQ ID NO : 4 diffère de la séquence de la SEQ ID NO : 4 uniquement au niveau des résidus 1, 120, 126, 128, 134, 135, 138, 171, 173, 182, 187, 197, 198, 204, 230, 231 et/ou 234.

4. Polypeptide selon la revendication 1 ou 2, lequel polypeptide comprend une séquence choisie dans le groupe constitué par les SEQ ID NO : 4 - 35.

5. Polypeptide selon l'une quelconque des revendications précédentes, lequel polypeptide comprend au moins une portion de séquence d'acides aminés additionnelle choisie dans le groupe constitué par : KRK et les SEQ ID NO : 36 - 106.

6. Polypeptide selon l'une quelconque des revendications précédentes, lequel polypeptide comprend au moins une portion de séquence d'acides aminés additionnelle ayant la séquence d'acides aminés SMAP-29, SEQ ID NO : 62 ou la séquence d'acides aminés KRKKRKKRK, SEQ ID NO : 40.

7. Polypeptide selon l'une quelconque des revendications précédentes, lequel polypeptide comprend une séquence d'acides aminés présentant une identité de séquence d'au moins 91,5 % avec une séquence d'acides aminés choisie parmi n'importe lesquelles des SEQ ID NO : 107 - 142, et lequel polypeptide ne comprend ni la séquence d'acides aminés de la SEQ ID NO : 2 ni de la SEQ ID NO : 183, et lequel polypeptide ne comprend pas une mutation E155A à la position respective correspondant à la position 155 de la SEQ ID NO : 4, mais présente de préférence un résidu d'acide glutamique à ladite position.

8. Polypeptide selon la revendication 7, lequel polypeptide comprend une séquence d'acides aminés présentant une identité de séquence d'au moins 95 %, d'au moins 97 %, d'au moins 98 %, d'au moins 98,5 %, d'au moins 99 %, d'au moins 99,25 % ou d'au moins 99,5 % avec une séquence d'acides aminés choisie parmi n'importe lesquelles des SEQ ID NO : 107 - 142.

9. Polypeptide selon l'une quelconque des revendications précédentes, lequel polypeptide comprend une séquence choisie dans le groupe constitué par les SEQ ID NO : 107 - 142.

10. Polypeptide selon l'une quelconque des revendications précédentes, lequel polypeptide comprend une séquence choisie dans le groupe constitué par les SEQ ID NO : 144 - 179.

11. Acide nucléique codant un polypeptide selon l'une quelconque des revendications 1 à 10.

12. Vecteur comprenant un acide nucléique selon la revendication 11.

13. Cellule hôte comprenant un polypeptide selon l'une quelconque des revendications 1 à 10, un acide nucléique selon la revendication 11, et/ou un vecteur selon la revendication 12.

14. Composition comprenant un polypeptide selon l'une quelconque des revendications 1 à 10, un acide nucléique selon la revendication 11, un vecteur selon la revendication 12 et/ou une cellule hôte selon la revendication 13.

15. Composition selon la revendication 14, laquelle composition est une composition pharmaceutique comprenant un diluant, excipient ou véhicule pharmaceutiquement acceptable.
